# EUROPEAN PATENT APPLICATION

(11) **EP 3 922 716 A1**
(43) Date of publication of application: **15.12.2021**
(21) Application number: 21187174.4
(22) Date of filing: 16.03.2017
(51) Int. Cl.: C12N 5/0783, G01N 35/08, G01N 33/00, C12M 1/24, B01D 37/00, C12M 1/30, C12M 1/26

(54) **SELECTION AND CLONING OF T LYMPHOCYTES IN A MICROFLUIDIC DEVICE**

(30) Priority: 17.03.2016 US 201662309454 P; 22.04.2016 US 201662326667 P; 24.10.2016 US 201662412212 P; 13.03.2017 US 201762470744 P
(62) Divisional of application: 17767586.5
(71) Applicant: Berkeley Lights, Inc., Emeryville, CA 94608 (US)
(72) Inventor: BRONEVETSKY, Yelena, Emeryville, CA 94608 (US); WANG, Xiaohua, Ho-Ho-Kus, NJ 07423 (US); BEEMILLER, Peter J., Emeryville, CA 94608 (US); BEAUMONT, Kristin G., New York, NY 10128 (US); LOWE, Jr., Randall D., Emeryville, CA 94608 (US); MASTROIANNI, Alexander J., Emeryville, CA 94608 (US); CHAPMAN, Kevin T., Emeryville, CA 94608 (US); MARKS, Natalie C., Emeryville, CA 94608 (US)
(74) Representative: D Young & Co LLP

(57) **Abstract**

Methods of expanding T lymphocytes in a microfluidic device are provided. The methods can include introducing one or more T lymphocytes into a microfluidic device; contacting the one or more T lymphocytes with an activating agent; and perfusing culture medium through the microfluidic device for a period of time sufficient to allow the one or more T lymphocytes to undergo at least one round of mitotic cell division. The expansion can be nonspecific or antigen-specific. T lymphocytes produced according to the disclosed methods are also provided, along with methods of treating cancer in a subject. The methods of treating cancer can include isolating T lymphocytes from a tissue sample obtained from the subject; expanding the isolated T lymphocytes in a microfluidic device; exporting the expanded T lymphocytes from the microfluidic device; and reintroducing the expanded T lymphocytes into the subject.

## Description

### CROSS REFERENCE TO RELATED APPLICATIONS

This application claims the benefit under 35 U. S. C. § 119 of U.S. Patent Application No. 62/309,454, filed on March 17, 2016, U.S. Patent Application No. 62/326,667, filed on April 22, 2016, U.S. Patent Application No. 62/412,212, filed on October 24, 2016, and U.S. Patent Application No. 62/470,744, filed on March 13, 2017, the entire disclosure of each of which is incorporated herein by reference.

### FIELD

The field generally relates to methods, systems and devices for selecting and expanding T lymphocytes within a microfluidic environment.

### BACKGROUND OF THE INVENTION

Immunotherapy is the burgeoning field of using a patient's immune system to help fight disease. A variety of immunotherapy strategies have been evaluated to combat cancer, including stimulating the patient's own immune system to attack cancer cells and administering immune system components from an external source. For example, monoclonal antibodies designed to attack cancer cells in vivo have been administered alone or in genetically engineered constructs. In addition, various T cell therapies have been investigated. Autologous T cell therapies involve obtaining T cells from a subject, expanding the T cells ex vivo, and reintroducing the expanded T cells into the subject. Chimeric antigen receptor T cell (CAR-T) therapies involve genetically engineering T cells to express chimeric antibody-containing fusion proteins on their surface which target the cancer in question and allows for the T cells to kill the cancer cells. Both types of T cell therapies offer advantages. However, the therapies still require further refinement.

One of the key problems in both autologous T cell therapies and CAR-T therapies is the lack of methods for expanding T cells ex vivo in a manner that selectively expands T cells having the highest tumor killing potential. The present disclosure offers solutions for the selective expansion of T cells ex vivo. The present disclosure also provides improved methods for treating subjects suffering from cancer using T cells expanded ex vivo according to the methods disclosed herein.

### SUMMARY

Methods of expanding T lymphocytes in a microfluidic device (or "chip") are disclosed. The microfluidic device can include a microfluidic channel and a sequestration pen fluidically connected to the channel, with the sequestration pen configured to support the culture and expansion of T lymphocytes. In certain embodiments, the microfluidic device can be a nanofluidic device. The sequestration pen can have a volume, for example, of about 0.5x10⁶ to about 5.0x10⁶ cubic microns, or about 0.5x10⁶ to about 2.0x10⁶ cubic microns. Furthermore, the microfluidic device can have more than one microfluidic channel and a plurality of sequestration pens fluidically connected to each microfluidic channel, with each sequestration pen of the plurality configured to support the culture and expansion of T lymphocytes. One or more surfaces of the sequestration pen(s) can be conditioned.

The methods of expanding T lymphocytes can include: introducing one or more T lymphocytes into the sequestration pen in the microfluidic device; and contacting the one or more T lymphocytes with an activating agent. In some embodiments, 20 or fewer, 10 or fewer, 6 to 10, 5 or fewer, about 5, about 4, about 3, about 2, or 1 T lymphocyte(s) are introduced into the sequestration pen. If the microfluidic device includes a plurality of sequestration pens, one or more T lymphocytes (e.g., 20 or fewer, 10 or fewer, 6 to 10, 5 or fewer, about 5, about 4, about 3, about 2, or 1 T lymphocyte(s)) can be introduced into each of one or more sequestration pens. The methods of expanding T lymphocytes can further include perfusing culture medium through the microfluidic channel of the microfluidic device. The perfusion can be continuous or intermittent and can last for a period of time sufficient to allow the one or more T lymphocytes introduced into the sequestration pen (or sequestration pens) to undergo at least one round of mitotic cell division.

In certain embodiments, the one or more T lymphocytes are from a population of T lymphocytes isolated from the peripheral blood of a subject. In other embodiments, the one or more T lymphocytes are from a population of T lymphocytes isolated from a solid tumor sample of a subject. The solid tumor sample can be a fine needle aspirate (FNA) or a tumor biopsy (e.g., a core biopsy). The solid tumor can be a breast cancer, genitourinary cancer (e.g., a cancer originating in the urinary tract, such as in the kidney (e.g., renal cell carcinoma), ureter, bladder, or urethra; cancer of the male reproductive tract (e.g., testicular cancer, prostate cancer, or a cancer of the seminal vesicles, seminal ducts, or penis); or of the female reproductive tract (e.g., ovarian cancer, uterine cancer, cervical cancer, vaginal cancer, or a cancer of the fallopian tubes)), a cancer of the nervous system (e.g., neuroblastoma, retinal blastoma), intestinal cancer (e.g., colorectal cancer), lung cancer, melanoma, or another type of cancer. In specific embodiments, the solid tumor can be a medullary breast cancer, a mesothelioma, or a melanoma.

In some embodiments, the population of T lymphocytes is enriched for CD3⁺ T lymphocytes. In some embodiments, the population of T lymphocytes is enriched for CD3⁺CD4⁺ T lymphocytes. In some embodiments, the population of T lymphocytes is enriched for CD3⁺CD8⁺ T lymphocytes. In some embodiments, the population of T lymphocytes is enriched for CD45RA⁺ T lymphocytes. In some embodiments, the population of T lymphocytes is enriched for CD45RO⁺ T lymphocytes. In some embodiments, the population of T lymphocytes is enriched for CCR7⁺ T lymphocytes. In some embodiments, the population of T lymphocytes is enriched for CD62L⁺ T lymphocytes. In some embodiments, the population of T lymphocytes is enriched for CD45RA⁺/CD7⁺/CD62L⁺ T lymphocytes. In some embodiments, the population of T lymphocytes is enriched for CD45RO⁺/CD7⁺/CD62L⁺ T lymphocytes. In some embodiments, the population of T lymphocytes is enriched for PD-1⁺ T lymphocytes. In some embodiments, the population of T lymphocytes is enriched for CD137⁺ T lymphocytes. In some embodiments, the population of T lymphocytes is enriched for PD-1⁺/CD137⁺ T lymphocytes.

In some embodiments, the population of T lymphocytes is depleted of CD4⁺ T lymphocytes. In some embodiments, the population of T lymphocytes is depleted of CD8⁺ T lymphocytes. In some embodiments, the population of T lymphocytes is depleted of CD45RO⁺ T lymphocytes. In some embodiments, the population of T lymphocytes is depleted of CD45RA⁺ T lymphocytes. In some embodiments, the population of T lymphocytes is depleted of CCR7⁺ T lymphocytes. In some embodiments, the population of T lymphocytes is depleted of CD62L⁺ T lymphocytes. In some embodiments, the population of T lymphocytes is depleted of CD45RO⁺/CD7⁺/CD62L⁺ T lymphocytes. In some embodiments, the population of T lymphocytes is depleted of CD45RA⁺/CD7⁺/CD62L⁺ T lymphocytes. In some embodiments, the population of T lymphocytes is depleted of PD-1⁺ T lymphocytes. In some embodiments, the population of T lymphocytes is depleted of CD137⁺ T lymphocytes. In some embodiments, the population of T lymphocytes is depleted of PD-1⁺/CD137⁺ T lymphocytes.

In some embodiments, the predominant cell type in the population of T lymphocytes is a naive T cell (T_{naïve}), a memory T cell, such as a central memory T cell (T_{CM}) or an effector memory T cell (T_{EM}), or an effector T cell (T_{EFF}).

In some embodiments, the method comprises pre-processing a sample that contains T lymphocytes to enrich for naive T lymphocytes, memory T lymphocytes (e.g., T_{CM} cells and/or T_{EM} cells), T_{EFF} lymphocytes, or any combination thereof. The processing can include removing debris and/or non-lymphocyte cell types from the sample. Alternatively, or in addition, the processing can include depleting the sample of naive T lymphocytes, memory T lymphocytes (such as T_{CM} and/or T_{EM} lymphocytes), T_{EFF} lymphocytes, or a combination thereof.

The sample can be from a subject, such as a subject that is suffering from cancer (e.g., any type of cancer described herein or known in the art). The sample can be a peripheral blood sample or a derivative thereof (e.g., a sample of PBMCs). Alternatively, the sample can be a solid tumor biopsy or FNA. In some embodiments, a peripheral blood sample is processed to enrich for naive T lymphocytes. In other embodiments, a tumor sample is processed to enrich for memory T lymphocytes, particularly T_{CM} lymphocytes although T_{EF} lymphocytes may be enriched. In still other embodiments, a tumor sample is processed to enrich for T_{EFF} lymphocytes. To enrich for the desired T lymphocyte cell type(s), binding agents (e.g., antibodies or the like) that specifically bind to one or more cell surface antigens can be employed. The cell surface antigens bound by the binding agents can be any one of the cell surface antigens described herein, including CD4, CD8, CD45RO, CD45RA, CCR7, CD62L, PD-1, and CD137T, or combinations thereof. The processing can comprise contacting the sample with one or more fluorescently labeled binding agents and performing FACS to select for labeled cells (e.g., if enriching based on the cell surface antigen(s) specifically bound by the one or more binding agents) or to remove labeled cells (e.g., if depleting based on the cell surface antigen(s) specifically bound by the one or more binding agents). Alternatively, or in addition, the processing can comprise contacting the sample with one or more binding agents that are linked to a solid support, and removing cells bound to the solid support (e.g., if depleting based on the cell surface antigen(s) specifically bound by the one or more binding agents) or removing cells that not bound to the solid support (e.g., if enriching based on the cell surface antigen(s) specifically bound by the one or more binding agents). The solid support can be, for example, one or more beads (e.g., a population of beads, which may be magnetic). If magnetic beads are used, a magnetic force can be applied to the sample such that the magnetic beads form a pellet, allowing a resulting supernatant to be separated from the pellet.

In certain embodiments, introducing one or more T lymphocytes into a sequestration pen includes flowing a fluid containing the one or more T lymphocytes into the microfluidic channel of the microfluidic device. Introducing the one or more T lymphocytes into the sequestration pen can further include using dielectrophoresis (DEP) to select and move at least one T lymphocyte located in the microfluidic channel into the sequestration pen. The at least one T lymphocytes can be selected based on cell-surface expression of a marker, such as CD3, CD4, CD8, CD45RO, CD45RA, CCR7, CD62L, PD-1, CD137T, or any combination thereof. Alternatively, introducing one or more T lymphocytes into a sequestration pen can further include tilting the microfluidic chip such that gravity pulls at least one T lymphocyte into the sequestration pen. In other alternatives, introducing one or more T lymphocytes into a sequestration pen can further include centrifuging the microfluidic device such that centrifugal force pulls at least one T lymphocyte into the sequestration pen.

In some embodiments, the at least one selected T lymphocyte is selected, at least in part, because its cell surface is CD4⁺ or CD3⁺CD4⁺. In some embodiments, the at least one selected T lymphocyte is selected, at least in part, because its cell surface is CD8⁺ or CD3⁺CD8⁺. In some embodiments, the at least one selected T lymphocyte is selected, at least in part, because its cell surface is CD45RA⁺ (e.g., CD45RA⁺CD45RO⁻). In some embodiments, the at least one selected T lymphocyte is selected, at least in part, because its cell surface is CD45RA⁻ (e.g., CD45RA-CD45RO⁺). In some embodiments, the at least one selected T lymphocyte is selected, at least in part, because its cell surface is CCR7⁺ and/or CD62L⁺. In some embodiments, the at least one selected T lymphocyte is selected, at least in part, because its cell surface is CCR7⁻ and/or CD62L⁻. In some embodiments, the at least one selected T lymphocyte is selected, at least in part, because its cell surface is CD45RA⁺, CD45RO⁻, CD45RA⁺CCR7⁺, CD45RO⁻ CCR7⁺, CD45RA⁺CD45RO⁻CCR7⁺, CD45RA⁺CD62L⁺, CD45RO⁻CD62L⁺, CD45RA⁺CD45RO⁻ CD62L⁺, CD45RA⁺CCR7⁺CD62L⁺, CD45RO⁻CCR7⁺CD62L⁺, or CD45RA⁺CD45RO⁻ CCR7⁺CD62L⁺. In some embodiments, the at least one selected T lymphocyte is selected, at least in part, because its cell surface is CD45RO⁺, CD45RA⁻, CD45RO⁺CCR7⁺, CD45RA⁻ CCR7⁺, CD45RO⁺CD45RA⁻CCR7⁺, CD45RO⁺CD62L⁺, CD45RA⁻CD62L⁺, CD45RO⁺CD45RA⁻ CD62L⁺, CD45RO⁺CCR7⁺CD62L⁺, CD45RA⁻CCR7⁺CD62L⁺, or CD45RO⁺CD45RA⁻ CCR7⁺CD62L⁺. In some embodiments, the at least one selected T lymphocyte is selected, at least in part, because its cell surface is CD45RO⁺CCR7⁻, CD45RA⁻CCR7⁻, CD45RO⁺CD45RA⁻ CCR7⁻, CD45RO⁺CD62L⁻, CD45RA⁻CD62L⁻, CD45RO⁺CD45RA⁻CD62L⁻, CD45RO⁺CCR7⁻ CD62L⁻, CD45RA⁻CCR7⁻CD62L⁻, CD45RO⁺CD45RA⁻CCR7⁻CD62L⁻. In some embodiments, the at least one selected T lymphocyte is selected, at least in part, because its cell surface is CD69⁻. In some embodiments, the at least one selected T lymphocyte is selected, at least in part, because its cell surface is PD-1⁺ and/or CD137⁺. In some embodiments, the at least one selected T lymphocyte is selected, at least in part, because its cell surface is PD-1⁻ and/or CD137⁻.

In some embodiments, the selecting step comprises contacting the at least one T lymphocyte with one or more binding agents (e.g., antibodies, tetramer of peptide-bound MHC complexes, or the like) and moving the at least one T lymphocyte into the sequestration pen based on the presence (or absence) of binding between the one or more binding agents and the at least one T lymphocyte. In some embodiments, each of the one or more binding agents comprises a label, such as a fluorophore.

In certain embodiments, introducing one or more T lymphocytes into a sequestration pen further comprises introducing an activating agent into the sequestration pen. The one or more T lymphocytes can be contacted with the activating agent prior to being introduced into a sequestration pen. For example, the T lymphocytes can be incubated with the activating agent for a period of at least one hour (e.g., at least 2, 3, 4, 5, or more hours) prior to be introduced into the microfluidic device. Alternatively, the one or more T lymphocytes can be contacted with the activating agent after being introduced into a sequestration pen.

In certain embodiments, the activating agent can include CD3 and CD28 agonists. The CD3 and/or CD28 agonist can be an antibody. In some embodiments, the anti-CD3 agonist (e.g., antibody) is conjugated to a solid support. In some embodiments, the anti-CD28 agonist (e.g., antibody) is conjugated to a solid support. In some embodiments, the anti-CD28 agonist (e.g., antibody) is a solute in an aqueous solution. Thus, for example, the activating agent can comprise one or more beads conjugated to anti-CD3 and anti-CD28 agonist antibodies. Alternatively, the activating agent can comprise one or more beads conjugated to anti-CD3 agonist antibodies and a solution of soluble anti-CD28 agonist antibody. In still other alternatives, the CD3 and/or CD28 agonists can be linked (covalently or non-covalently) to one or more surfaces of a sequestration pen. The surface(s) of the sequestration pen can be conditioned in a manner that facilitates linkage of the CD3 and/or CD28 agonists to the surface(s).

In certain embodiments, the activating agent can comprise a dendritic cell (DC). The dendritic cell can be pulsed with an antigen of interest (e.g., a cancer-associated antigen, an infectious disease-associated antigen, or an antigen associated with some other type of condition) prior to contacting the one or more T lymphocytes. The cancer-associated antigen, which can be a well-known antigen or a neoantigen, can be identified through genomic analysis of tumor cells obtained from a tumor biopsy.

In certain embodiments, the activating agent can comprise a complex between a peptide antigen and an MHC molecule. Such complexes can be linked, as in the case of peptide-MHC tetramer complexes. The peptide-MHC complexes can be conjugated to a solid support. In some embodiments, the solid support can be one or more beads. In other embodiments, the solid support can be one or more surfaces of a sequestration pen. Thus, the surface(s) of the sequestration pen can be conditioned in a manner that facilitates linkage of the peptide-MHC complexes to the surface(s).

In certain embodiments, culture medium is perfused through the microfluidic channel of the microfluidic device for a period of at least 24 hours (e.g., at least 48, 72, 96, 110, or more hours, or any number therebetween). In certain embodiments, the culture medium includes mammalian serum, which can be a human serum (e.g., Human AB serum), optionally in combination with a bovine serum (e.g., fetal bovine serum or calf serum). In certain embodiments, the culture medium further comprises Interleukin 2 (IL2), Interleukin 7 (IL7), Interleukin 21 (IL21), Interleukin 15 (IL15), or any combination thereof. In some embodiments, the culture medium comprises about 50 U/ml IL2 (e.g., about 1 U/ml to about 10 U/ml IL2). In some embodiments, the culture medium comprises about 5 ng/ml IL7 (e.g., about 1 ng/ml to about 10 ng/ml IL7). In some embodiments, the culture medium comprises about 30 ng/ml IL21 (e.g., about 10 ng/ml to about 50 ng/ml IL21).

In some embodiments, the method comprises assaying the in-pen proliferation rate of the one or more T lymphocytes. In some embodiments, the method comprises assaying the expression of one or more cytokines, e.g., INFgamma, TNFalpha, IL2, or a combination thereof, by the one or more T lymphocytes in the sequestration pen. In some embodiments, the method comprises assaying the expression of one or more regulatory T cell markers, such as CTLA4, by the one or more T lymphocytes in the sequestration pen.

In some embodiments, T lymphocytes are exported from the microfluidic device, e.g., after expansion. Export can be selective, e.g., based at least in part on proliferation rate, cytokine expression, expression of regulatory T cell marker(s), or a combination thereof. In some embodiments, a sample of exported T lymphocytes is genomically profiled.

In certain embodiments, methods of treating cancer in a subject are disclosed. The subject can be a mammal, such as a human. The cancer can be a breast cancer, genitourinary cancer (e.g., a cancer originating in the urinary tract, such as in the kidneys (e.g., renal cell carcinoma), ureters, bladder, or urethra; cancer of the male reproductive tract (e.g., testicular cancer, prostate cancer, or a cancer of the seminal vesicles, seminal ducts, or penis); or of the female reproductive tract (e.g., ovarian cancer, uterine cancer, cervical cancer, vaginal cancer, or a cancer of the fallopian tubes)), a cancer of the nervous system (e.g., neuroblastoma), intestinal cancer (e.g., colorectal cancer), lung cancer, melanoma, or another type of cancer. In particular embodiments, the cancer can be medullary breast cancer, mesothelioma, or melanoma.

The methods of treating cancer can include: isolating T lymphocytes from a tissue sample obtained from a subject; expanding the isolated T lymphocytes in a microfluidic device according to any of the methods disclosed herein; exporting the expanded T lymphocytes from the microfluidic device; and reintroducing the expanded T lymphocytes into the subject.

In certain embodiments, the tissue sample is a sample of peripheral blood. In certain embodiments, the tissue sample is from a solid tumor. The tissue sample, for example, can be a FNA or a biopsy (e.g., a core biopsy) from the solid tumor. The solid tumor sample can be from any of the cancers discussed above.

In certain embodiments, isolating T lymphocytes from the tissue sample comprises performing a selection for CD3⁺ cells in the tissue sample. In some embodiments, the selection can be based on one or more of the markers CD3, CD4, CD8, CD45RA, CD45RO, CCR7, CD62L, PD-1, PD-L1, and CD137, as described above and elsewhere herein. The selection can include using beads, such as magnetic beads, having a CD3-binding agent (or other marker-binding agent) attached thereto. In certain embodiments, isolating T lymphocytes from the tissue sample further comprises dissociating the tissue sample prior to performing the selection for CD3⁺ (or other marker-positive) cells in the tumor sample.

In certain embodiments, the isolated T lymphocytes are introduced into a microfluidic device. The microfluidic device can be a nanofluidic device. In certain embodiments, expanding the isolated T lymphocytes comprises introducing one or more isolated T lymphocytes into a sequestration pen of the microfluidic (or nanofluidic) device. The sequestration pen can, for example, have a volume of about 0.5x10⁶ to about 5x10⁶ cubic microns, or about 0.5x10⁶ to about 2.0x10⁶ cubic microns.

In certain embodiments, expanding the isolated T lymphocytes comprises contacting the isolated T lymphocytes with an activating agent. In some embodiments, the activating agent comprises CD3 and/or CD28 agonists. The CD3 and/or CD28 agonist can be an antibody. In some embodiments, the anti-CD3 agonist is conjugated to a solid support. In some embodiments, the anti-CD28 agonist is conjugated to a solid support. Thus, the activating agent can comprise, for example, one or more solid supports (e.g., one or more beads), each of which can be conjugated to anti-CD3 and/or anti-CD28 agonist antibodies. In some embodiments, the anti-CD28 agonist (e.g., antibody) is provided as a solute in an aqueous solution. In other embodiments, the activating agent comprises dendritic cells (DCs). The DCs can be obtained from the subject being treated and/or can be pulsed with a tumor antigen prior to contacting the isolated T lymphocytes. The tumor antigen can be identified through sequencing of nucleic acid molecules (e.g., gDNA, mRNA, etc.) from cancer cells obtained from the subject. In still other embodiments, the activating agent can be a peptide-MHC complex or cluster of complexes (such as a peptide-MHC tetramer complex), which may be linked to a solid support, such as one or more beads or one or more surfaces (e.g., conditioned surfaces) of the sequestration pen.

In certain embodiments, the T lymphocytes are incubated with the activating agent for a period of at least one hour (e.g., at least 2, 3, 4, 5, or more hours) prior to be introduced into the microfluidic device. In certain embodiments, introducing the isolated T lymphocytes into the sequestration pen further comprises introducing the activating agent into the sequestration pen.

In certain embodiments, isolated T lymphocytes that undergo expansion in response to being contacted by activating agent exhibit at least 100-fold (e.g., at least 200-fold, at least 500-fold, at least 1000-fold, etc.) expansion. In certain embodiments, the expanded T lymphocytes are further expanded "off chip" after being exported from the microfluidic device but prior to being reintroduced into the subject.

Any of the methods of described herein may be performed using a microfluidic device that has one or more inner surfaces (e.g., a substrate surface, a cover surface, and/or the surfaces of the circuit material) that have been conditioned or coated so as to present a layer of organic and/or hydrophilic molecules that provides the primary interface between the microfluidic device and T cells grown therein. For example, the flow path and the sequestration pen(s) can be treated with a coating solution that bonds to one or more inner surfaces and presents and organic and/or hydrophilic layer of molecules. Thus, the coating solution can comprise a coating agent that binds to the one or more inner surfaces, such as serum, serum albumin (e.g., BSA), polymer, detergent, enzymes, or any combination thereof.

In certain embodiments, the microfluidic device can comprise an inner substrate surface (and/or an inner cover surface and/or inner surfaces of the circuit material) that comprise a coating material. In some embodiments, the coating material includes molecules having a linking group and an alkyl moiety. The linking group can be covalently bonded to the inner substrate surface, and can be, for example, a siloxy linking group. The alkyl moiety can be, for example, an unsubstituted alkyl moiety or a substituted alkyl moiety, such as a fluoroalkyl moiety or a perflouroalkyl moiety. The alkyl moiety can include a linear chain of carbons comprising at least 10 carbon atoms (e.g., at least 12, 14, 16, 18, 20, 22, or more carbon atoms). The molecules of the coating material can form a densely-packed monolayer structure covalently bound to the inner substrate surface (and/or the inner cover surface and/or the inner surfaces of the circuit material).

In some embodiments, the coating material comprises molecules having a linking group and a cationic moiety and/or an anionic moiety, wherein the linking group is covalently bonded to the inner substrate surface (and/or the inner cover surface and/or the inner surfaces of the circuit material). The cationic moiety can include a quaternary ammonium group. The anionic moiety can include a phosphonic acid, carboxylic acid, or sulfonic acid. In some related embodiments, the coating material can comprise molecules having a linking group and a zwitterionic moiety, wherein the linking group is covalently bound to the inner substrate surface (and/or the inner cover surface and/or the inner surfaces of the circuit material). The zwitterionic moiety is selected from carboxybetaines, sulfobetaines, sulfamic acids, and amino acids. In some embodiments, the cationic, anionic, or zwitterionic moieties are capable of ionically bonding with a blocking agent).

In some embodiments, the coating material comprises a polymer comprising alkylene ether moieties, saccharide moieties, or amino acid moieties. For example, the coating material can comprise dextran or polyethylene glycol (PEG). Alternatively, or in addition, the coating material can comprise protein polymers (e.g., proteins that promote T cell activation).

Also provided is a T lymphocyte produced according to a method disclosed herein. Also provided is a microfluidic device comprising one or more, e.g., 1, 2, 3, 4, 5, 6, 7, 8, 9, 10, 11, 12, 13, 14, 15, 16, 17, 18, 19, 20, or more T lymphocytes produced according to a method disclosed herein, e.g., in one or more sequestration pens of the microfluidic device. Also provided is a pharmaceutical composition comprising T lymphocyte produced according to a method disclosed herein and, optionally, a pharmaceutically acceptable carrier.

Additional objects and advantages will be set forth in part in the description which follows, and in part will be obvious from the description, or may be learned by practice. The objects and advantages will be realized and attained by means of the elements and combinations particularly pointed out in the appended claims.

It is to be understood that both the foregoing general description and the following detailed description are exemplary and explanatory only and are not restrictive of the claims.

The accompanying drawings, which are incorporated in and constitute a part of this specification, illustrate one (several) embodiment(s) and, together with the description, serve to explain the principles described herein.

### BRIEF DESCRIPTION OF THE DRAWINGS

Figure 1A illustrates an example of a system for use with a microfluidic device and associated control equipment according to some embodiments of the disclosure.
Figures 1B and 1C illustrate a microfluidic device according to some embodiments of the disclosure.
Figures 2A and 2B illustrate isolation pens according to some embodiments of the disclosure.
Figure 2C illustrates a detailed sequestration pen according to some embodiments of the disclosure.
Figures 2D-F illustrate sequestration pens according to some other embodiments of the disclosure.
Figure 2G illustrates a microfluidic device according to an embodiment of the disclosure.
Figure 2H illustrates a coated surface of the microfluidic device according to an embodiment of the disclosure.
Figure 3A illustrates a specific example of a system for use with a microfluidic device and associated control equipment according to some embodiments of the disclosure.
Figure 3B illustrates an imaging device according to some embodiments of the disclosure.
Figure 4 provides a series of time-lapse images of a single nanowell in a nanofluidic chip containing human T lymphocytes cultured with anti-CD3/anti-CD28 T cell-activating beads. The images show the nanowell at 0, 24, 48, 72, and 96 hours of culture.
Figure 5A provides a series of time-lapse images of a single nanowell in a nanofluidic chip containing human T lymphocytes cultured with allogeneic dendritic cells (DCs). The images show the nanowell at 0, 24, 48, 72, and 96 hours of culture.
Figure 5B provides a series of time-lapse images of a single nanowell in a nanofluidic chip containing human T lymphocytes cultured with dendritic cells (DCs) that have been pulsed with tetanus toxin and Epstein bar virus antigens. The images show the nanowell at 0, 24, 48, 72, 96, and 110 hours of culture.
Figure 6A provides an image showing the incorporation of EdU (fluorescence signal) in the T cells in the nanowell of Figure 6A. EdU incorporation is shown (red) for the 96-hour culture time point and is overlaid on a bright-field image of the selectively expanded T cells.
Figure 6B provides an image showing the incorporation of EdU (fluorescence signal) in the T cells in the nanowell of Figure 6B. EdU incorporation is shown (red) for the 110-hour culture time point and is overlaid on a bright-field image of the selectively expanded T cells.
Figures 7A and 7B are photographic representations of an embodiment of culturing T cells in a microfluidic device having at least one conditioned surface.
Figure 8A illustrates an exemplary workflow for obtaining T lymphocytes from a sample and introducing them into sequestration pens in a microfluidic device, thereby providing penned cells.
Figure 8B illustrates an exemplary workflow for expanding penned T lymphocytes, such as T lymphocytes penned according to a workflow as illustrated in Figure 8A.
Figure 8C illustrates an exemplary workflow for exporting expanded T lymphocytes, such as T lymphocytes expanded according to a workflow as illustrated in Figure 8B.

### DETAILED DESCRIPTION OF EMBODIMENTS

This specification describes exemplary embodiments and applications of the disclosure. The disclosure, however, is not limited to these exemplary embodiments and applications or to the manner in which the exemplary embodiments and applications operate or are described herein. Moreover, the figures may show simplified or partial views, and the dimensions of elements in the figures may be exaggerated or otherwise not in proportion. In addition, as the terms "on," "attached to," "connected to," "coupled to," or similar words are used herein, one element (e.g., a material, a layer, a substrate, etc.) can be "on," "attached to," "connected to," or "coupled to" another element regardless of whether the one element is directly on, attached to, connected to, or coupled to the other element or there are one or more intervening elements between the one element and the other element. Also, unless the context dictates otherwise, directions (e.g., above, below, top, bottom, side, up, down, under, over, upper, lower, horizontal, vertical, "x," "y," "z," etc.), if provided, are relative and provided solely by way of example and for ease of illustration and discussion and not by way of limitation. In addition, where reference is made to a list of elements (e.g., elements a, b, c), such reference is intended to include any one of the listed elements by itself, any combination of less than all of the listed elements, and/or a combination of all of the listed elements. Section divisions in the specification are for ease of review only and do not limit any combination of elements discussed.

Where dimensions of microfluidic features are described as having a width or an area, the dimension typically is described relative to an x-axial and/or y-axial dimension, both of which lie within a plane that is parallel to the substrate and/or cover of the microfluidic device. The height of a microfluidic feature may be described relative to a z-axial direction, which is perpendicular to a plane that is parallel to the substrate and/or cover of the microfluidic device. In some instances, a cross sectional area of a microfluidic feature, such as a channel or a passageway, may be in reference to a x-axial/z-axial, a y-axial/z-axial, or an x-axial/y-axial area.

As used herein, "substantially" means sufficient to work for the intended purpose. The term "substantially" thus allows for minor, insignificant variations from an absolute or perfect state, dimension, measurement, result, or the like such as would be expected by a person of ordinary skill in the field but that do not appreciably affect overall performance. When used with respect to numerical values or parameters or characteristics that can be expressed as numerical values, "substantially" means within ten percent.

The term "ones" means more than one.

As used herein, the term "plurality" can be 2, 3, 4, 5, 6, 7, 8, 9, 10, or more.

As used herein, the term "disposed" encompasses within its meaning "located."

As used herein, a "microfluidic device" or "microfluidic apparatus" is a device that includes one or more discrete microfluidic circuits configured to hold a fluid, each microfluidic circuit comprised of fluidically interconnected circuit elements, including but not limited to region(s), flow path(s), channel(s), chamber(s), and/or pen(s), and at least one port configured to allow the fluid (and, optionally, micro-objects suspended in the fluid) to flow into and/or out of the microfluidic device. Typically, a microfluidic circuit of a microfluidic device will include a flow path, which may include a microfluidic channel, and at least one chamber, and will hold a volume of fluid of less than about 1 mL, e.g., less than about 750, 500, 250, 200, 150, 100, 75, 50, 25, 20, 15, 10, 9, 8, 7, 6, 5, 4, 3, or 2 µL. In certain embodiments, the microfluidic circuit holds about 1-2, 1-3, 1-4, 1-5, 2-5, 2-8, 2-10, 2-12, 2-15, 2-20, 5-20, 5-30, 5-40, 5-50, 10-50, 10-75, 10-100, 20-100, 20-150, 20-200, 50-200, 50-250, or 50-300 µL. The microfluidic circuit may be configured to have a first end fluidically connected with a first port (e.g., an inlet) in the microfluidic device and a second end fluidically connected with a second port (e.g., an outlet) in the microfluidic device.

As used herein, a "nanofluidic device" or "nanofluidic apparatus" is a type of microfluidic device having a microfluidic circuit that contains at least one circuit element configured to hold a volume of fluid of less than about 1 µL, e.g., less than about 750, 500, 250, 200, 150, 100, 75, 50, 25, 20, 15, 10, 9, 8, 7, 6, 5, 4, 3, 2, 1 nL or less. A nanofluidic device may comprise a plurality of circuit elements (e.g., at least 2, 3, 4, 5, 6, 7, 8, 9, 10, 15, 20, 25, 50, 75, 100, 150, 200, 250, 300, 400, 500, 600, 700, 800, 900, 1000, 1500, 2000, 2500, 3000, 3500, 4000, 4500, 5000, 6000, 7000, 8000, 9000, 10,000, or more). In certain embodiments, one or more (e.g., all) of the at least one circuit elements is configured to hold a volume of fluid of about 100 pL to 1 nL, 100 pL to 2 nL, 100 pL to 5 nL, 250 pL to 2 nL, 250 pL to 5 nL, 250 pL to 10 nL, 500 pL to 5 nL, 500 pL to 10 nL, 500 pL to 15 nL, 750 pL to 10 nL, 750 pL to 15 nL, 750 pL to 20 nL, 1 to 10 nL, 1 to 15 nL, 1 to 20 nL, 1 to 25 nL, or 1 to 50 nL. In other embodiments, one or more (e.g., all) of the at least one circuit elements are configured to hold a volume of fluid of about 20 nL to 200nL, 100 to 200 nL, 100 to 300 nL, 100 to 400 nL, 100 to 500 nL, 200 to 300 nL, 200 to 400 nL, 200 to 500 nL, 200 to 600 nL, 200 to 700 nL, 250 to 400 nL, 250 to 500 nL, 250 to 600 nL, or 250 to 750 nL.

A "microfluidic channel" or "flow channel" as used herein refers to flow path of a microfluidic device having a length that is significantly longer than both the horizontal and vertical dimensions. For example, the flow channel can be at least 5 times the length of either the horizontal or vertical dimension, e.g., at least 10 times the length, at least 25 times the length, at least 100 times the length, at least 200 times the length, at least 500 times the length, at least 1,000 times the length, at least 5,000 times the length, or longer. In some embodiments, the length of a flow channel is in the range of from about 100,000 microns to about 500,000 microns, including any range therebetween. In some embodiments, the horizontal dimension is in the range of from about 100 microns to about 1000 microns (e.g., about 150 to about 500 microns) and the vertical dimension is in the range of from about 25 microns to about 200 microns, e.g., from about 40 to about 150 microns. It is noted that a flow channel may have a variety of different spatial configurations in a microfluidic device, and thus is not restricted to a perfectly linear element. For example, a flow channel may be, or include one or more sections having, the following configurations: curve, bend, spiral, incline, decline, fork (e.g., multiple different flow paths), and any combination thereof. In addition, a flow channel may have different cross-sectional areas along its path, widening and constricting to provide a desired fluid flow therein. The flow channel may include valves, and the valves may be of any type known in the art of microfluidics. Examples of microfluidic channels that include valves are disclosed in U.S. Patents 6,408,878 and 9,227,200, each of which is herein incorporated by reference in its entirety.

As used herein, the term "obstruction" refers generally to a bump or similar type of structure that is sufficiently large so as to partially (but not completely) impede movement of target micro-objects between two different regions or circuit elements in a microfluidic device. The two different regions/circuit elements can be, for example, the connection region and the isolation region of a microfluidic sequestration pen.

As used herein, the term "constriction" refers generally to a narrowing of a width of a circuit element (or an interface between two circuit elements) in a microfluidic device. The constriction can be located, for example, at the interface between the isolation region and the connection region of a microfluidic sequestration pen of the instant disclosure.

As used herein, the term "transparent" refers to a material which allows visible light to pass through without substantially altering the light as is passes through.

As used herein, the term "micro-object" refers generally to any microscopic object that may be isolated and/or manipulated in accordance with the present disclosure. Non-limiting examples of micro-objects include: inanimate micro-objects such as microparticles; microbeads (e.g., polystyrene beads, Luminex^{™} beads, or the like); magnetic beads; microrods; microwires; quantum dots, and the like; biological micro-objects such as cells; biological organelles; vesicles, or complexes; synthetic vesicles; liposomes (e.g., synthetic or derived from membrane preparations); lipid nanorafts, and the like; or a combination of inanimate micro-objects and biological micro-objects (e.g., microbeads attached to cells, liposome-coated micro-beads, liposome-coated magnetic beads, or the like). Beads may include moieties/molecules covalently or non-covalently attached, such as fluorescent labels, proteins, carbohydrates, antigens, small molecule signaling moieties, or other chemical/biological species capable of use in an assay. Lipid nanorafts have been described, for example, in Ritchie et al. (2009) "Reconstitution of Membrane Proteins in Phospholipid Bilayer Nanodiscs," Methods Enzymol., 464:211-231.

As used herein, the term "cell" is used interchangeably with the term "biological cell." Non-limiting examples of biological cells include eukaryotic cells, plant cells, animal cells, such as mammalian cells, reptilian cells, avian cells, fish cells, or the like, prokaryotic cells, bacterial cells, fungal cells, protozoan cells, or the like, cells dissociated from a tissue, such as muscle, cartilage, fat, skin, liver, lung, neural tissue, and the like, immunological cells, such as T cells, B cells, natural killer cells, macrophages, and the like, embryos (e.g., zygotes), oocytes, ova, sperm cells, hybridomas, cultured cells, cells from a cell line, cancer cells, infected cells, transfected and/or transformed cells, reporter cells, and the like. A mammalian cell can be, for example, from a human, a mouse, a rat, a horse, a goat, a sheep, a cow, a primate, or the like.

A colony of biological cells is "clonal" if all of the living cells in the colony that are capable of reproducing are daughter cells derived from a single parent cell. In certain embodiments, all the daughter cells in a clonal colony are derived from the single parent cell by no more than 10 divisions. In other embodiments, all the daughter cells in a clonal colony are derived from the single parent cell by no more than 14 divisions. In other embodiments, all the daughter cells in a clonal colony are derived from the single parent cell by no more than 17 divisions. In other embodiments, all the daughter cells in a clonal colony are derived from the single parent cell by no more than 20 divisions. The term "clonal cells" refers to cells of the same clonal colony.

As used herein, a "colony" of biological cells refers to 2 or more cells (e.g. about 2 to about 20, about 4 to about 40, about 6 to about 60, about 8 to about 80, about 10 to about 100, about 20 to about 200, about 40 to about 400, about 60 to about 600, about 80 to about 800, about 100 to about 1000, or greater than 1000 cells).

As used herein, the term "maintaining (a) cell(s)" refers to providing an environment comprising both fluidic and gaseous components and, optionally a surface, that provides the conditions necessary to keep the cells viable and/or expanding.

As used herein, the term "expanding" when referring to cells, refers to increasing in cell number.

A "component" of a fluidic medium is any chemical or biochemical molecule present in the medium, including solvent molecules, ions, small molecules, antibiotics, nucleotides and nucleosides, nucleic acids, amino acids, peptides, proteins, sugars, carbohydrates, lipids, fatty acids, cholesterol, metabolites, or the like.

As used herein, "capture moiety" is a chemical or biological species, functionality, or motif that provides a recognition site for a micro-object. A selected class of micro-objects may recognize the in situ-generated capture moiety and may bind or have an affinity for the in situ-generated capture moiety. Non-limiting examples include antigens, antibodies, and cell surface binding motifs.

As used herein, "flowable polymer" is a polymer monomer or macromer that is soluble or dispersible within a fluidic medium (e.g., a pre-polymer solution). The flowable polymer may be input into a microfluidic flow path, where it can flow with other components of a fluidic medium therein.

As used herein, "photoinitiated polymer" refers to a polymer (or a monomeric molecule that can be used to generate the polymer) that upon exposure to light, is capable of crosslinking covalently, forming specific covalent bonds, changing regiochemistry around a rigidified chemical motif, or forming ion pairs which cause a change in physical state, and thereby forming a polymer network. In some instances, a photoinitiated polymer may include a polymer segment bound to one or more chemical moieties capable of crosslinking covalently, forming specific covalent bonds, changing regiochemistry around a rigidified chemical motif, or forming ion pairs which cause a change in physical state. In some instances, a photoinitiated polymer may require a photoactivatable radical initiator to initiate formation of the polymer network (e.g., via polymerization of the polymer).

As used herein, "antibody" refers to an immunoglobulin (Ig) and includes both polyclonal and monoclonal antibodies; primatized (e.g., humanized); murine; mouse-human; mouse-primate; and chimeric; and may be an intact molecule, a fragment thereof (such as scFv, Fv, Fd, Fab, Fab' and F(ab)'2 fragments), or multimers or aggregates of intact molecules and/or fragments; and may occur in nature or be produced, e.g., by immunization, synthesis or genetic engineering. An "antibody fragment," as used herein, refers to fragments, derived from or related to an antibody, which bind antigen and which in some embodiments may be derivatized to exhibit structural features that facilitate clearance and uptake, e.g., by the incorporation of galactose residues. This includes, e.g., F(ab), F(ab)'2, scFv, light chain variable region (VL), heavy chain variable region (VH), and combinations thereof.

As used herein in reference to a fluidic medium, "diffuse" and "diffusion" refer to thermodynamic movement of a component of the fluidic medium down a concentration gradient.

The phrase "flow of a medium" means bulk movement of a fluidic medium primarily due to any mechanism other than diffusion. For example, flow of a medium can involve movement of the fluidic medium from one point to another point due to a pressure differential between the points. Such flow can include a continuous, pulsed, periodic, random, intermittent, or reciprocating flow of the liquid, or any combination thereof. When one fluidic medium flows into another fluidic medium, turbulence and mixing of the media can result.

The phrase "substantially no flow" refers to a rate of flow of a fluidic medium that, averaged over time, is less than the rate of diffusion of components of a material (e.g., an analyte of interest) into or within the fluidic medium. The rate of diffusion of components of such a material can depend on, for example, temperature, the size of the components, and the strength of interactions between the components and the fluidic medium.

As used herein in reference to different regions within a microfluidic device, the phrase "fluidically connected" means that, when the different regions are substantially filled with fluid, such as fluidic media, the fluid in each of the regions is connected so as to form a single body of fluid. This does not mean that the fluids (or fluidic media) in the different regions are necessarily identical in composition. Rather, the fluids in different fluidically connected regions of a microfluidic device can have different compositions (e.g., different concentrations of solutes, such as proteins, carbohydrates, ions, or other molecules) which are in flux as solutes move down their respective concentration gradients and/or fluids flow through the microfluidic device.

As used herein, a "flow path" or "flow region" refers to one or more fluidically connected circuit elements (e.g. channel(s), region(s), chamber(s) and the like) that define, and are subject to, the trajectory of a flow of medium. A flow path is thus an example of a swept region of a microfluidic device. Other circuit elements (e.g., unswept regions) may be fluidically connected with the circuit elements that comprise the flow path without being subject to the flow of medium in the flow path.

As used herein, "isolating a micro-object" confines a micro-object to a defined area within the microfluidic device. The micro-object may still be capable of motion within an in situ-generated capture structure.

A microfluidic (or nanofluidic) device can comprise "swept" regions and "unswept" regions. As used herein, a "swept" region is comprised of one or more fluidically interconnected circuit elements of a microfluidic circuit, each of which experiences a flow of medium when fluid is flowing through the microfluidic circuit. The circuit elements of a swept region can include, for example, regions, channels, and all or parts of chambers. As used herein, an "unswept" region is comprised of one or more fluidically interconnected circuit element of a microfluidic circuit, each of which experiences substantially no flux of fluid when fluid is flowing through the microfluidic circuit. An unswept region can be fluidically connected to a swept region, provided the fluidic connections are structured to enable diffusion but substantially no flow of media between the swept region and the unswept region. The microfluidic device can thus be structured to substantially isolate an unswept region from a flow of medium in a swept region, while enabling substantially only diffusive fluidic communication between the swept region and the unswept region. For example, a flow channel of a micro-fluidic device is an example of a swept region while an isolation region (described in further detail below) of a microfluidic device is an example of an unswept region.

As used herein, "enrich" means to increase the concentration of a component, such as a cell type of interest, in a composition or population relative to other components, such as other cell types. The enrichment can be the result of reducing the concentration of the other components (e.g., other cell types) in the composition or population. For example, as used herein, "enriching" for a certain subpopulation of T lymphocytes in a sample means increasing the proportion of the subpopulation relative to the total number of cells in the sample.

As used herein, "deplete" means to decrease the concentration of a component, such as an undesired cell type, in a composition or population relative to other components, such as one or more desired cell types. The depletion can be the result of increasing the concentration of one or more of the other components. For example, as used herein, "depleting" a sample of a certain subpopulation of T lymphocytes means decreasing the proportion of the subpopulation relative to the total number of cells in the sample.

The capability of biological micro-objects (e.g., biological cells) to produce specific biological materials (e.g., proteins, such as antibodies) can be assayed in such a microfluidic device. In a specific embodiment of an assay, sample material comprising biological micro-objects (e.g., cells) to be assayed for production of an analyte of interest can be loaded into a swept region of the microfluidic device. Ones of the biological micro-objects (e.g., mammalian cells, such as human cells) can be selected for particular characteristics and disposed in unswept regions. The remaining sample material can then be flowed out of the swept region and an assay material flowed into the swept region. Because the selected biological micro-objects are in unswept regions, the selected biological micro-objects are not substantially affected by the flowing out of the remaining sample material or the flowing in of the assay material. The selected biological micro-objects can be allowed to produce the analyte of interest, which can diffuse from the unswept regions into the swept region, where the analyte of interest can react with the assay material to produce localized detectable reactions, each of which can be correlated to a particular unswept region. Any unswept region associated with a detected reaction can be analyzed to determine which, if any, of the biological micro-objects in the unswept region are sufficient producers of the analyte of interest.

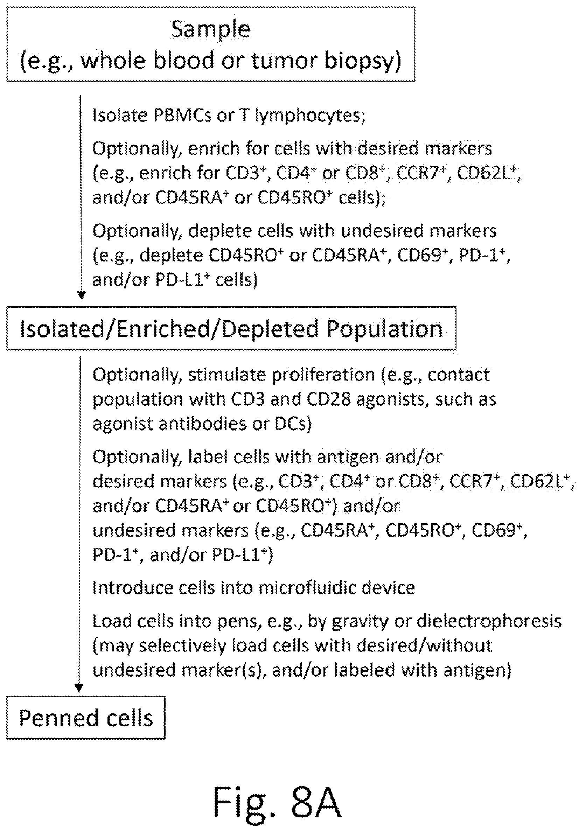

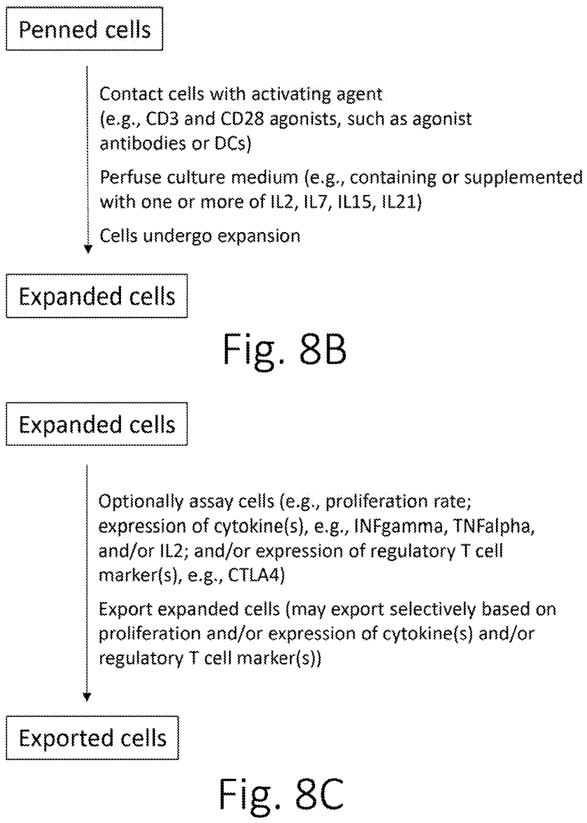

**Culturing, Selecting, and Expanding T Lymphocytes in Microfluidic/Nanofluidic Devices.** T lymphocytes can be cultured, selected, and expanded in the microfluidic and nanofluidic devices described herein. In addition to the discussion in this section, the methods of culture, selection, and expansion are described in the Summary of the Invention (above), in the Examples (below), and in the claims appended hereto. Exemplary workflows are illustrated in Figs. 8A-C. Methods of expanding biological cells, including T cells, within the presently disclosed microfluidic devices have also been described in U.S. Patent Application No. 15/135,707, filed on April 22, 2016, the entire contents of which are incorporated herein by reference.

A population of T lymphocytes can be obtained through known methods. In some embodiments, peripheral blood mononuclear cells (PBMCs) comprising T lymphocytes are isolated from a blood sample, such as a whole blood sample. In some embodiments, T lymphocytes are isolated from a solid tumor sample, e.g., a fine needle aspirate or a biopsy. The population of isolated T lymphocytes can be enriched for desired cell types or depleted of undesired cell types. Enrichment and depletion can be performed using, e.g., flow cytometry, T cell enrichment columns, antibody-conjugated beads (e.g., magnetic beads), etc., and may use positive or negative selections such as isolating desired cells from a population or removing undesired cells from a population, respectively. For example, magnetic beads conjugated to antibodies to CD45RO can be used to deplete CD45RO⁺ cells from a population. As another example, magnetic beads conjugated to antibodies to CD45RA can be used to deplete CD45RA⁺ cells from a population.

In some embodiments, the T lymphocytes are enriched for CD3⁺ T lymphocytes. In some embodiments, the one or more T lymphocytes are from a population of CD3⁺ T lymphocytes isolated from a peripheral blood sample or a solid tumor sample. In some embodiments, the T lymphocytes are enriched for CD3⁺CD4⁺ cells (e.g., helper T cells). In some embodiments, the T lymphocytes are enriched for CD3⁺CD8⁺ cells (e.g., cytotoxic T cells). In some embodiments, the T lymphocytes are enriched for both CD3⁺CD4⁺ and CD3⁺CD8⁺ cells. In some embodiments, the T lymphocytes are enriched for CCR7⁺ cells, e.g., CD3⁺CCR7⁺, CD3⁺CD4⁺CCR7⁺, or CD3⁺CD8⁺ CCR7⁺ cells. In some embodiments, the T lymphocytes are enriched for CD45RA⁺ cells, e.g., CD3⁺CD45RA⁺, CD3⁺CD4⁺CD45RA⁺, or CD3⁺CD8⁺CD45RA⁺ cells. In some embodiments, the T lymphocytes are enriched for CD45RO⁺ cells, e.g., CD3⁺CD45RO⁺, CD3⁺CD4⁺CD45RO⁺, or CD3⁺CD8⁺CD45RO⁺ cells.

In some embodiments, the T lymphocytes are depleted of CD45RO⁺, CD45RO⁺CCR7⁺, CD45RO⁺CD62L⁺, or CD45RO⁺CCR7⁺CD62L⁺cells. In some embodiments, the T lymphocytes are depleted of CD45RA⁺, CD45RA⁺CCR7⁺, CD45RA⁺CD62L⁺, or CD45RA⁺CCR7⁺CD62L⁺cells.

T lymphocytes in a microfluidic device can also be selected based on the presence or absence of desired or undesired markers alternatively or in addition to enrichment or depletion as discussed above. Dielectrophoresis can be used to move a T lymphocyte located in the microfluidic channel into the sequestration pen. For example, a T lymphocyte can be selected, at least in part, for placement in a sequestration pen of the microfluidic device because the cell surface is CD3⁺, CD4⁺, CD8⁺, or any combination thereof, e.g., CD3⁺CD4⁺ or CD3⁺CD8⁺. Selection can comprise labeling with an antibody (e.g., a fluorescent antibody) and moving the T lymphocyte associated with the antibody into the sequestration pen.

In some embodiments, a T lymphocyte can be selected, at least in part, for placement in a sequestration pen of the microfluidic device because the cell surface is CD45RA⁺, CCR7⁺ and/or CD62L⁺, or both. CD45RA⁺ is considered a marker of naive T lymphocytes (T_{naïve} cells), while CCR7⁺ and CD62L⁺ are considered markers consistent with T_{naïve} status (depending on whether CD45RA⁺ is also present). In some embodiments, a T lymphocyte is selected, at least in part, for placement in a sequestration pen of the microfluidic device because the cell surface lacks at least one marker inconsistent with T_{naïve} status. For example, the T lymphocyte can be selected for lacking a CD45RO⁺, PD-1⁺, PD-L1⁺, CD137⁺, or CD69⁺ cell surface, or any combination thereof. In some embodiments, the T lymphocyte is also selected for having a CD4⁺ cell surface. In some embodiments, the T lymphocyte is also selected for having a CD8⁺ cell surface.

In some embodiments, a T lymphocyte can be selected, at least in part, for placement in a sequestration pen of the microfluidic device because the cell surface is CD45RO⁺, CCR7⁺ and/or CD62L⁺, or both. The combination of CD45RO⁺ with CCR7⁺ and/or CD62L⁺ markers is considered consistent with memory T lymphocytes (e.g., T_{CM} cells). In some embodiments, a T lymphocyte is selected, at least in part, for placement in a sequestration pen of the microfluidic device because the cell surface lacks at least one marker inconsistent with a memory T lymphocyte status. For example, the T lymphocyte can be selected for lacking a CD45RA⁺, PD-1⁺, PD-L1⁺, CD137⁺, or CD69⁺ cell surface, or any combination thereof. In some embodiments, the T lymphocyte is also selected for having a CD4⁺ cell surface. In some embodiments, the T lymphocyte is also selected for having a CD8⁺ cell surface.

In some embodiments, a T lymphocyte can be selected, at least in part, for placement in a sequestration pen of the microfluidic device because the cell surface is CD45RO⁺, PD-1⁺ and/or PD-L1⁺, CD137⁺, or a combination thereof. The presence of CD45RO in conjunction with PD-1, PD-L1, and/or CD137 markers is considered consistent with effector T lymphocytes (T_{EFF} cells). In some embodiments, a T lymphocyte is selected, at least in part, for placement in a sequestration pen of the microfluidic device because the cell surface lacks at least one marker inconsistent with T_{EFF} status. For example, T lymphocytes can be labeled with antibodies to CD45RA, CCR7, CD62L, or any combination thereof, and a T lymphocyte not associated with an antibody to CD45RA, CCR7, CD62L, or a combination thereof can be moved (e.g., using dielectrophoresis) into the sequestration pen of the microfluidic device (e.g., from the microfluidic channel).

In some embodiments, a T lymphocyte is selected, at least in part, for placement in a sequestration pen of the microfluidic device because the cell surface lacks at least one, at least two, or at least three markers inconsistent with T_{naïve} In some embodiments, a T lymphocyte is selected, at least in part, for placement in a sequestration pen of the microfluidic device because the cell surface lacks at least one, at least two, or at least three markers inconsistent with memory T lymphocyte (e.g., T_{CM} or T_{EM}) status. In some embodiments, a T lymphocyte is selected, at least in part, for placement in a sequestration pen of the microfluidic device because the cell surface lacks at least one, at least two, or at least three markers inconsistent with T_{EFF} status.

Accordingly, methods disclosed herein can comprise moving a T lymphocyte into a sequestration pen which is at least one, at least two, at least three, at least four, or all of CD3⁺ CD45RA⁺ CCR7⁺ CD62L⁺ CD45RO⁻, e.g., CD3⁺ CD45RA⁺ CCR7⁺ CD62L⁺; CD3⁺ CD45RA⁺ CCR7⁺ CD45RO⁻; CD3⁺ CD45RA⁺ CD62L⁺ CD45RO⁻; CD45RA⁺ CCR7⁺ CD62L⁺ CD45RO⁻; CD3⁺ CCR7⁺ CD62L⁺ CD45RO⁻; CD3⁺ CD45RA⁺ CCR7⁺; CD3⁺ CD45RA⁺ CD62L⁺; CD45RA⁺ CCR7⁺ CD62L⁺; CD3⁺ CCR7⁺ CD45RO⁻, CD3⁺ CD62L⁺ CD45RO⁻, CCR7⁺ CD62L⁺ CD45RO⁻, CD45RA⁺ CCR7⁺; CD45RA⁺ CD62L⁺; CCR7⁺ CD45RO⁻; CD62L⁺ CD45RO⁻. Any of the foregoing types of T lymphocytes may additionally be CD4⁺ or CD8⁺ and/or at least one, two, three, or all of CD69⁻ PD-1⁻ PD-L1⁻ CD137⁻, e.g., CD69⁻ PD-L1⁻ PD-1⁻, CD69⁻ PD-1⁻ CD137⁻, CD69⁻ PD-L1⁻ CD137⁻, PD-1⁻ PD-L1⁻ CD137⁻, CD69⁻PD-1⁻, CD69⁻PD-L1⁻, CD69⁻ CD137⁻, PD-1⁻ PD-L1⁻, PD-1⁻ CD137⁻, PD-L1⁻ CD137⁻, CD69⁻, PD-1⁻, PD-L1⁻, or CD137⁻.

Methods disclosed herein can comprise moving a T lymphocyte into a sequestration pen which is at least one, at least two, at least three, at least four, or all of CD3⁺ CD45RA⁻ CCR7⁺ CD62L⁺ CD45RO⁺, e.g., CD3⁺ CD45RA⁻ CCR7⁺ CD62L⁺; CD3⁺ CD45RA⁻ CCR7⁺ CD45RO⁺; CD3⁺ CD45RA⁻ CD62L⁺ CD45RO⁺; CD3⁺ CCR7⁺ CD62L⁺ CD45RO⁺; CD45RA⁻ CCR7⁺ CD62L⁺ CD45RO⁺; CD3⁺ CD45RA⁻ CCR7⁺; CD3⁺ CD45RA⁻ CD62L⁺; CD45RA⁻ CCR7⁺ CD62L⁺; CD3⁺ CCR7⁺ CD45RO⁺, CD3⁺ CD62L⁺ CD45RO⁺, CCR7⁺ CD62L⁺ CD45RO⁺, CD45RA⁻ CCR7⁺; CD45RA⁻ CD62L⁺; CCR7⁺ CD45RO⁺; CD62L⁺ CD45RO⁺. Any of the foregoing types of T lymphocytes may additionally be CD4⁺ or CD8⁺ and/or at least one, two, three, or all of CD69⁻ PD-1⁻ PD-L1⁻ CD137⁻, e.g., CD69⁻ PD-L1⁻ PD-1⁻, CD69⁻ PD-1⁻ CD137⁻, CD69⁻ PD-L1⁻ CD137⁻, PD-1⁻ PD-L1⁻ CD137⁻, CD69⁻ PD-1⁻, CD69⁻ PD-L1⁻, CD69⁻ CD137⁻, PD-1⁻ PD-L1⁻, PD-1⁻ CD137⁻, PD-L1⁻ CD137⁻, CD69⁻, PD-1⁻, PD-L1⁻, or CD137⁻.

Methods disclosed herein can comprise moving a T lymphocyte into a sequestration pen which is at least one, at least two, at least three, at least four, or all of CD3⁺ CD45RA⁻ PD-1⁺ CD137⁺ CD45RO⁺, e.g., CD3⁺ CD45RA⁻ PD-1⁺ CD137⁺; CD3⁺ CD45RA⁻ PD-1⁺ CD45RO⁺; CD3⁺ CD45RA⁻ CD137⁺ CD45RO⁺; CD3⁺ PD-1⁺ CD137⁺ CD45RO⁺; CD45RA⁻ PD-1⁺ CD137⁺ CD45RO⁺; CD3⁺ CD45RA⁻ PD-1⁺; CD3⁺ CD45RA⁻ CD137⁺; CD3⁺ PD-1⁺ CD137⁺; CD45RA⁻ PD-1⁺ CD137⁺; CD3⁺ CD45RA⁻ CD45RO⁺; CD3⁺ PD-1⁺ CD45RO⁺; CD45RA⁻ PD-1⁺ CD45RO⁺; CD3⁺ CD137⁺ CD45RO⁺; CD45RA⁻ CD137⁺ CD45RO⁺; PD-1⁺ CD137⁺ CD45RO⁺; CD45RA⁻ PD-1⁺; CD45RA⁻ CD137⁺; CD137⁺ CD45RO⁺; or PD-1⁺ CD45RO⁺. Any of the foregoing types of T lymphocytes may additionally be CD4⁺ or CD8⁺ and/or at least one, two, three, or all of CD69⁺ PD-L1⁺ CCR7⁻ CD62L⁻, e.g., CD69⁺ PD-L1⁺ CCR7⁻; CD69⁺ PD-L1⁺ CD62L⁻; CD69⁺ CCR7⁻ CD62L⁻; PD-L1⁺ CCR7⁻ CD62L⁻; CD69⁺ PD-L1⁺; CD69⁺ CCR7⁻; PD-L1⁺ CCR7⁻; CD69⁺ CD62L⁻; PD-L1⁺ CD62L⁻; CCR7⁻ CD62L⁻; CD69⁺; PD-L1⁺; CCR7⁻; or CD62L⁻.

Methods disclosed herein can comprise introducing a T lymphocyte into a sequestration pen of the microfluidic device, e.g., by dielectrophoresis, by gravity (such as tilting the microfluidic device such that gravity pulls the one or more T lymphocyte into the sequestration pen), by centrifugal force, or by localized flow.

In some embodiments, the methods comprise contacting the one or more T lymphocytes with an activating agent. Such contacting can occur, e.g., before the T lymphocytes are introduced into the microfluidic device, e.g., for at least about 1, 2, 3, 4, 5, 6, 7, 8, 9, or 10 hours before the introduction into the device, or for a period of 0.5 to 2, 2 to 4, 4 to 6, 6 to 8, or 8 to 10 hours. The activating agent can be introduced into the sequestration pen along with the T lymphocytes. Alternatively, or in addition, contacting (e.g., addition of the activating agent) can occur when the T lymphocytes are in the sequestration pen. In some embodiments, an anti-CD3 antibody or agonist is used to contact the lymphocytes. In some embodiments, an anti-CD28 antibody or agonist is used to contact the lymphocytes, e.g., in combination with the anti-CD3 antibody or agonist. Activating agents can be provided in soluble form or attached to beads. For example, beads with attached anti-CD3 antibody or agonist can be used in combination with soluble anti-CD28 antibody or agonist. Alternatively, anti-CD3 antibody or agonist and anti-CD28 antibody or agonist can be used wherein both are attached to beads (e.g., both antibodies can be attached to the same beads or some beads can be attached to anti-CD3 antibodies and other beads can be attached to anti-CD28 antibodies). In some embodiments, at least one activating agent (e.g., antibody) is attached to the surface of a sequestration pen, e.g., an anti-CD3 antibody, such that stimulation occurs when a T lymphocyte is in the pen. In some embodiments, both an anti-CD3 antibody and an anti-CD28 antibody are attached to the surface of a sequestration pen. In some embodiments, an anti-CD3 antibody is attached to the surface of a sequestration pen and at least one T lymphocyte is also contacted with a soluble anti-CD28 antibody while in the sequestration pen. Typically, anti-CD3 and anti-CD28 antibodies when used as activating agents will be agonist antibodies.

In some embodiments, isolated T lymphocytes that undergo expansion in response to being contacted by activating agent exhibit at least 100-fold, 200-fold, 300-fold, 400-fold, 500-fold, 600-fold, 700-fold, 800-fold, 900-fold, 1000-fold expansion, or more.

In some embodiments, T lymphocytes are stimulated with dendritic cells, e.g., autologous dendritic cells. In some embodiments, the dendritic cells are loaded (e.g., pulsed) with one or more antigens, e.g., cancer-derived peptides, such as antigens or peptides isolated from cancer cells autologous with the T lymphocytes. See, e.g., Example 3 below. The dendritic cells can also be autologous with the T lymphocytes. Alternatively, dendritic cells can be loaded (e.g., pulsed) with a synthetic antigen.

In some embodiments, T lymphocytes are stimulated with an antigen, which may be bound to an MHC molecule to form a peptide-MHC complex. The peptide-MHC complexes may be joined together, in the manner of an MHC tetramer structure. The antigen (or antigenic complex) can be attached to a solid support, such as one or more beads. Alternatively, the solid support can be at least one surface of the sequestration pen, and the antigen can be covalently bound to the at least one surface or non-covalently bound (e.g., via a biotin-streptavidin binding interaction).

In some embodiments, culture medium is perfused through the microfluidic channel of the microfluidic device. Perfusion can be intermittent, e.g., in cycles comprising two or three phases at different perfusion rates. In some embodiments, perfusion comprises one or more cycles of a first phase with a perfusion rate of about 0.002-0.1 µl/sec and a second phase of perfusion of about 0.5-10 µl/sec. The cycles can further comprise a third phase, e.g., in which the perfusion is at a rate less than about 0.01 µl/sec, e.g., is substantially stopped. The length of the phase at the higher perfusion rate can be, e.g., about 1-10 minutes, such as 1-2 minutes. The length of the phase(s) at the lower perfusion rate can be, e.g., about 0.5 min to about 3 hours, e.g., about 1 min to about 2 hours. When present, the length of the phase in which the perfusion is at a rate less than about 0.01 µl/sec can be, e.g., about 2-30 or 2-20 minutes, or about 5-10 minutes. Culture medium can be perfused through the flow path of the microfluidic device for a period of at least 24, 48, 72, 96, 120, or more hours, or for a length of time from 0.5 to 10 days, such as 0.5 to 1, 1 to 2, 2 to 3, 3 to 4, 4 to 5, 5 to 7, 6 to 8, 7 to 9, or 8 to 10 days.

In some embodiments, the culture medium is supplemented with a cytokine, e.g., IL7, IL15, IL21, or a combination thereof, such as IL15 and IL21. In some embodiments, the culture medium comprises IL2. The perfusion (which can include an incubation period following active perfusion in which the medium is introduced into the sequestration pens) can be for a period of time sufficient for one or more T lymphocytes to undergo expansion, e.g., at least one, two, three, or more rounds of mitotic cell division. In some embodiments, the culture medium comprises serum, such human AB serum, optionally in combination with FBS or calf serum.

In some embodiments, 20 or fewer, 10 or fewer, 6-10, 5 or fewer, about 5, about 4, about 3, about 2, or 1 T lymphocyte(s) are introduced into the sequestration pen in the microfluidic device. In some embodiments, 20 or fewer, 10 or fewer, 6-10, 5 or fewer, about 5, about 4, about 3, about 2, or 1 T lymphocyte(s) are introduced into a plurality of sequestration pens in the microfluidic device.

The sequestration pen can comprise a coating, e.g., so that a T lymphocyte in the pen is not in direct contact with a hard surface. In some embodiments, the pen surface is coated with dextran or polyethylene glycol. In some embodiments, the sequestration pen has a volume of about 5x10⁵ to about 5x10⁶ cubic microns.

In some embodiments, proliferation rate of one or more T lymphocytes is monitored. For example, this can be done during perfusion by observing the frequency of cell division in a given sequestration pen, e.g., by periodically observing the number of cells in the pen.

In some embodiments, expression of one or more cytokines (e.g., INFgamma, TNFalpha, IL2, or a combination thereof) by a T lymphocyte is monitored. In some embodiments, expression of one or more regulatory T cell markers, e.g., CTLA4, is monitored.

Monitoring as described above can be performed, e.g., following expansion of T lymphocytes in sequestration pens. For example, a sample of expanded T lymphocytes can be characterized with respect to expression of regulatory T cell markers and/or cytokines using on-chip expression assays, which have been described, e.g., in US Patent Application Publication Nos. 2015/0151298 and 2015/0165436 and US Patent Application Serial No. 15/372,094 (filed December 7, 2016), the contents of each of which is incorporated herein by reference.

In some embodiments, following expansion, T lymphocytes are exported from the microfluidic device. In some embodiments, a sample of expanded T lymphocytes is exported from the microfluidic device for genomic profiling, e.g., genomic sequence analysis and/or expression analysis (e.g., RNA-Seq). In some embodiments, T lymphocytes are selected for a downstream use such as cancer treatment based on genomic profiling results, e.g., absence of loss of function mutations, cytokine expression, or the like. In some embodiments, T lymphocytes are selectively exported based at least in part on having a proliferation rate greater than or equal to a predetermined threshold, e.g., having shown at least a threshold level of proliferation, such as having undergone at least 100-fold expansion in a period of less than or equal to about one week. Selective export can alternatively or in addition be based at least in part on expression of one or more cytokines, such as INFgamma, TNFalpha, IL2, or a combination thereof. Selective export can alternatively or in addition be based at least in part on expression of one or more regulatory T cell markers, such as CTLA4 or the like.

In some embodiments, T lymphocytes exported from the microfluidic device are used to treat cancer, e.g., by introducing them into a subject, such as the subject from whom the T lymphocytes were obtained. The subject can be a mammal, e.g., a human or a non-human mammal.

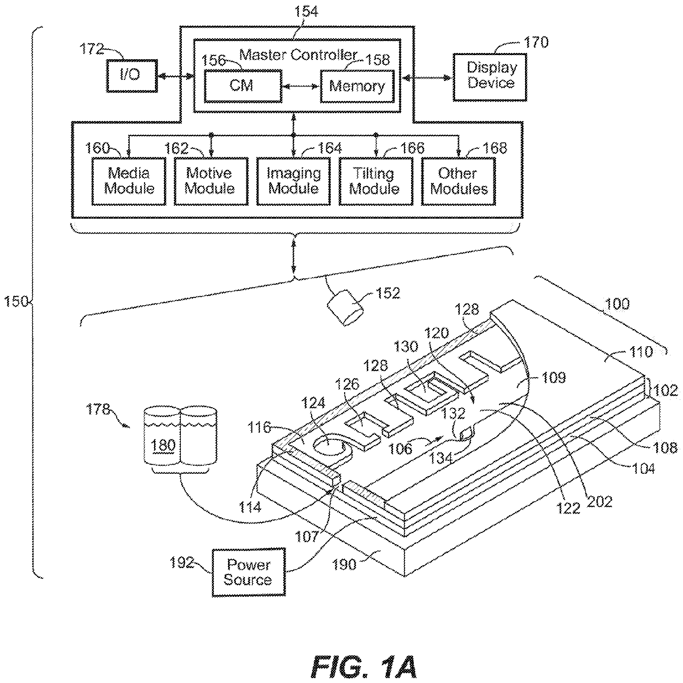

**Microfluidic devices and systems for operating and observing such devices.** Figure 1A illustrates an example of a microfluidic device 100 and a system 150 which can be used for selecting, activating, expanding, and/or cloning T lymphocytes. A perspective view of the microfluidic device 100 is shown having a partial cut-away of its cover 110 to provide a partial view into the microfluidic device 100. The microfluidic device 100 generally comprises a microfluidic circuit 120 comprising a flow path 106 through which a fluidic medium 180 can flow, optionally carrying one or more micro-objects (not shown) into and/or through the microfluidic circuit 120. Although a single microfluidic circuit 120 is illustrated in Figure 1A, suitable microfluidic devices can include a plurality (e.g., 2 or 3) of such microfluidic circuits. Regardless, the microfluidic device 100 can be configured to be a nanofluidic device. As illustrated in Figure 1A, the microfluidic circuit 120 may include a plurality of microfluidic sequestration pens 124, 126, 128, and 130, where each sequestration pens may have one or more openings in fluidic communication with flow path 106. In some embodiments of the device of Figure 1A, the sequestration pens may have only a single opening in fluidic communication with the flow path 106. As discussed further below, the microfluidic sequestration pens comprise various features and structures that have been optimized for retaining micro-objects in the microfluidic device, such as microfluidic device 100, even when a medium 180 is flowing through the flow path 106. Before turning to the foregoing, however, a brief description of microfluidic device 100 and system 150 is provided.

As generally illustrated in Figure 1A, the microfluidic circuit 120 is defined by an enclosure 102. Although the enclosure 102 can be physically structured in different configurations, in the example shown in Figure 1A the enclosure 102 is depicted as comprising a support structure 104 (e.g., a base), a microfluidic circuit structure 108, and a cover 110. The support structure 104, microfluidic circuit structure 108, and cover 110 can be attached to each other. For example, the microfluidic circuit structure 108 can be disposed on an inner surface 109 of the support structure 104, and the cover 110 can be disposed over the microfluidic circuit structure 108. Together with the support structure 104 and cover 110, the microfluidic circuit structure 108 can define the elements of the microfluidic circuit 120.

The support structure 104 can be at the bottom and the cover 110 at the top of the microfluidic circuit 120 as illustrated in Figure 1A. Alternatively, the support structure 104 and the cover 110 can be configured in other orientations. For example, the support structure 104 can be at the top and the cover 110 at the bottom of the microfluidic circuit 120. Regardless, there can be one or more ports 107 each comprising a passage into or out of the enclosure 102. Examples of a passage include a valve, a gate, a pass-through hole, or the like. As illustrated, port 107 is a pass-through hole created by a gap in the microfluidic circuit structure 108. However, the port 107 can be situated in other components of the enclosure 102, such as the cover 110. Only one port 107 is illustrated in Figure 1A but the microfluidic circuit 120 can have two or more ports 107. For example, there can be a first port 107 that functions as an inlet for fluid entering the microfluidic circuit 120, and there can be a second port 107 that functions as an outlet for fluid exiting the microfluidic circuit 120. Whether a port 107 function as an inlet or an outlet can depend upon the direction that fluid flows through flow path 106.

The support structure 104 can comprise one or more electrodes (not shown) and a substrate or a plurality of interconnected substrates. For example, the support structure 104 can comprise one or more semiconductor substrates, each of which is electrically connected to an electrode (e.g., all or a subset of the semiconductor substrates can be electrically connected to a single electrode). The support structure 104 can further comprise a printed circuit board assembly ("PCBA"). For example, the semiconductor substrate(s) can be mounted on a PCBA.

The microfluidic circuit structure 108 can define circuit elements of the microfluidic circuit 120. Such circuit elements can comprise spaces or regions that can be fluidly interconnected when microfluidic circuit 120 is filled with fluid, such as flow paths (which may include or be one or more flow channels), chambers, pens, traps, and the like. In the microfluidic circuit 120 illustrated in Figure 1A, the microfluidic circuit structure 108 comprises a frame 114 and a microfluidic circuit material 116. The frame 114 can partially or completely enclose the microfluidic circuit material 116. The frame 114 can be, for example, a relatively rigid structure substantially surrounding the microfluidic circuit material 116. For example, the frame 114 can comprise a metal material.

The microfluidic circuit material 116 can be patterned with cavities or the like to define circuit elements and interconnections of the microfluidic circuit 120. The microfluidic circuit material 116 can comprise a flexible material, such as a flexible polymer (e.g. rubber, plastic, elastomer, silicone, polydimethylsiloxane ("PDMS"), or the like), which can be gas permeable. Other examples of materials that can compose microfluidic circuit material 116 include molded glass, an etchable material such as silicone (e.g. photo-patternable silicone or "PPS"), photo-resist (e.g., SU8), or the like. In some embodiments, such materials-and thus the microfluidic circuit material 116-can be rigid and/or substantially impermeable to gas. Regardless, microfluidic circuit material 116 can be disposed on the support structure 104 and inside the frame 114.

The cover 110 can be an integral part of the frame 114 and/or the microfluidic circuit material 116. Alternatively, the cover 110 can be a structurally distinct element, as illustrated in Figure 1A. The cover 110 can comprise the same or different materials than the frame 114 and/or the microfluidic circuit material 116. Similarly, the support structure 104 can be a separate structure from the frame 114 or microfluidic circuit material 116 as illustrated, or an integral part of the frame 114 or microfluidic circuit material 116. Likewise, the frame 114 and microfluidic circuit material 116 can be separate structures as shown in Figure 1A or integral portions of the same structure.

In some embodiments, the cover 110 can comprise a rigid material. The rigid material may be glass or a material with similar properties. In some embodiments, the cover 110 can comprise a deformable material. The deformable material can be a polymer, such as PDMS. In some embodiments, the cover 110 can comprise both rigid and deformable materials. For example, one or more portions of cover 110 (e.g., one or more portions positioned over sequestration pens 124, 126, 128, 130) can comprise a deformable material that interfaces with rigid materials of the cover 110. In some embodiments, the cover 110 can further include one or more electrodes. The one or more electrodes can comprise a conductive oxide, such as indium-tin-oxide (ITO), which may be coated on glass or a similarly insulating material. Alternatively, the one or more electrodes can be flexible electrodes, such as single-walled nanotubes, multi-walled nanotubes, nanowires, clusters of electrically conductive nanoparticles, or combinations thereof, embedded in a deformable material, such as a polymer (e.g., PDMS). Flexible electrodes that can be used in microfluidic devices have been described, for example, in U.S. 2012/0325665 (Chiou et al.), the contents of which are incorporated herein by reference. In some embodiments, the cover 110 can be modified (e.g., by conditioning all or part of a surface that faces inward toward the microfluidic circuit 120) to support cell adhesion, viability and/or growth. The modification may include a coating of a synthetic or natural polymer. In some embodiments, the cover 110 and/or the support structure 104 can be transparent to light. The cover 110 may also include at least one material that is gas permeable (e.g., PDMS or PPS).

Figure 1A also shows a system 150 for operating and controlling microfluidic devices, such as microfluidic device 100. System 150 includes an electrical power source 192, an imaging device 194 (incorporated within imaging module 164, where device 194 is not illustrated in Figure 1A, per se), and a tilting device 190 (part of tilting module 166, where device 190 is not illustrated in Figure 1A).

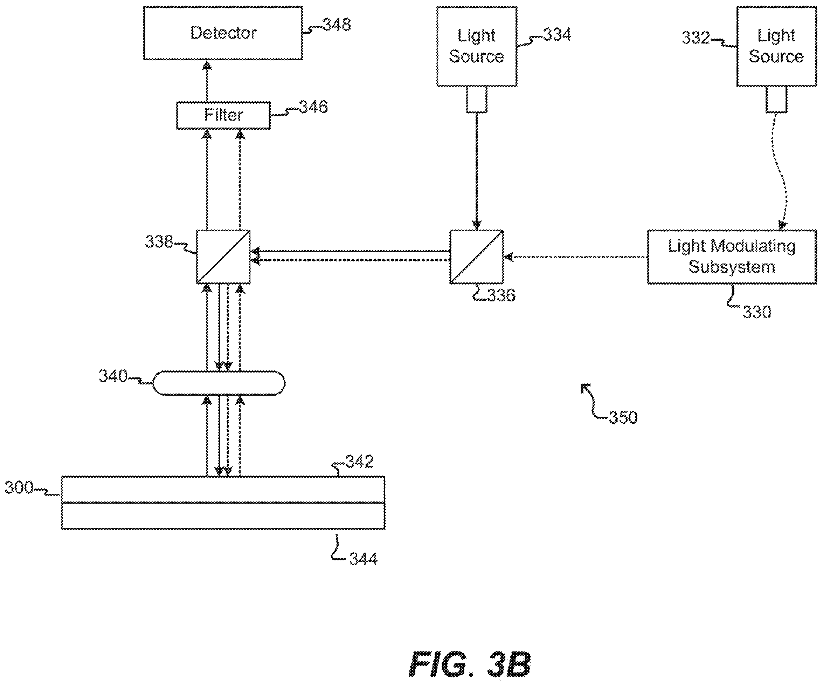

The electrical power source 192 can provide electric power to the microfluidic device 100 and/or tilting device 190, providing biasing voltages or currents as needed. The electrical power source 192 can, for example, comprise one or more alternating current (AC) and/or direct current (DC) voltage or current sources. The imaging device 194 (part of imaging module 164, discussed below) can comprise a device, such as a digital camera, for capturing images inside microfluidic circuit 120. In some instances, the imaging device 194 further comprises a detector having a fast frame rate and/or high sensitivity (e.g. for low light applications). The imaging device 194 can also include a mechanism for directing stimulating radiation and/or light beams into the microfluidic circuit 120 and collecting radiation and/or light beams reflected or emitted from the microfluidic circuit 120 (or micro-objects contained therein). The emitted light beams may be in the visible spectrum and may, e.g., include fluorescent emissions. The reflected light beams may include reflected emissions originating from an LED or a wide spectrum lamp, such as a mercury lamp (e.g. a high pressure mercury lamp) or a Xenon arc lamp. As discussed with respect to Figure 3B, the imaging device 194 may further include a microscope (or an optical train), which may or may not include an eyepiece.

System 150 further comprises a tilting device 190 (part of tilting module 166, discussed below) configured to rotate a microfluidic device 100 about one or more axes of rotation. In some embodiments, the tilting device 190 is configured to support and/or hold the enclosure 102 comprising the microfluidic circuit 120 about at least one axis such that the microfluidic device 100 (and thus the microfluidic circuit 120) can be held in a level orientation (i.e. at 0° relative to x- and y-axes), a vertical orientation (i.e. at 90° relative to the x-axis and/or the y-axis), or any orientation therebetween. The orientation of the microfluidic device 100 (and the microfluidic circuit 120) relative to an axis is referred to herein as the "tilt" of the microfluidic device 100 (and the microfluidic circuit 120). For example, the tilting device 190 can tilt the microfluidic device 100 at 0.1°, 0.2°, 0.3°, 0.4°, 0.5°, 0.6°, 0.7°, 0.8°, 0.9°, 1°, 2°, 3°, 4°, 5°, 10°, 15°, 20°, 25°, 30°, 35°, 40°, 45°, 50°, 55°, 60°, 65°, 70°, 75°, 80°, 90° relative to the x-axis or any degree therebetween. The level orientation (and thus the x- and y-axes) is defined as normal to a vertical axis defined by the force of gravity. The tilting device can also tilt the microfluidic device 100 (and the microfluidic circuit 120) to any degree greater than 90° relative to the x-axis and/or y-axis, or tilt the microfluidic device 100 (and the microfluidic circuit 120) 180° relative to the x-axis or the y-axis in order to fully invert the microfluidic device 100 (and the microfluidic circuit 120). Similarly, in some embodiments, the tilting device 190 tilts the microfluidic device 100 (and the microfluidic circuit 120) about an axis of rotation defined by flow path 106 or some other portion of microfluidic circuit 120.

In some instances, the microfluidic device 100 is tilted into a vertical orientation such that the flow path 106 is positioned above or below one or more sequestration pens. The term "above" as used herein denotes that the flow path 106 is positioned higher than the one or more sequestration pens on a vertical axis defined by the force of gravity (i.e. an object in a sequestration pen above a flow path 106 would have a higher gravitational potential energy than an object in the flow path). The term "below" as used herein denotes that the flow path 106 is positioned lower than the one or more sequestration pens on a vertical axis defined by the force of gravity (i.e. an object in a sequestration pen below a flow path 106 would have a lower gravitational potential energy than an object in the flow path).

In some instances, the tilting device 190 tilts the microfluidic device 100 about an axis that is parallel to the flow path 106. Moreover, the microfluidic device 100 can be tilted to an angle of less than 90° such that the flow path 106 is located above or below one or more sequestration pens without being located directly above or below the sequestration pens. In other instances, the tilting device 190 tilts the microfluidic device 100 about an axis perpendicular to the flow path 106. In still other instances, the tilting device 190 tilts the microfluidic device 100 about an axis that is neither parallel nor perpendicular to the flow path 106.

System 150 can further include a media source 178. The media source 178 (e.g., a container, reservoir, or the like) can comprise multiple sections or containers, each for holding a different fluidic medium 180. Thus, the media source 178 can be a device that is outside of and separate from the microfluidic device 100, as illustrated in Figure 1A. Alternatively, the media source 178 can be located in whole or in part inside the enclosure 102 of the microfluidic device 100. For example, the media source 178 can comprise reservoirs that are part of the microfluidic device 100.

Figure 1A also illustrates simplified block diagram depictions of examples of control and monitoring equipment 152 that constitute part of system 150 and can be utilized in conjunction with a microfluidic device 100. As shown, examples of such control and monitoring equipment 152 include a master controller 154 comprising a media module 160 for controlling the media source 178, a motive module 162 for controlling movement and/or selection of micro-objects (not shown) and/or medium (e.g., droplets of medium) in the microfluidic circuit 120, an imaging module 164 for controlling an imaging device 194 (e.g., a camera, microscope, light source or any combination thereof) for capturing images (e.g., digital images), and a tilting module 166 for controlling a tilting device 190. The control equipment 152 can also include other modules 168 for controlling, monitoring, or performing other functions with respect to the microfluidic device 100. As shown, the equipment 152 can further include a display device 170 and an input/output device 172.

The master controller 154 can comprise a control module 156 and a digital memory 158. The control module 156 can comprise, for example, a digital processor configured to operate in accordance with machine executable instructions (e.g., software, firmware, source code, or the like) stored as non-transitory data or signals in the memory 158. Alternatively, or in addition, the control module 156 can comprise hardwired digital circuitry and/or analog circuitry. The media module 160, motive module 162, imaging module 164, tilting module 166, and/or other modules 168 can be similarly configured. Thus, functions, processes acts, actions, or steps of a process discussed herein as being performed with respect to the microfluidic device 100 or any other microfluidic apparatus can be performed by any one or more of the master controller 154, media module 160, motive module 162, imaging module 164, tilting module 166, and/or other modules 168 configured as discussed above. Similarly, the master controller 154, media module 160, motive module 162, imaging module 164, tilting module 166, and/or other modules 168 may be communicatively coupled to transmit and receive data used in any function, process, act, action or step discussed herein.

The media module 160 controls the media source 178. For example, the media module 160 can control the media source 178 to input a selected fluidic medium 180 into the enclosure 102 (e.g., through an inlet port 107). The media module 160 can also control removal of media from the enclosure 102 (e.g., through an outlet port (not shown)). One or more media can thus be selectively input into and removed from the microfluidic circuit 120. The media module 160 can also control the flow of fluidic medium 180 in the flow path 106 inside the microfluidic circuit 120. For example, in some embodiments media module 160 stops the flow of media 180 in the flow path 106 and through the enclosure 102 prior to the tilting module 166 causing the tilting device 190 to tilt the microfluidic device 100 to a desired angle of incline.

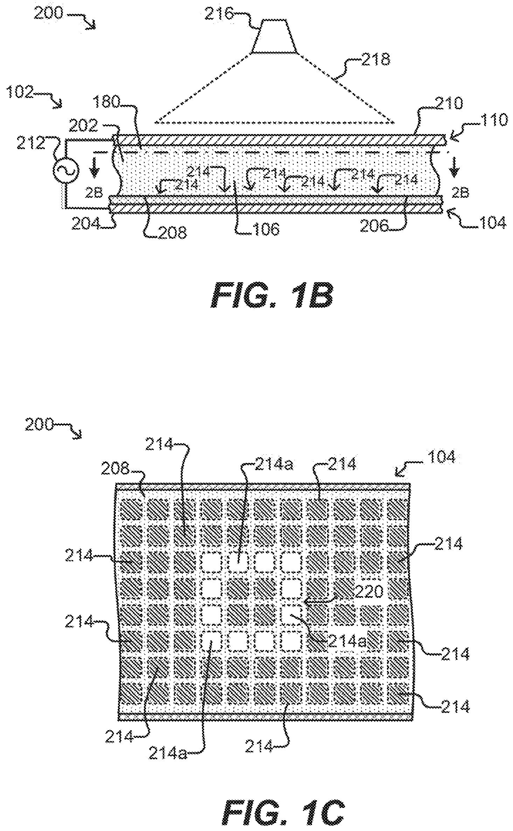

The motive module 162 can be configured to control selection, trapping, and movement of micro-objects (not shown) in the microfluidic circuit 120. As discussed below with respect to Figures 1B and 1C, the enclosure 102 can comprise a dielectrophoresis (DEP), optoelectronic tweezers (OET) and/or opto-electrowetting (OEW) configuration (not shown in Figure 1A), and the motive module 162 can control the activation of electrodes and/or transistors (e.g., phototransistors) to select and move micro-objects (not shown) and/or droplets of medium (not shown) in the flow path 106 and/or sequestration pens 124, 126, 128, 130.

The imaging module 164 can control the imaging device 194. For example, the imaging module 164 can receive and process image data from the imaging device 194. Image data from the imaging device 194 can comprise any type of information captured by the imaging device 194 (e.g., the presence or absence of micro-objects, droplets of medium, accumulation of label, such as fluorescent label, etc.). Using the information captured by the imaging device 194, the imaging module 164 can further calculate the position of objects (e.g., micro-objects, droplets of medium) and/or the rate of motion of such objects within the microfluidic device 100.

The tilting module 166 can control the tilting motions of tilting device 190. Alternatively, or in addition, the tilting module 166 can control the tilting rate and timing to optimize transfer of micro-objects to the one or more sequestration pens via gravitational forces. The tilting module 166 is communicatively coupled with the imaging module 164 to receive data describing the motion of micro-objects and/or droplets of medium in the microfluidic circuit 120. Using this data, the tilting module 166 may adjust the tilt of the microfluidic circuit 120 in order to adjust the rate at which micro-objects and/or droplets of medium move in the microfluidic circuit 120. The tilting module 166 may also use this data to iteratively adjust the position of a micro-object and/or droplet of medium in the microfluidic circuit 120.

In the example shown in Figure 1A, the microfluidic circuit 120 is illustrated as comprising a microfluidic channel 122 and sequestration pens 124, 126, 128, 130. Each pen comprises an opening to channel 122, but otherwise is enclosed such that the pens can substantially isolate micro-objects inside the pen from fluidic medium 180 and/or micro-objects in the flow path 106 of channel 122 or in other pens. The walls of the sequestration pen extend from the inner surface 109 of the base to the inside surface of the cover 110 to provide enclosure. The opening of the pen to the microfluidic channel 122 is oriented at an angle to the flow 106 of fluidic medium 180 such that flow 106 is not directed into the pens. The flow may be tangential or orthogonal to the plane of the opening of the pen. In some instances, pens 124, 126, 128, 130 are configured to physically corral one or more micro-objects within the microfluidic circuit 120. Sequestration pens in accordance with the present disclosure can comprise various shapes, surfaces and features that are optimized for use with DEP, OET, OEW, fluid flow, gravitational forces, and/or centrifugal forces, as will be discussed and shown in detail below.

The microfluidic circuit 120 may comprise any number of microfluidic sequestration pens. Although five sequestration pens are shown, microfluidic circuit 120 may have fewer or more sequestration pens. As shown, microfluidic sequestration pens 124, 126, 128, and 130 of microfluidic circuit 120 each comprise differing features and shapes which may provide one or more benefits useful for maintaining, isolating, assaying, stimulating (e.g., activating), or culturing biological micro-objects. In some embodiments, the microfluidic circuit 120 comprises a plurality of identical microfluidic sequestration pens.

In the embodiment illustrated in Figure 1A, a single channel 122 and flow path 106 is shown. However, other embodiments may contain multiple channels 122, each configured to comprise a flow path 106. The microfluidic circuit 120 further comprises an inlet valve or port 107 in fluid communication with the flow path 106 and fluidic medium 180, whereby fluidic medium 180 can access channel 122 via the inlet port 107. In some instances, the flow path 106 comprises a single path. In some instances, the single path is arranged in a zigzag pattern whereby the flow path 106 travels across the microfluidic device 100 two or more times in alternating directions.

In some instances, microfluidic circuit 120 comprises a plurality of parallel channels 122 and flow paths 106, wherein the fluidic medium 180 within each flow path 106 flows in the same direction. In some instances, the fluidic medium within each flow path 106 flows in at least one of a forward or reverse direction. In some instances, a plurality of sequestration pens is configured (e.g., relative to a channel 122) such that the sequestration pens can be loaded with target micro-objects in parallel.

In some embodiments, microfluidic circuit 120 further comprises one or more micro-object traps 132. The traps 132 are generally formed in a wall forming the boundary of a channel 122, and may be positioned opposite an opening of one or more of the microfluidic sequestration pens 124, 126, 128, 130. In some embodiments, the traps 132 are configured to receive or capture a single micro-object from the flow path 106. In some embodiments, the traps 132 are configured to receive or capture a plurality of micro-objects from the flow path 106. In some instances, the traps 132 comprise a volume approximately equal to the volume of a single target micro-object.

The traps 132 may further comprise an opening which is configured to assist the flow of targeted micro-objects into the traps 132. In some instances, the traps 132 comprise an opening having a height and width that is approximately equal to the dimensions of a single target micro-object, whereby larger micro-objects are prevented from entering into the micro-object trap. The traps 132 may further comprise other features configured to assist in retention of targeted micro-objects within the trap 132. In some instances, the trap 132 is aligned with and situated on the opposite side of a channel 122 relative to the opening of a microfluidic sequestration pen, such that upon tilting the microfluidic device 100 about an axis parallel to the microfluidic channel 122, the trapped micro-object exits the trap 132 at a trajectory that causes the micro-object to fall into the opening of the sequestration pen. In some instances, the trap 132 comprises a side passage 134 that is smaller than the target micro-object in order to facilitate flow through the trap 132 and thereby increase the likelihood of capturing a micro-object in the trap 132.

In some embodiments, dielectrophoretic (DEP) forces are applied across the fluidic medium 180 (e.g., in the flow path and/or in the sequestration pens) via one or more electrodes (not shown) to manipulate, transport, separate and sort micro-objects located therein. For example, in some embodiments, DEP forces are applied to one or more portions of microfluidic circuit 120 in order to transfer a single micro-object from the flow path 106 into a desired microfluidic sequestration pen. In some embodiments, DEP forces are used to prevent a micro-object within a sequestration pen (e.g., sequestration pen 124, 126, 128, or 130) from being displaced therefrom. Further, in some embodiments, DEP forces are used to selectively remove a micro-object from a sequestration pen that was previously collected in accordance with the embodiments of the current disclosure. In some embodiments, the DEP forces comprise optoelectronic tweezer (OET) forces.

In other embodiments, optoelectrowetting (OEW) forces are applied to one or more positions in the support structure 104 (and/or the cover 110) of the microfluidic device 100 (e.g., positions helping to define the flow path and/or the sequestration pens) via one or more electrodes (not shown) to manipulate, transport, separate and sort droplets located in the microfluidic circuit 120. For example, in some embodiments, OEW forces are applied to one or more positions in the support structure 104 (and/or the cover 110) in order to transfer a single droplet from the flow path 106 into a desired microfluidic sequestration pen. In some embodiments, OEW forces are used to prevent a droplet within a sequestration pen (e.g., sequestration pen 124, 126, 128, or 130) from being displaced therefrom. Further, in some embodiments, OEW forces are used to selectively remove a droplet from a sequestration pen that was previously collected in accordance with the embodiments of the current disclosure.

In some embodiments, DEP and/or OEW forces are combined with other forces, such as flow and/or gravitational force, so as to manipulate, transport, separate and sort micro-objects and/or droplets within the microfluidic circuit 120. For example, the enclosure 102 can be tilted (e.g., by tilting device 190) to position the flow path 106 and micro-objects located therein above the microfluidic sequestration pens, and the force of gravity can transport the micro-objects and/or droplets into the pens. In some embodiments, the DEP and/or OEW forces can be applied prior to the other forces. In other embodiments, the DEP and/or OEW forces can be applied after the other forces. In still other instances, the DEP and/or OEW forces can be applied at the same time as the other forces or in an alternating manner with the other forces.

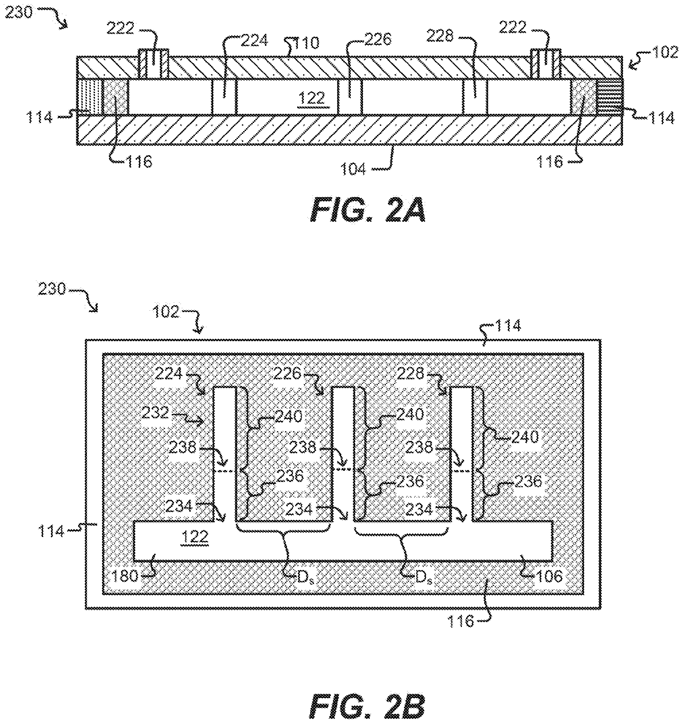

Figures 1B, 1C, and 2A-2H illustrates various embodiments of microfluidic devices that can be used in the practice of the embodiments of the present disclosure. Figure 1B depicts an embodiment in which the microfluidic device 200 is configured as an optically-actuated electrokinetic device. A variety of optically-actuated electrokinetic devices are known in the art, including devices having an optoelectronic tweezer (OET) configuration and devices having an opto-electrowetting (OEW) configuration. Examples of suitable OET configurations are illustrated in the following U.S. patent documents, each of which is incorporated herein by reference in its entirety: U.S. Patent No. RE 44,711 (Wu et al.) (originally issued as U.S. Patent No. 7,612,355); and U.S. Patent No. 7,956,339 (Ohta et al.). Examples of OEW configurations are illustrated in U.S. Patent No. 6,958,132 (Chiou et al.) and U.S. Patent Application Publication No. 2012/0024708 (Chiou et al.), both of which are incorporated by reference herein in their entirety. Yet another example of an optically-actuated electrokinetic device includes a combined OET/OEW configuration, examples of which are shown in U.S. Patent Publication Nos. 20150306598 (Khandros et al.) and 20150306599 (Khandros et al.) and their corresponding PCT Publications WO2015/164846 and WO2015/164847, all of which are incorporated herein by reference in their entirety.

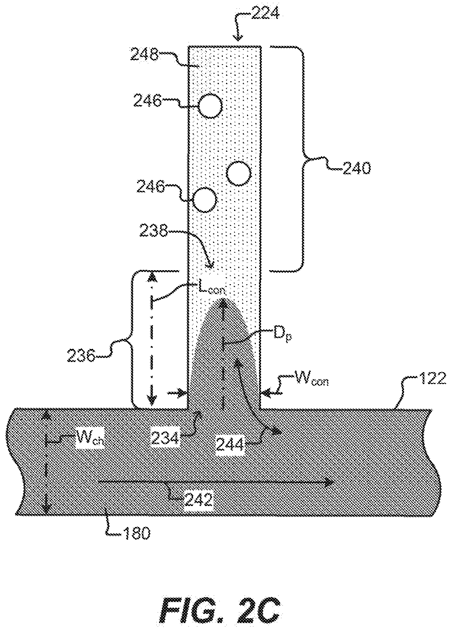

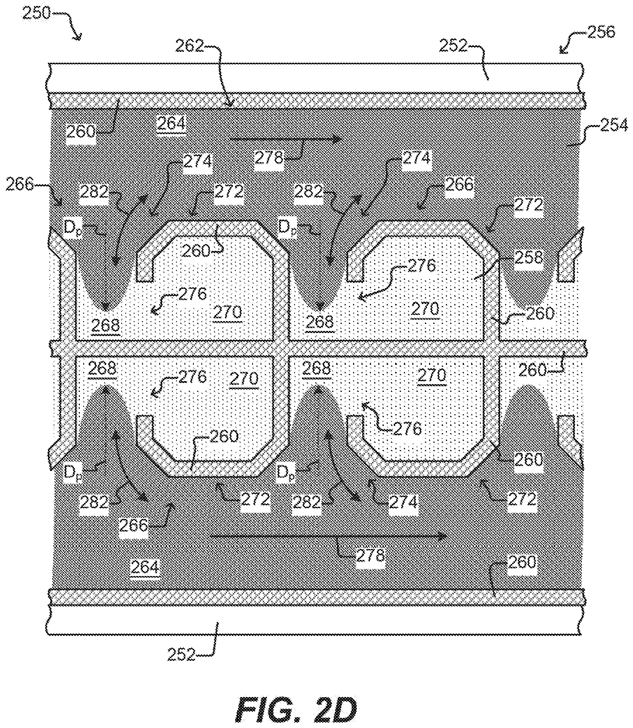

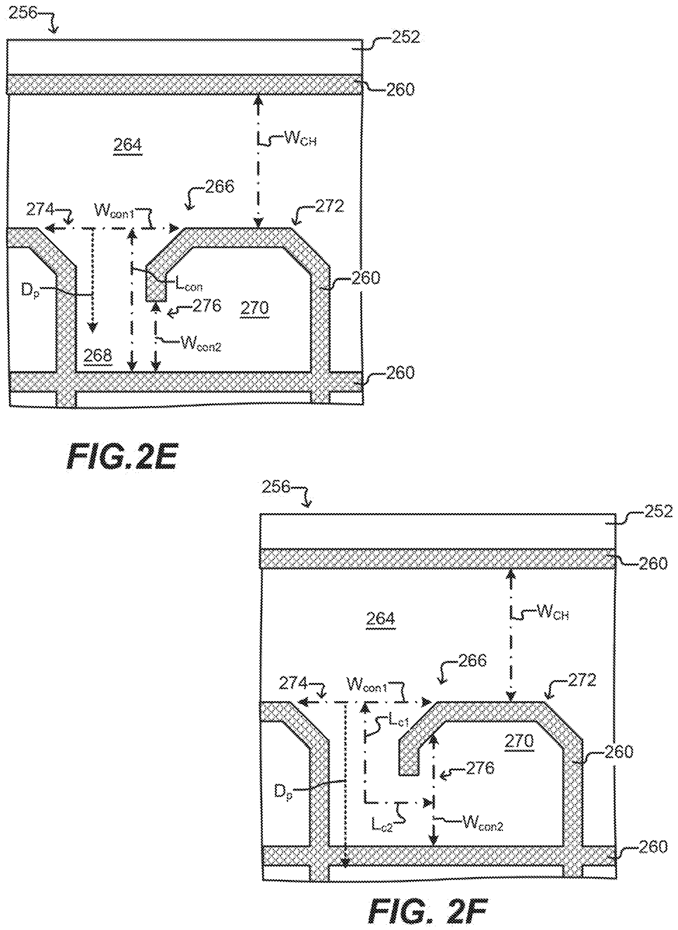

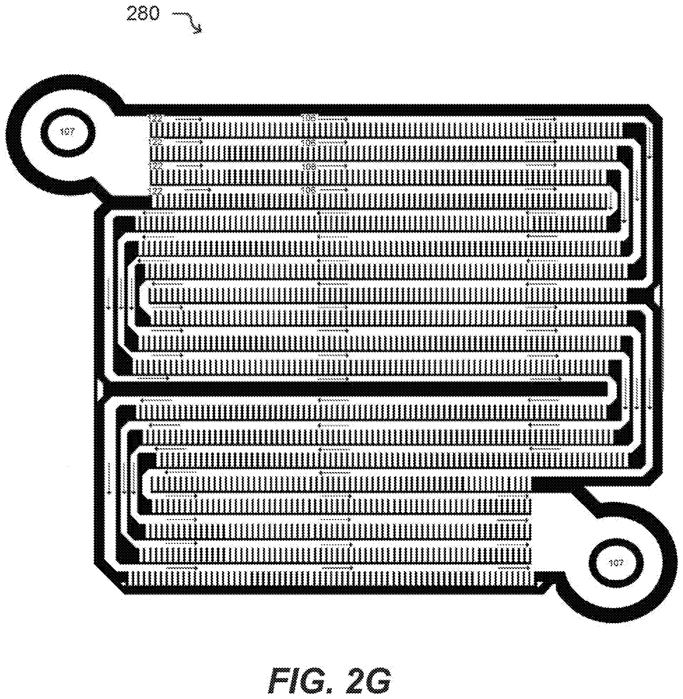

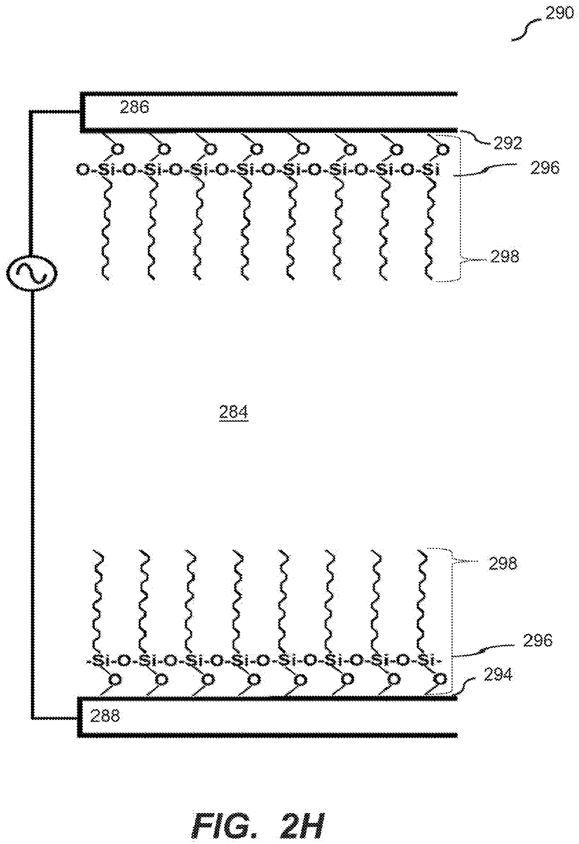

Examples of microfluidic devices having pens in which biological micro-objects can be placed, cultured, and/or monitored have been described, for example, in US 2014/0116881 (application no. 14/060,117, filed October 22, 2013), US 2015/0151298 (application no. 14/520,568, filed October 22, 2014), and US 2015/0165436 (application no. 14/521,447, filed October 22, 2014), each of which is incorporated herein by reference in its entirety. US application nos. 14/520,568 and 14/521,447 also describe exemplary methods of analyzing secretions of cells cultured in a microfluidic device. Each of the foregoing applications further describes microfluidic devices configured to produce dielectrophoretic (DEP) forces, such as optoelectronic tweezers (OET) or configured to provide opto-electro wetting (OEW). For example, the optoelectronic tweezers device illustrated in Figure 2 of US 2014/0116881 is an example of a device that can be utilized in embodiments of the present disclosure to select and move an individual biological micro-object or a group of biological micro-objects.

**Microfluidic device motive configurations.** As described above, the control and monitoring equipment of the system can comprise a motive module for selecting and moving objects, such as micro-objects or droplets, in the microfluidic circuit of a microfluidic device. The microfluidic device can have a variety of motive configurations, depending upon the type of object being moved and other considerations. For example, a dielectrophoresis (DEP) configuration can be utilized to select and move micro-objects in the microfluidic circuit. Thus, the support structure 104 and/or cover 110 of the microfluidic device 100 can comprise a DEP configuration for selectively inducing DEP forces on micro-objects in a fluidic medium 180 in the microfluidic circuit 120 and thereby select, capture, and/or move individual micro-objects or groups of micro-objects. Alternatively, the support structure 104 and/or cover 110 of the microfluidic device 100 can comprise an electrowetting (EW) configuration for selectively inducing EW forces on droplets in a fluidic medium 180 in the microfluidic circuit 120 and thereby select, capture, and/or move individual droplets or groups of droplets.

One example of a microfluidic device 200 comprising a DEP configuration is illustrated in Figures 1B and 1C. While for purposes of simplicity Figures 1B and 1C show a side cross-sectional view and a top cross-sectional view, respectively, of a portion of an enclosure 102 of the microfluidic device 200 having a region/chamber 202, it should be understood that the region/chamber 202 may be part of a fluidic circuit element having a more detailed structure, such as a growth chamber, a sequestration pen, a flow path, or a flow channel. Furthermore, the microfluidic device 200 may include other fluidic circuit elements. For example, the microfluidic device 200 can include a plurality of growth chambers or sequestration pens and/or one or more flow regions or flow channels, such as those described herein with respect to microfluidic device 100. A DEP configuration may be incorporated into any such fluidic circuit elements of the microfluidic device 200, or select portions thereof. It should be further appreciated that any of the above or below described microfluidic device components and system components may be incorporated in and/or used in combination with the microfluidic device 200. For example, system 150 including control and monitoring equipment 152, described above, may be used with microfluidic device 200, including one or more of the media module 160, motive module 162, imaging module 164, tilting module 166, and other modules 168.

As seen in Figure 1B, the microfluidic device 200 includes a support structure 104 having a bottom electrode 204 and an electrode activation substrate 206 overlying the bottom electrode 204, and a cover 110 having a top electrode 210, with the top electrode 210 spaced apart from the bottom electrode 204. The top electrode 210 and the electrode activation substrate 206 define opposing surfaces of the region/chamber 202. A medium 180 contained in the region/chamber 202 thus provides a resistive connection between the top electrode 210 and the electrode activation substrate 206. A power source 212 configured to be connected to the bottom electrode 204 and the top electrode 210 and create a biasing voltage between the electrodes, as required for the generation of DEP forces in the region/chamber 202, is also shown. The power source 212 can be, for example, an alternating current (AC) power source.

In certain embodiments, the microfluidic device 200 illustrated in Figures 1B and 1C can have an optically-actuated DEP configuration. Accordingly, changing patterns of light 218 from the light source 216, which may be controlled by the motive module 162, can selectively activate and deactivate changing patterns of DEP electrodes at regions 214 of the inner surface 208 of the electrode activation substrate 206. (Hereinafter the regions 214 of a microfluidic device having a DEP configuration are referred to as "DEP electrode regions.") As illustrated in Figure 1C, a light pattern 218 directed onto the inner surface 208 of the electrode activation substrate 206 can illuminate select DEP electrode regions 214a (shown in white) in a pattern, such as a square. The non-illuminated DEP electrode regions 214 (cross-hatched) are hereinafter referred to as "dark" DEP electrode regions 214. The relative electrical impedance through the DEP electrode activation substrate 206 (i.e., from the bottom electrode 204 up to the inner surface 208 of the electrode activation substrate 206 which interfaces with the medium 180 in the flow path 106) is greater than the relative electrical impedance through the medium 180 in the region/chamber 202 (i.e., from the inner surface 208 of the electrode activation substrate 206 to the top electrode 210 of the cover 110) at each dark DEP electrode region 214. An illuminated DEP electrode region 214a, however, exhibits a reduced relative impedance through the electrode activation substrate 206 that is less than the relative impedance through the medium 180 in the region/chamber 202 at each illuminated DEP electrode region 214a.

With the power source 212 activated, the foregoing DEP configuration creates an electric field gradient in the fluidic medium 180 between illuminated DEP electrode regions 214a and adjacent dark DEP electrode regions 214, which in turn creates local DEP forces that attract or repel nearby micro-objects (not shown) in the fluidic medium 180. DEP electrodes that attract or repel micro-objects in the fluidic medium 180 can thus be selectively activated and deactivated at many different such DEP electrode regions 214 at the inner surface 208 of the region/chamber 202 by changing light patterns 218 projected from a light source 216 into the microfluidic device 200. Whether the DEP forces attract or repel nearby micro-objects can depend on such parameters as the frequency of the power source 212 and the dielectric properties of the medium 180 and/or micro-objects (not shown).

The square pattern 220 of illuminated DEP electrode regions 214a illustrated in Figure 1C is an example only. Any pattern of the DEP electrode regions 214 can be illuminated (and thereby activated) by the pattern of light 218 projected into the microfluidic device 200, and the pattern of illuminated/activated DEP electrode regions 214 can be repeatedly changed by changing or moving the light pattern 218.

In some embodiments, the electrode activation substrate 206 can comprise or consist of a photoconductive material. In such embodiments, the inner surface 208 of the electrode activation substrate 206 can be featureless. For example, the electrode activation substrate 206 can comprise or consist of a layer of hydrogenated amorphous silicon (a-Si:H). The a-Si:H can comprise, for example, about 8% to 40% hydrogen (calculated as 100 * the number of hydrogen atoms / the total number of hydrogen and silicon atoms). The layer of a-Si:H can have a thickness of about 500 nm to about 2.0 □m. In such embodiments, the DEP electrode regions 214 can be created anywhere and in any pattern on the inner surface 208 of the electrode activation substrate 206, in accordance with the light pattern 218. The number and pattern of the DEP electrode regions 214 thus need not be fixed, but can correspond to the light pattern 218. Examples of microfluidic devices having a DEP configuration comprising a photoconductive layer such as discussed above have been described, for example, in U.S. Patent No. RE 44,711 (Wu et al.) (originally issued as U.S. Patent No. 7,612,355), the entire contents of which are incorporated herein by reference.

In other embodiments, the electrode activation substrate 206 can comprise a substrate comprising a plurality of doped layers, electrically insulating layers (or regions), and electrically conductive layers that form semiconductor integrated circuits, such as is known in semiconductor fields. For example, the electrode activation substrate 206 can comprise a plurality of phototransistors, including, for example, lateral bipolar phototransistors, each phototransistor corresponding to a DEP electrode region 214. Alternatively, the electrode activation substrate 206 can comprise electrodes (e.g., conductive metal electrodes) controlled by phototransistor switches, with each such electrode corresponding to a DEP electrode region 214. The electrode activation substrate 206 can include a pattern of such phototransistors or phototransistor-controlled electrodes. The pattern, for example, can be an array of substantially square phototransistors or phototransistor-controlled electrodes arranged in rows and columns, such as shown in Fig. 2B. Alternatively, the pattern can be an array of substantially hexagonal phototransistors or phototransistor-controlled electrodes that form a hexagonal lattice. Regardless of the pattern, electric circuit elements can form electrical connections between the DEP electrode regions 214 at the inner surface 208 of the electrode activation substrate 206 and the bottom electrode 210, and those electrical connections (i.e., phototransistors or electrodes) can be selectively activated and deactivated by the light pattern 218. When not activated, each electrical connection can have high impedance such that the relative impedance through the electrode activation substrate 206 (i.e., from the bottom electrode 204 to the inner surface 208 of the electrode activation substrate 206 which interfaces with the medium 180 in the region/chamber 202) is greater than the relative impedance through the medium 180 (i.e., from the inner surface 208 of the electrode activation substrate 206 to the top electrode 210 of the cover 110) at the corresponding DEP electrode region 214. When activated by light in the light pattern 218, however, the relative impedance through the electrode activation substrate 206 is less than the relative impedance through the medium 180 at each illuminated DEP electrode region 214, thereby activating the DEP electrode at the corresponding DEP electrode region 214 as discussed above. DEP electrodes that attract or repel micro-objects (not shown) in the medium 180 can thus be selectively activated and deactivated at many different DEP electrode regions 214 at the inner surface 208 of the electrode activation substrate 206 in the region/chamber 202 in a manner determined by the light pattern 218.

Examples of microfluidic devices having electrode activation substrates that comprise phototransistors have been described, for example, in U.S. Patent No. 7,956,339 (Ohta et al.) (see, e.g., device 300 illustrated in Figures 21 and 22, and descriptions thereof), and U.S. Patent Publication No. 2016/0184821 (Hobbs et al.) (see, e.g., devices 200, 502, 504, 600, and 700 illustrated throughout the drawings, and descriptions thereof), the entire contents of each of which are incorporated herein by reference. Examples of microfluidic devices having electrode activation substrates that comprise electrodes controlled by phototransistor switches have been described, for example, in U.S. Patent Publication No. 2014/0124370 (Short et al.) (see, e.g., devices 200, 400, 500, 600, and 900 illustrated throughout the drawings, and descriptions thereof), the entire contents of which are incorporated herein by reference.

In some embodiments of a DEP configured microfluidic device, the top electrode 210 is part of a first wall (or cover 110) of the enclosure 102, and the electrode activation substrate 206 and bottom electrode 204 are part of a second wall (or support structure 104) of the enclosure 102. The region/chamber 202 can be between the first wall and the second wall. In other embodiments, the electrode 210 is part of the second wall (or support structure 104) and one or both of the electrode activation substrate 206 and/or the electrode 210 are part of the first wall (or cover 110). Moreover, the light source 216 can alternatively be used to illuminate the enclosure 102 from below.

With the microfluidic device 200 of Figures 1B-1C having a DEP configuration, the motive module 162 can select a micro-object (not shown) in the medium 180 in the region/chamber 202 by projecting a light pattern 218 into the microfluidic device 200 to activate a first set of one or more DEP electrodes at DEP electrode regions 214a of the inner surface 208 of the electrode activation substrate 206 in a pattern (e.g., square pattern 220) that surrounds and captures the micro-object. The motive module 162 can then move the in situ-generated captured micro-object by moving the light pattern 218 relative to the microfluidic device 200 to activate a second set of one or more DEP electrodes at DEP electrode regions 214. Alternatively, the microfluidic device 200 can be moved relative to the light pattern 218.

In other embodiments, the microfluidic device 200 can have a DEP configuration that does not rely upon light activation of DEP electrodes at the inner surface 208 of the electrode activation substrate 206. For example, the electrode activation substrate 206 can comprise selectively addressable and energizable electrodes positioned opposite to a surface including at least one electrode (e.g., cover 110). Switches (e.g., transistor switches in a semiconductor substrate) may be selectively opened and closed to activate or inactivate DEP electrodes at DEP electrode regions 214, thereby creating a net DEP force on a micro-object (not shown) in region/chamber 202 in the vicinity of the activated DEP electrodes. Depending on such characteristics as the frequency of the power source 212 and the dielectric properties of the medium (not shown) and/or micro-objects in the region/chamber 202, the DEP force can attract or repel a nearby micro-object. By selectively activating and deactivating a set of DEP electrodes (e.g., at a set of DEP electrodes regions 214 that forms a square pattern 220), one or more micro-objects in region/chamber 202 can be trapped and moved within the region/chamber 202. The motive module 162 in Figure 1A can control such switches and thus activate and deactivate individual ones of the DEP electrodes to select, trap, and move particular micro-objects (not shown) around the region/chamber 202. Microfluidic devices having a DEP configuration that includes selectively addressable and energizable electrodes are known in the art and have been described, for example, in U.S. Patent Nos. 6,294,063 (Becker et al.) and 6,942,776 (Medoro), the entire contents of which are incorporated herein by reference.

As yet another example, the microfluidic device 200 can have an electrowetting (EW) configuration, which can be in place of the DEP configuration or can be located in a portion of the microfluidic device 200 that is separate from the portion which has the DEP configuration. The EW configuration can be an opto-electrowetting configuration or an electrowetting on dielectric (EWOD) configuration, both of which are known in the art. In some EW configurations, the support structure 104 has an electrode activation substrate 206 sandwiched between a dielectric layer (not shown) and the bottom electrode 204. The dielectric layer can comprise a hydrophobic material and/or can be coated with a hydrophobic material, as described below. For microfluidic devices 200 that have an EW configuration, the inner surface 208 of the support structure 104 is the inner surface of the dielectric layer or its hydrophobic coating.

The dielectric layer (not shown) can comprise one or more oxide layers, and can have a thickness of about 50 nm to about 250 nm (e.g., about 125 nm to about 175 nm). In certain embodiments, the dielectric layer may comprise a layer of oxide, such as a metal oxide (e.g., aluminum oxide or hafnium oxide). In certain embodiments, the dielectric layer can comprise a dielectric material other than a metal oxide, such as silicon oxide or a nitride. Regardless of the exact composition and thickness, the dielectric layer can have an impedance of about 10 kOhms to about 50 kOhms.

In some embodiments, the surface of the dielectric layer that faces inward toward region/chamber 202 is coated with a hydrophobic material. The hydrophobic material can comprise, for example, fluorinated carbon molecules. Examples of fluorinated carbon molecules include perfluoro-polymers such as polytetrafluoroethylene (e.g., TEFLON^{®}) or poly(2,3-difluoromethylenyl-perfluorotetrahydrofuran) (e.g., CYTOP^{™}). Molecules that make up the hydrophobic material can be covalently bonded to the surface of the dielectric layer. For example, molecules of the hydrophobic material can be covalently bound to the surface of the dielectric layer by means of a linker such as a siloxane group, a phosphonic acid group, or a thiol group. Thus, in some embodiments, the hydrophobic material can comprise alkyl-terminated siloxane, alkyl-termination phosphonic acid, or alkyl-terminated thiol. The alkyl group can be long-chain hydrocarbons (e.g., having a chain of at least 10 carbons, or at least 16, 18, 20, 22, or more carbons). Alternatively, fluorinated (or perfluorinated) carbon chains can be used in place of the alkyl groups. Thus, for example, the hydrophobic material can comprise fluoroalkyl-terminated siloxane, fluoroalkyl-terminated phosphonic acid, or fluoroalkyl-terminated thiol. In some embodiments, the hydrophobic coating has a thickness of about 10 nm to about 50 nm. In other embodiments, the hydrophobic coating has a thickness of less than 10 nm (e.g., less than 5 nm, or about 1.5 to 3.0 nm).

In some embodiments, the cover 110 of a microfluidic device 200 having an electrowetting configuration is coated with a hydrophobic material (not shown) as well. The hydrophobic material can be the same hydrophobic material used to coat the dielectric layer of the support structure 104, and the hydrophobic coating can have a thickness that is substantially the same as the thickness of the hydrophobic coating on the dielectric layer of the support structure 104. Moreover, the cover 110 can comprise an electrode activation substrate 206 sandwiched between a dielectric layer and the top electrode 210, in the manner of the support structure 104. The electrode activation substrate 206 and the dielectric layer of the cover 110 can have the same composition and/or dimensions as the electrode activation substrate 206 and the dielectric layer of the support structure 104. Thus, the microfluidic device 200 can have two electrowetting surfaces.

In some embodiments, the electrode activation substrate 206 can comprise a photoconductive material, such as described above. Accordingly, in certain embodiments, the electrode activation substrate 206 can comprise or consist of a layer of hydrogenated amorphous silicon (a-Si:H). The a-Si:H can comprise, for example, about 8% to 40% hydrogen (calculated as 100 * the number of hydrogen atoms / the total number of hydrogen and silicon atoms). The layer of a-Si:H can have a thickness of about 500 nm to about 2.0 □m. Alternatively, the electrode activation substrate 206 can comprise electrodes (e.g., conductive metal electrodes) controlled by phototransistor switches, as described above. Microfluidic devices having an opto-electrowetting configuration are known in the art and/or can be constructed with electrode activation substrates known in the art. For example, U.S. Patent No. 6,958,132 (Chiou et al.), the entire contents of which are incorporated herein by reference, discloses opto-electrowetting configurations having a photoconductive material such as a-Si:H, while U.S. Patent Publication No. 2014/0124370 (Short et al.), referenced above, discloses electrode activation substrates having electrodes controlled by phototransistor switches.

The microfluidic device 200 thus can have an opto-electrowetting configuration, and light patterns 218 can be used to activate photoconductive EW regions or photoresponsive EW electrodes in the electrode activation substrate 206. Such activated EW regions or EW electrodes of the electrode activation substrate 206 can generate an electrowetting force at the inner surface 208 of the support structure 104 (i.e., the inner surface of the overlaying dielectric layer or its hydrophobic coating). By changing the light patterns 218 (or moving microfluidic device 200 relative to the light source 216) incident on the electrode activation substrate 206, droplets (e.g., containing an aqueous medium, solution, or solvent) contacting the inner surface 208 of the support structure 104 can be moved through an immiscible fluid (e.g., an oil medium) present in the region/chamber 202.

In other embodiments, microfluidic devices 200 can have an EWOD configuration, and the electrode activation substrate 206 can comprise selectively addressable and energizable electrodes that do not rely upon light for activation. The electrode activation substrate 206 thus can include a pattern of such electrowetting (EW) electrodes. The pattern, for example, can be an array of substantially square EW electrodes arranged in rows and columns, such as shown in Fig. 2B. Alternatively, the pattern can be an array of substantially hexagonal EW electrodes that form a hexagonal lattice. Regardless of the pattern, the EW electrodes can be selectively activated (or deactivated) by electrical switches (e.g., transistor switches in a semiconductor substrate). By selectively activating and deactivating EW electrodes in the electrode activation substrate 206, droplets (not shown) contacting the inner surface 208 of the overlaying dielectric layer or its hydrophobic coating can be moved within the region/chamber 202. The motive module 162 in Figure 1A can control such switches and thus activate and deactivate individual EW electrodes to select and move particular droplets around region/chamber 202. Microfluidic devices having a EWOD configuration with selectively addressable and energizable electrodes are known in the art and have been described, for example, in U.S. Patent No. 8,685,344 (Sundarsan et al.), the entire contents of which are incorporated herein by reference.

Regardless of the configuration of the microfluidic device 200, a power source 212 can be used to provide a potential (e.g., an AC voltage potential) that powers the electrical circuits of the microfluidic device 200. The power source 212 can be the same as, or a component of, the power source 192 referenced in Fig. 1. Power source 212 can be configured to provide an AC voltage and/or current to the top electrode 210 and the bottom electrode 204. For an AC voltage, the power source 212 can provide a frequency range and an average or peak power (e.g., voltage or current) range sufficient to generate net DEP forces (or electrowetting forces) strong enough to trap and move individual micro-objects (not shown) in the region/chamber 202, as discussed above, and/or to change the wetting properties of the inner surface 208 of the support structure 104 (i.e., the dielectric layer and/or the hydrophobic coating on the dielectric layer) in the region/chamber 202, as also discussed above. Such frequency ranges and average or peak power ranges are known in the art. See, e.g., US Patent No. 6,958,132 (Chiou et al.), US Patent No. RE44,711 (Wu et al.) (originally issued as US Patent No. 7,612,355), and US Patent Application Publication Nos. US2014/0124370 (Short et al.), US2015/0306598 (Khandros et al.), and US2015/0306599 (Khandros et al.).

**Sequestration pens.** Non-limiting examples of generic sequestration pens 224, 226, and 228 are shown within the microfluidic device 230 depicted in Figures 2A-2C. Each sequestration pen 224, 226, and 228 can comprise an isolation structure 232 defining an isolation region 240 and a connection region 236 fluidically connecting the isolation region 240 to a channel 122. The connection region 236 can comprise a proximal opening 234 to the microfluidic channel 122 and a distal opening 238 to the isolation region 240. The connection region 236 can be configured so that the maximum penetration depth of a flow of a fluidic medium (not shown) flowing from the microfluidic channel 122 into the sequestration pen 224, 226, 228 does not extend into the isolation region 240. Thus, due to the connection region 236, a micro-object (not shown) or other material (not shown) disposed in an isolation region 240 of a sequestration pen 224, 226, 228 can thus be isolated from, and not substantially affected by, a flow of medium 180 in the microfluidic channel 122.

The sequestration pens 224, 226, and 228 of Figures 2A-2C each have a single opening which opens directly to the microfluidic channel 122. The opening of the sequestration pen opens laterally from the microfluidic channel 122. The electrode activation substrate 206 underlays both the microfluidic channel 122 and the sequestration pens 224, 226, and 228. The upper surface of the electrode activation substrate 206 within the enclosure of a sequestration pen, forming the floor of the sequestration pen, is disposed at the same level or substantially the same level of the upper surface the of electrode activation substrate 206 within the microfluidic channel 122 (or flow path if a channel is not present), forming the floor of the flow channel (or flow path, respectively) of the microfluidic device. The electrode activation substrate 206 may be featureless or may have an irregular or patterned surface that varies from its highest elevation to its lowest depression by less than about 3 microns, 2.5 microns, 2 microns, 1.5 microns, 1 micron, 0.9 microns, 0.5 microns, 0.4 microns, 0.2 microns, 0.1 microns or less. The variation of elevation in the upper surface of the substrate across both the microfluidic channel 122 (or flow path) and sequestration pens may be less than about 3%, 2%, 1%. 0.9%, 0.8%, 0.5%, 0.3% or 0.1% of the height of the walls of the sequestration pen or walls of the microfluidic device. While described in detail for the microfluidic device 200, this also applies to any of the microfluidic devices 100, 230, 250, 280, 290, 320, 400, 450, 500, 700 described herein.

The microfluidic channel 122 can thus be an example of a swept region, and the isolation regions 240 of the sequestration pens 224, 226, 228 can be examples of unswept regions. As noted, the microfluidic channel 122 and sequestration pens 224, 226, 228 can be configured to contain one or more fluidic media 180. In the example shown in Figures 2A-2B, the ports 222 are connected to the microfluidic channel 122 and allow a fluidic medium 180 to be introduced into or removed from the microfluidic device 230. Prior to introduction of the fluidic medium 180, the microfluidic device may be primed with a gas such as carbon dioxide gas. Once the microfluidic device 230 contains the fluidic medium 180, the flow 242 of fluidic medium 180 in the microfluidic channel 122 can be selectively generated and stopped. For example, as shown, the ports 222 can be disposed at different locations (e.g., opposite ends) of the microfluidic channel 122, and a flow 242 of medium can be created from one port 222 functioning as an inlet to another port 222 functioning as an outlet.

Figure 2C illustrates a detailed view of an example of a sequestration pen 224 according to the present disclosure. Examples of micro-objects 246 are also shown.

As is known, a flow 242 of fluidic medium 180 in a microfluidic channel 122 past a proximal opening 234 of sequestration pen 224 can cause a secondary flow 244 of the medium 180 into and/or out of the sequestration pen 224. To isolate micro-objects 246 in the isolation region 240 of a sequestration pen 224 from the secondary flow 244, the length L_{con} of the connection region 236 of the sequestration pen 224 (i.e., from the proximal opening 234 to the distal opening 238) should be greater than the penetration depth Dₚ of the secondary flow 244 into the connection region 236. The penetration depth Dₚ of the secondary flow 244 depends upon the velocity of the fluidic medium 180 flowing in the microfluidic channel 122 and various parameters relating to the configuration of the microfluidic channel 122 and the proximal opening 234 of the connection region 236 to the microfluidic channel 122. For a given microfluidic device, the configurations of the microfluidic channel 122 and the opening 234 will be fixed, whereas the rate of flow 242 of fluidic medium 180 in the microfluidic channel 122 will be variable. Accordingly, for each sequestration pen 224, a maximal velocity Vₘₐₓ for the flow 242 of fluidic medium 180 in channel 122 can be identified that ensures that the penetration depth Dₚ of the secondary flow 244 does not exceed the length L_{con} of the connection region 236. As long as the rate of the flow 242 of fluidic medium 180 in the microfluidic channel 122 does not exceed the maximum velocity Vₘₐₓ, the resulting secondary flow 244 can be limited to the microfluidic channel 122 and the connection region 236 and kept out of the isolation region 240. The flow 242 of medium 180 in the microfluidic channel 122 will thus not draw micro-objects 246 out of the isolation region 240. Rather, micro-objects 246 located in the isolation region 240 will stay in the isolation region 240 regardless of the flow 242 of fluidic medium 180 in the microfluidic channel 122.

Moreover, as long as the rate of flow 242 of medium 180 in the microfluidic channel 122 does not exceed Vₘₐₓ, the flow 242 of fluidic medium 180 in the microfluidic channel 122 will not move miscellaneous particles (e.g., microparticles and/or nanoparticles) from the microfluidic channel 122 into the isolation region 240 of a sequestration pen 224. Having the length L_{con} of the connection region 236 be greater than the maximum penetration depth Dₚ of the secondary flow 244 can thus prevent contamination of one sequestration pen 224 with miscellaneous particles from the microfluidic channel 122 or another sequestration pen (e.g., sequestration pens 226, 228 in Fig. 2D).

Because the microfluidic channel 122 and the connection regions 236 of the sequestration pens 224, 226, 228 can be affected by the flow 242 of medium 180 in the microfluidic channel 122, the microfluidic channel 122 and connection regions 236 can be deemed swept (or flow) regions of the microfluidic device 230. The isolation regions 240 of the sequestration pens 224, 226, 228, on the other hand, can be deemed unswept (or non-flow) regions. For example, components (not shown) in a first fluidic medium 180 in the microfluidic channel 122 can mix with a second fluidic medium 248 in the isolation region 240 substantially only by diffusion of components of the first medium 180 from the microfluidic channel 122 through the connection region 236 and into the second fluidic medium 248 in the isolation region 240. Similarly, components (not shown) of the second medium 248 in the isolation region 240 can mix with the first medium 180 in the microfluidic channel 122 substantially only by diffusion of components of the second medium 248 from the isolation region 240 through the connection region 236 and into the first medium 180 in the microfluidic channel 122. In some embodiments, the extent of fluidic medium exchange between the isolation region of a sequestration pen and the flow path by diffusion is greater than about 90%, 91%, 92%, 93%, 94% 95%, 96%, 97%, 98%, or greater than about 99% of fluidic exchange. The first medium 180 can be the same medium or a different medium than the second medium 248. Moreover, the first medium 180 and the second medium 248 can start out being the same, then become different (e.g., through conditioning of the second medium 248 by one or more cells in the isolation region 240, or by changing the medium 180 flowing through the microfluidic channel 122).

The maximum penetration depth Dₚ of the secondary flow 244 caused by the flow 242 of fluidic medium 180 in the microfluidic channel 122 can depend on a number of parameters, as mentioned above. Examples of such parameters include: the shape of the microfluidic channel 122 (e.g., the microfluidic channel can direct medium into the connection region 236, divert medium away from the connection region 236, or direct medium in a direction substantially perpendicular to the proximal opening 234 of the connection region 236 to the microfluidic channel 122); a width W_{ch} (or cross-sectional area) of the microfluidic channel 122 at the proximal opening 234; and a width W_{con} (or cross-sectional area) of the connection region 236 at the proximal opening 234; the velocity V of the flow 242 of fluidic medium 180 in the microfluidic channel 122; the viscosity of the first medium 180 and/or the second medium 248, or the like.

In some embodiments, the dimensions of the microfluidic channel 122 and sequestration pens 224, 226, 228 can be oriented as follows with respect to the vector of the flow 242 of fluidic medium 180 in the microfluidic channel 122: the microfluidic channel width W_{ch} (or cross-sectional area of the microfluidic channel 122) can be substantially perpendicular to the flow 242 of medium 180; the width W_{con} (or cross-sectional area) of the connection region 236 at opening 234 can be substantially parallel to the flow 242 of medium 180 in the microfluidic channel 122; and/or the length L_{con} of the connection region can be substantially perpendicular to the flow 242 of medium 180 in the microfluidic channel 122. The foregoing are examples only, and the relative position of the microfluidic channel 122 and sequestration pens 224, 226, 228 can be in other orientations with respect to each other.

As illustrated in Figure 2C, the width W_{con} of the connection region 236 can be uniform from the proximal opening 234 to the distal opening 238. The width W_{con} of the connection region 236 at the distal opening 238 can thus be in any of the ranges identified herein for the width W_{con} of the connection region 236 at the proximal opening 234. Alternatively, the width W_{con} of the connection region 236 at the distal opening 238 can be larger than the width W_{con} of the connection region 236 at the proximal opening 234.

As illustrated in Figure 2C, the width of the isolation region 240 at the distal opening 238 can be substantially the same as the width W_{con} of the connection region 236 at the proximal opening 234. The width of the isolation region 240 at the distal opening 238 can thus be in any of the ranges identified herein for the width W_{con} of the connection region 236 at the proximal opening 234. Alternatively, the width of the isolation region 240 at the distal opening 238 can be larger or smaller than the width W_{con} of the connection region 236 at the proximal opening 234. Moreover, the distal opening 238 may be smaller than the proximal opening 234 and the width W_{con} of the connection region 236 may be narrowed between the proximal opening 234 and distal opening 238. For example, the connection region 236 may be narrowed between the proximal opening and the distal opening, using a variety of different geometries (e.g. chamfering the connection region, beveling the connection region). Further, any part or subpart of the connection region 236 may be narrowed (e.g. a portion of the connection region adjacent to the proximal opening 234).

Figures 2D-2F depict another exemplary embodiment of a microfluidic device 250 containing a microfluidic circuit 262 and flow channels 264, which are variations of the respective microfluidic device 100, circuit 132 and channel 134 of Figure 1A. The microfluidic device 250 also has a plurality of sequestration pens 266 that are additional variations of the above-described sequestration pens 124, 126, 128, 130, 224, 226 or 228. In particular, it should be appreciated that the sequestration pens 266 of device 250 shown in Figures 2D-2F can replace any of the above-described sequestration pens 124, 126, 128, 130, 224, 226 or 228 in devices 100, 200, 230, 280, 290, 300. Likewise, the microfluidic device 250 is another variant of the microfluidic device 100, and may also have the same or a different DEP configuration as the above-described microfluidic device 100, 200, 230, 280, 290, 300, as well as any of the other microfluidic system components described herein.

The microfluidic device 250 of Figures 2D-2F comprises a support structure (not visible in Figures 2D-2F, but can be the same or generally similar to the support structure 104 of device 100 depicted in Figure 1A), a microfluidic circuit structure 256, and a cover (not visible in Figures 2D-2F, but can be the same or generally similar to the cover 122 of device 100 depicted in Figure 1A). The microfluidic circuit structure 256 includes a frame 252 and microfluidic circuit material 260, which can be the same as or generally similar to the frame 114 and microfluidic circuit material 116 of device 100 shown in Figure 1A. As shown in Figure 2D, the microfluidic circuit 262 defined by the microfluidic circuit material 260 can comprise multiple channels 264 (two are shown but there can be more) to which multiple sequestration pens 266 are fluidically connected.

Each sequestration pen 266 can comprise an isolation structure 272, an isolation region 270 within the isolation structure 272, and a connection region 268. From a proximal opening 274 at the microfluidic channel 264 to a distal opening 276 at the isolation structure 272, the connection region 268 fluidically connects the microfluidic channel 264 to the isolation region 270. Generally, in accordance with the above discussion of Figures 2B and 2C, a flow 278 of a first fluidic medium 254 in a channel 264 can create secondary flows 282 of the first medium 254 from the microfluidic channel 264 into and/or out of the respective connection regions 268 of the sequestration pens 266.

As illustrated in Figure 2E, the connection region 268 of each sequestration pen 266 generally includes the area extending between the proximal opening 274 to a channel 264 and the distal opening 276 to an isolation structure 272. The length L_{con} of the connection region 268 can be greater than the maximum penetration depth Dₚ of secondary flow 282, in which case the secondary flow 282 will extend into the connection region 268 without being redirected toward the isolation region 270 (as shown in Figure 2D). Alternatively, at illustrated in Figure 2F, the connection region 268 can have a length L_{con} that is less than the maximum penetration depth Dₚ, in which case the secondary flow 282 will extend through the connection region 268 and be redirected toward the isolation region 270. In this latter situation, the sum of lengths L_{c1} and L_{c2} of connection region 268 is greater than the maximum penetration depth Dₚ, so that secondary flow 282 will not extend into isolation region 270. Whether length L_{con} of connection region 268 is greater than the penetration depth Dₚ, or the sum of lengths L_{c1} and L_{c2} of connection region 268 is greater than the penetration depth Dₚ, a flow 278 of a first medium 254 in channel 264 that does not exceed a maximum velocity Vₘₐₓ will produce a secondary flow having a penetration depth Dₚ, and micro-objects (not shown but can be the same or generally similar to the micro-objects 246 shown in Figure 2C) in the isolation region 270 of a sequestration pen 266 will not be drawn out of the isolation region 270 by a flow 278 of first medium 254 in channel 264. Nor will the flow 278 in channel 264 draw miscellaneous materials (not shown) from channel 264 into the isolation region 270 of a sequestration pen 266. As such, diffusion is the only mechanism by which components in a first medium 254 in the microfluidic channel 264 can move from the microfluidic channel 264 into a second medium 258 in an isolation region 270 of a sequestration pen 266. Likewise, diffusion is the only mechanism by which components in a second medium 258 in an isolation region 270 of a sequestration pen 266 can move from the isolation region 270 to a first medium 254 in the microfluidic channel 264. The first medium 254 can be the same medium as the second medium 258, or the first medium 254 can be a different medium than the second medium 258. Alternatively, the first medium 254 and the second medium 258 can start out being the same, then become different, e.g., through conditioning of the second medium by one or more cells in the isolation region 270, or by changing the medium flowing through the microfluidic channel 264.

As illustrated in Figure 2E, the width W_{ch} of the microfluidic channels 264 (i.e., taken transverse to the direction of a fluid medium flow through the microfluidic channel indicated by arrows 278 in Figure 2D) in the microfluidic channel 264 can be substantially perpendicular to a width W_{con1} of the proximal opening 274 and thus substantially parallel to a width W_{con2} of the distal opening 276. The width W_{con1} of the proximal opening 274 and the width W_{con2} of the distal opening 276, however, need not be substantially perpendicular to each other. For example, an angle between an axis (not shown) on which the width W_{con1} of the proximal opening 274 is oriented and another axis on which the width W_{con2} of the distal opening 276 is oriented can be other than perpendicular and thus other than 90°. Examples of alternatively oriented angles include angles in any of the following ranges: from about 30° to about 90°, from about 45° to about 90°, from about 60° to about 90°, or the like.

In various embodiments of sequestration pens (e.g. 124, 126, 128, 130, 224, 226, 228, or 266), the isolation region (e.g. 240 or 270) is configured to contain a plurality of micro-objects. In other embodiments, the isolation region can be configured to contain only one, two, three, four, five, or a similar relatively small number of micro-objects. Accordingly, the volume of an isolation region can be, for example, at least 2x10⁵, 4x10⁵, 8x10⁵, 1x10⁶, 2x10⁶, 4x10⁶, 8x10⁶ cubic microns, or more.

In various embodiments of sequestration pens, the width W_{ch} of the microfluidic channel (e.g., 122) at a proximal opening (e.g. 234) can be within any of the following ranges: about 50-1000 microns, 50-500 microns, 50-400 microns, 50-300 microns, 50-250 microns, 50-200 microns, 50-150 microns, 50-100 microns, 70-500 microns, 70-400 microns, 70-300 microns, 70-250 microns, 70-200 microns, 70-150 microns, 90-400 microns, 90-300 microns, 90-250 microns, 90-200 microns, 90-150 microns, 100-300 microns, 100-250 microns, 100-200 microns, 100-150 microns, and 100-120 microns. In some other embodiments, the width W_{ch} of the microfluidic channel (e.g., 122) at a proximal opening (e.g. 234) can be in a range of about 200-800 microns, 200-700 microns, or 200-600 microns. The foregoing are examples only, and the width W_{ch} of the microfluidic channel 122 can be in other ranges (e.g., a range defined by any of the endpoints listed above). Moreover, the W_{ch} of the microfluidic channel 122 can be selected to be in any of these ranges in regions of the microfluidic channel other than at a proximal opening of a sequestration pen.

In some embodiments, a sequestration pen has a height of about 30 to about 200 microns, or about 50 to about 150 microns. In some embodiments, the sequestration pen has a cross-sectional area of about 1 x10⁴ - 3 x10⁶ square microns, 2 x10⁴ - 2 x10⁶ square microns, 4 x10⁴ - 1 x10⁶ square microns, 2 x10⁴- 5 x10⁵ square microns, 2 x10⁴- 1 x10⁵ square microns or about 2 x10⁵ - 2x10⁶ square microns.

In various embodiments of sequestration pens, the height H_{ch} of the microfluidic channel (e.g.,122) at a proximal opening (e.g., 234) can be within any of the following ranges: 20-100 microns, 20-90 microns, 20-80 microns, 20-70 microns, 20-60 microns, 20-50 microns, 30-100 microns, 30-90 microns, 30-80 microns, 30-70 microns, 30-60 microns, 30-50 microns, 40-100 microns, 40-90 microns, 40-80 microns, 40-70 microns, 40-60 microns, or 40-50 microns. The foregoing are examples only, and the height H_{ch} of the microfluidic channel (e.g.,122) can be in other ranges (e.g., a range defined by any of the endpoints listed above). The height H_{ch} of the microfluidic channel 122 can be selected to be in any of these ranges in regions of the microfluidic channel other than at a proximal opening of a sequestration pen.

In various embodiments of sequestration pens, a cross-sectional area of the microfluidic channel (e.g., 122) at a proximal opening (e.g., 234) can be within any of the following ranges: 500-50,000 square microns, 500-40,000 square microns, 500-30,000 square microns, 500-25,000 square microns, 500-20,000 square microns, 500-15,000 square microns, 500-10,000 square microns, 500-7,500 square microns, 500-5,000 square microns, 1,000-25,000 square microns, 1,000-20,000 square microns, 1,000-15,000 square microns, 1,000-10,000 square microns, 1,000-7,500 square microns, 1,000-5,000 square microns, 2,000-20,000 square microns, 2,000-15,000 square microns, 2,000-10,000 square microns, 2,000-7,500 square microns, 2,000-6,000 square microns, 3,000-20,000 square microns, 3,000-15,000 square microns, 3,000-10,000 square microns, 3,000-7,500 square microns, or 3,000 to 6,000 square microns. The foregoing are examples only, and the cross-sectional area of the microfluidic channel (e.g., 122) at a proximal opening (e.g., 234) can be in other ranges (e.g., a range defined by any of the endpoints listed above).

In various embodiments of sequestration pens, the length L_{con} of the connection region (e.g., 236) can be in any of the following ranges: about 1-600 microns, 5-550 microns, 10-500 microns, 15-400 microns, 20-300 microns, 20-500 microns, 40-400 microns, 60-300 microns, 80-200 microns, or about 100-150 microns. The foregoing are examples only, and length L_{con} of a connection region (e.g., 236) can be in a different range than the foregoing examples (e.g., a range defined by any of the endpoints listed above).

In various embodiments of sequestration pens, the width W_{con} of a connection region (e.g., 236) at a proximal opening (e.g., 234) can be in any of the following ranges: 20-500 microns, 20-400 microns, 20-300 microns, 20-200 microns, 20-150 microns, 20-100 microns, 20-80 microns, 20-60 microns, 30-400 microns, 30-300 microns, 30-200 microns, 30-150 microns, 30-100 microns, 30-80 microns, 30-60 microns, 40-300 microns, 40-200 microns, 40-150 microns, 40-100 microns, 40-80 microns, 40-60 microns, 50-250 microns, 50-200 microns, 50-150 microns, 50-100 microns, 50-80 microns, 60-200 microns, 60-150 microns, 60-100 microns, 60-80 microns, 70-150 microns, 70-100 microns, and 80-100 microns. The foregoing are examples only, and the width W_{con} of a connection region (e.g., 236) at a proximal opening (e.g., 234) can be different than the foregoing examples (e.g., a range defined by any of the endpoints listed above).

In various embodiments of sequestration pens, the width W_{con} of a connection region (e.g., 236) at a proximal opening (e.g., 234) can be at least as large as the largest dimension of a micro-object (e.g., a biological cell, such as an activated T cell) that the sequestration pen is intended for. The foregoing are examples only, and the width W_{con} of a connection region (e.g., 236) at a proximal opening (e.g., 234) can be different than the foregoing examples (e.g., a range defined by any of the endpoints listed above).

In various embodiments of sequestration pens, a ratio of the length L_{con} of a connection region (e.g., 236) to a width W_{con} of the connection region (e.g., 236) at the proximal opening 234 can be greater than or equal to any of the following ratios: 0.5, 1.0, 1.5, 2.0, 2.5, 3.0, 3.5, 4.0, 4.5, 5.0, 6.0, 7.0, 8.0, 9.0, 10.0, or more. The foregoing are examples only, and the ratio of the length L_{con} of a connection region 236 to a width W_{con} of the connection region 236 at the proximal opening 234 can be different than the foregoing examples.

In various embodiments of microfluidic devices 100, 200, 23, 250, 280, 290, 300, Vₘₐₓ can be set around 0.1, 0.2, 0.3, 0.4, 0.5, 0.6, 0.7, 0.8, 0.9, 1.0, 1.1, 1.2, 1.3, 1.5, 2.0, 2.5, 3.0, 3.5, 4.0, 4.5, 5.0, 5.5, 6.0, 6.7, 7.0, 7.5, 8.0, 8.5, 9.0, 10, 11, 12, 13, 14, 15 microliters/sec, or more.

In various embodiments of microfluidic devices having sequestration pens, the volume of an isolation region (e.g., 240) of a sequestration pen can be, for example, at least 1X10⁵, 2x10⁵, 4x10⁵, 8x10⁵, 1x10⁶, 2x10⁶, 4x10⁶, 6x10⁶, 8x10⁶, 1x10⁷, 5x10⁷, 1x10⁸, 5x10⁸, or 8x10⁸ cubic microns, or more. In various embodiments of microfluidic devices having sequestration pens, the volume of a sequestration pen may be about 2x10⁵, 5x10⁵, 8x10⁵, 1x10⁶, 2x10⁶, 4x10⁶, 8x10⁶, 1x10⁷, 3x10⁷, 5x10⁷, or about 8x10⁷ cubic microns, or more. In some other embodiments, the volume of a sequestration pen may be about 0.1 nanoliter to about 50 nanoliters, 0.2 nanoliters to about 25 nanoliters, 0.5 nanoliters to about 20 nanoliters, about 0.8 nanoliters to about 15 nanoliters, or about 1 nanoliters to about 10 nanoliters.

In various embodiment, the microfluidic device has sequestration pens configured as in any of the embodiments discussed herein where the microfluidic device has about 5 to about 10 sequestration pens, about 10 to about 50 sequestration pens, about 100 to about 500 sequestration pens; about 200 to about 1000 sequestration pens, about 500 to about 1500 sequestration pens, about 1000 to about 2000 sequestration pens, about 1500 to about 3000, about 2000 to about 4000, about 2500 to about 5000, about 3000 to about 6000, about 3500 to about 7000, about 4000 to about 8000, about 4500 to about 9000, or about 5000 to about 10,000 sequestration pens. The sequestration pens need not all be the same size and may include a variety of configurations (e.g., different widths, different features within the sequestration pen).

Figure 2G illustrates a microfluidic device 280 according to one embodiment. The microfluidic device 280 illustrated in Figure 2G is a stylized diagram of a microfluidic device 100. In practice the microfluidic device 280 and its constituent circuit elements (e.g. channels 122 and sequestration pens 128) would have the dimensions discussed herein. The microfluidic circuit 120 illustrated in Figure 2G has two ports 107, four distinct channels 122 and four distinct flow paths 106. The microfluidic device 280 further comprises a plurality of sequestration pens opening off of each channel 122. In the microfluidic device illustrated in Figure 2G, the sequestration pens have a geometry similar to the pens illustrated in Figure 2C and thus, have both connection regions and isolation regions. Accordingly, the microfluidic circuit 120 includes both swept regions (e.g. channels 122 and portions of the connection regions 236 within the maximum penetration depth Dₚ of the secondary flow 244) and non-swept regions (e.g. isolation regions 240 and portions of the connection regions 236 not within the maximum penetration depth Dₚ of the secondary flow 244).

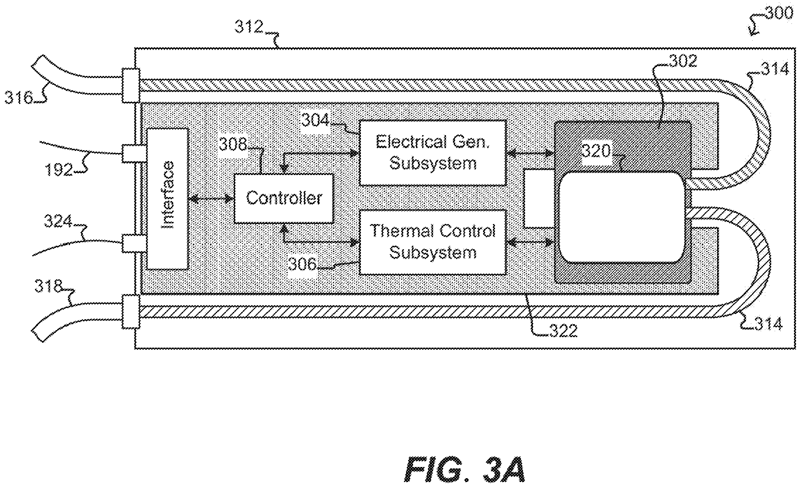

Figures 3A through 3B shows various embodiments of system 150 which can be used to operate and observe microfluidic devices (e.g. 100, 200, 230, 250, 280, 290, 300) according to the present disclosure. As illustrated in Figure 3A, the system 150 can include a structure ("nest") 300 configured to hold a microfluidic device 100 (not shown), or any other microfluidic device described herein. The nest 300 can include a socket 302 capable of interfacing with the microfluidic device 320 (e.g., an optically-actuated electrokinetic device 100) and providing electrical connections from power source 192 to microfluidic device 320. The nest 300 can further include an integrated electrical signal generation subsystem 304. The electrical signal generation subsystem 304 can be configured to supply a biasing voltage to socket 302 such that the biasing voltage is applied across a pair of electrodes in the microfluidic device 320 when it is being held by socket 302. Thus, the electrical signal generation subsystem 304 can be part of power source 192. The ability to apply a biasing voltage to microfluidic device 320 does not mean that a biasing voltage will be applied at all times when the microfluidic device 320 is held by the socket 302. Rather, in most cases, the biasing voltage will be applied intermittently, e.g., only as needed to facilitate the generation of electrokinetic forces, such as dielectrophoresis or electro-wetting, in the microfluidic device 320.

As illustrated in Figure 3A, the nest 300 can include a printed circuit board assembly (PCBA) 322. The electrical signal generation subsystem 304 can be mounted on and electrically integrated into the PCBA 322. The exemplary support includes socket 302 mounted on PCBA 322, as well.

Typically, the electrical signal generation subsystem 304 will include a waveform generator (not shown). The electrical signal generation subsystem 304 can further include an oscilloscope (not shown) and/or a waveform amplification circuit (not shown) configured to amplify a waveform received from the waveform generator. The oscilloscope, if present, can be configured to measure the waveform supplied to the microfluidic device 320 held by the socket 302. In certain embodiments, the oscilloscope measures the waveform at a location proximal to the microfluidic device 320 (and distal to the waveform generator), thus ensuring greater accuracy in measuring the waveform actually applied to the device. Data obtained from the oscilloscope measurement can be, for example, provided as feedback to the waveform generator, and the waveform generator can be configured to adjust its output based on such feedback. An example of a suitable combined waveform generator and oscilloscope is the Red Pitaya^{™}.

In certain embodiments, the nest 300 further comprises a controller 308, such as a microprocessor used to sense and/or control the electrical signal generation subsystem 304. Examples of suitable microprocessors include the Arduino^{™} microprocessors, such as the Arduino Nano^{™}. The controller 308 may be used to perform functions and analysis or may communicate with an external master controller 154 (shown in Figure 1A) to perform functions and analysis. In the embodiment illustrated in Figure 3A the controller 308 communicates with a master controller 154 through an interface 310 (e.g., a plug or connector).

In some embodiments, the nest 300 can comprise an electrical signal generation subsystem 304 comprising a Red Pitaya^{™} waveform generator/oscilloscope unit ("Red Pitaya unit") and a waveform amplification circuit that amplifies the waveform generated by the Red Pitaya unit and passes the amplified voltage to the microfluidic device 100. In some embodiments, the Red Pitaya unit is configured to measure the amplified voltage at the microfluidic device 320 and then adjust its own output voltage as needed such that the measured voltage at the microfluidic device 320 is the desired value. In some embodiments, the waveform amplification circuit can have a +6.5V to -6.5V power supply generated by a pair of DC-DC converters mounted on the PCBA 322, resulting in a signal of up to 13 Vpp at the microfluidic device 100.

As illustrated in Figure 3A, the support structure 300 (e.g., nest) can further include a thermal control subsystem 306. The thermal control subsystem 306 can be configured to regulate the temperature of microfluidic device 320 held by the support structure 300. For example, the thermal control subsystem 306 can include a Peltier thermoelectric device (not shown) and a cooling unit (not shown). The Peltier thermoelectric device can have a first surface configured to interface with at least one surface of the microfluidic device 320. The cooling unit can be, for example, a cooling block (not shown), such as a liquid-cooled aluminum block. A second surface of the Peltier thermoelectric device (e.g., a surface opposite the first surface) can be configured to interface with a surface of such a cooling block. The cooling block can be connected to a fluidic path 314 configured to circulate cooled fluid through the cooling block. In the embodiment illustrated in Figure 3A, the support structure 300 comprises an inlet 316 and an outlet 318 to receive cooled fluid from an external reservoir (not shown), introduce the cooled fluid into the fluidic path 314 and through the cooling block, and then return the cooled fluid to the external reservoir. In some embodiments, the Peltier thermoelectric device, the cooling unit, and/or the fluidic path 314 can be mounted on a casing 312of the support structure 300. In some embodiments, the thermal control subsystem 306 is configured to regulate the temperature of the Peltier thermoelectric device so as to achieve a target temperature for the microfluidic device 320. Temperature regulation of the Peltier thermoelectric device can be achieved, for example, by a thermoelectric power supply, such as a Pololu^{™} thermoelectric power supply (Pololu Robotics and Electronics Corp.). The thermal control subsystem 306 can include a feedback circuit, such as a temperature value provided by an analog circuit. Alternatively, the feedback circuit can be provided by a digital circuit.

In some embodiments, the nest 300 can include a thermal control subsystem 306 with a feedback circuit that is an analog voltage divider circuit (not shown) which includes a resistor (e.g., with resistance 1 kOhm+/-0.1 %, temperature coefficient +/-0.02 ppm/C0) and a NTC thermistor (e.g., with nominal resistance 1 kOhm+/-0.01 %). In some instances, the thermal control subsystem 306 measures the voltage from the feedback circuit and then uses the calculated temperature value as input to an on-board PID control loop algorithm. Output from the PID control loop algorithm can drive, for example, both a directional and a pulse-width-modulated signal pin on a Pololu^{™} motor drive (not shown) to actuate the thermoelectric power supply, thereby controlling the Peltier thermoelectric device.

The nest 300 can include a serial port 324 which allows the microprocessor of the controller 308 to communicate with an external master controller 154 via the interface 310 (not shown). In addition, the microprocessor of the controller 308 can communicate (e.g., via a Plink tool (not shown)) with the electrical signal generation subsystem 304 and thermal control subsystem 306. Thus, via the combination of the controller 308, the interface 310, and the serial port 324, the electrical signal generation subsystem 304 and the thermal control subsystem 306 can communicate with the external master controller 154. In this manner, the master controller 154 can, among other things, assist the electrical signal generation subsystem 304 by performing scaling calculations for output voltage adjustments. A Graphical User Interface (GUI) (not shown) provided via a display device 170 coupled to the external master controller 154, can be configured to plot temperature and waveform data obtained from the thermal control subsystem 306 and the electrical signal generation subsystem 304, respectively. Alternatively, or in addition, the GUI can allow for updates to the controller 308, the thermal control subsystem 306, and the electrical signal generation subsystem 304.

As discussed above, system 150 can include an imaging device 194. In some embodiments, the imaging device 194 comprises a light modulating subsystem 330 (See Figure 3B). The light modulating subsystem 330 can include a digital mirror device (DMD) or a microshutter array system (MSA), either of which can be configured to receive light from a light source 332 and transmits a subset of the received light into an optical train of microscope 350. Alternatively, the light modulating subsystem 330 can include a device that produces its own light (and thus dispenses with the need for a light source 332), such as an organic light emitting diode display (OLED), a liquid crystal on silicon (LCOS) device, a ferroelectric liquid crystal on silicon device (FLCOS), or a transmissive liquid crystal display (LCD). The light modulating subsystem 330 can be, for example, a projector. Thus, the light modulating subsystem 330 can be capable of emitting both structured and unstructured light. In certain embodiments, imaging module 164 and/or motive module 162 of system 150 can control the light modulating subsystem 330.

In certain embodiments, the imaging device 194 further comprises a microscope 350. In such embodiments, the nest 300 and light modulating subsystem 330 can be individually configured to be mounted on the microscope 350. The microscope 350 can be, for example, a standard research-grade light microscope or fluorescence microscope. Thus, the nest 300 can be configured to be mounted on the stage 344 of the microscope 350 and/or the light modulating subsystem 330 can be configured to mount on a port of microscope 350. In other embodiments, the nest 300 and the light modulating subsystem 330 described herein can be integral components of microscope 350.

In certain embodiments, the microscope 350 can further include one or more detectors 348. In some embodiments, the detector 348 is controlled by the imaging module 164. The detector 348 can include an eye piece, a charge-coupled device (CCD), a camera (e.g., a digital camera), or any combination thereof. If at least two detectors 348 are present, one detector can be, for example, a fast-frame-rate camera while the other detector can be a high sensitivity camera. Furthermore, the microscope 350 can include an optical train configured to receive reflected and/or emitted light from the microfluidic device 320 and focus at least a portion of the reflected and/or emitted light on the one or more detectors 348. The optical train of the microscope can also include different tube lenses (not shown) for the different detectors, such that the final magnification on each detector can be different.

In certain embodiments, imaging device 194 is configured to use at least two light sources. For example, a first light source 332 can be used to produce structured light (e.g., via the light modulating subsystem 330) and a second light source 334 can be used to provide unstructured light. The first light source 332 can produce structured light for optically-actuated electrokinesis and/or fluorescent excitation, and the second light source 334 can be used to provide bright field illumination. In these embodiments, the motive module 164 can be used to control the first light source 332 and the imaging module 164 can be used to control the second light source 334. The optical train of the microscope 350 can be configured to (1) receive structured light from the light modulating subsystem 330 and focus the structured light on at least a first region in a microfluidic device, such as an optically-actuated electrokinetic device, when the device is being held by the nest 300, and (2) receive reflected and/or emitted light from the microfluidic device and focus at least a portion of such reflected and/or emitted light onto detector 348. The optical train can be further configured to receive unstructured light from a second light source and focus the unstructured light on at least a second region of the microfluidic device, when the device is held by the nest 300. In certain embodiments, the first and second regions of the microfluidic device can be overlapping regions. For example, the first region can be a subset of the second region. In other embodiments, the second light source 334 may additionally or alternatively include a laser, which may have any suitable wavelength of light. The representation of the optical system shown in Figure 3B is a schematic representation only, and the optical system may include additional filters, notch filters, lenses and the like. When the second light source 334 includes one or more light source(s) for brightfield and/or fluorescent excitation, as well as laser illumination the physical arrangement of the light source(s) may vary from that shown in Figure 3B, and the laser illumination may be introduced at any suitable physical location within the optical system. The schematic locations of light source 432 and light source 402/light modulating subsystem 404 may be interchanged as well.

In Figure 3B, the first light source 332 is shown supplying light to a light modulating subsystem 330, which provides structured light to the optical train of the microscope 350 of system 355 (not shown). The second light source 334 is shown providing unstructured light to the optical train via a beam splitter 336. Structured light from the light modulating subsystem 330 and unstructured light from the second light source 334 travel from the beam splitter 336 through the optical train together to reach a second beam splitter (or dichroic filter 338, depending on the light provided by the light modulating subsystem 330), where the light gets reflected down through the objective 336 to the sample plane 342. Reflected and/or emitted light from the sample plane 342 then travels back up through the objective 340, through the beam splitter and/or dichroic filter 338, and to a dichroic filter 346. Only a fraction of the light reaching dichroic filter 346 passes through and reaches the detector 348.

In some embodiments, the second light source 334 emits blue light. With an appropriate dichroic filter 346, blue light reflected from the sample plane 342 is able to pass through dichroic filter 346 and reach the detector 348. In contrast, structured light coming from the light modulating subsystem 330 gets reflected from the sample plane 342, but does not pass through the dichroic filter 346. In this example, the dichroic filter 346 is filtering out visible light having a wavelength longer than 495 nm. Such filtering out of the light from the light modulating subsystem 330 would only be complete (as shown) if the light emitted from the light modulating subsystem did not include any wavelengths shorter than 495 nm. In practice, if the light coming from the light modulating subsystem 330 includes wavelengths shorter than 495 nm (e.g., blue wavelengths), then some of the light from the light modulating subsystem would pass through filter 346 to reach the detector 348. In such an embodiment, the filter 346 acts to change the balance between the amount of light that reaches the detector 348 from the first light source 332 and the second light source 334. This can be beneficial if the first light source 332 is significantly stronger than the second light source 334. In other embodiments, the second light source 334 can emit red light, and the dichroic filter 346 can filter out visible light other than red light (e.g., visible light having a wavelength shorter than 650 nm).

**Coating solutions and coating agents.** Without intending to be limited by theory, maintenance of a biological micro-object (e.g., a biological cell) within a microfluidic device (e.g., a DEP-configured and/or EW-configured microfluidic device) may be facilitated (i.e., the biological micro-object exhibits increased viability, greater expansion and/or greater portability within the microfluidic device) when at least one or more inner surfaces of the microfluidic device have been conditioned or coated so as to present a layer of organic and/or hydrophilic molecules that provides the primary interface between the microfluidic device and biological micro-object(s) maintained therein. In some embodiments, one or more of the inner surfaces of the microfluidic device (e.g. the inner surface of the electrode activation substrate of a DEP-configured microfluidic device, the cover of the microfluidic device, and/or the surfaces of the circuit material) may be treated with or modified by a coating solution and/or coating agent to generate the desired layer of organic and/or hydrophilic molecules.

The coating may be applied before or after introduction of biological micro-object(s), or may be introduced concurrently with the biological micro-object(s). In some embodiments, the biological micro-object(s) may be imported into the microfluidic device in a fluidic medium that includes one or more coating agents. In other embodiments, the inner surface(s) of the microfluidic device (e.g., a DEP-configured microfluidic device) are treated or "primed" with a coating solution comprising a coating agent prior to introduction of the biological micro-object(s) into the microfluidic device.

In some embodiments, at least one surface of the microfluidic device includes a coating material that provides a layer of organic and/or hydrophilic molecules suitable for maintenance and/or expansion of biological micro-object(s) (e.g. provides a conditioned surface as described below). In some embodiments, substantially all the inner surfaces of the microfluidic device include the coating material. The coated inner surface(s) may include the surface of a flow path (e.g., channel), chamber, or sequestration pen, or a combination thereof. In some embodiments, each of a plurality of sequestration pens has at least one inner surface coated with coating materials. In other embodiments, each of a plurality of flow paths or channels has at least one inner surface coated with coating materials. In some embodiments, at least one inner surface of each of a plurality of sequestration pens and each of a plurality of channels is coated with coating materials.

**Coating agent/Solution.** Any convenient coating agent/coating solution can be used, including but not limited to: serum or serum factors, bovine serum albumin (BSA), polymers, detergents, enzymes, and any combination thereof.

**Polymer-based coating materials.** The at least one inner surface may include a coating material that comprises a polymer. The polymer may be covalently or non-covalently bound (or may be non-specifically adhered) to the at least one surface. The polymer may have a variety of structural motifs, such as found in block polymers (and copolymers), star polymers (star copolymers), and graft or comb polymers (graft copolymers), all of which may be suitable for the methods disclosed herein.

The polymer may include a polymer including alkylene ether moieties. A wide variety of alkylene ether containing polymers may be suitable for use in the microfluidic devices described herein. One non-limiting exemplary class of alkylene ether containing polymers are amphiphilic nonionic block copolymers which include blocks of polyethylene oxide (PEO) and polypropylene oxide (PPO) subunits in differing ratios and locations within the polymer chain. Pluronic^{®} polymers (BASF) are block copolymers of this type and are known in the art to be suitable for use when in contact with living cells. The polymers may range in average molecular mass M_{w} from about 2000Da to about 20KDa. In some embodiments, the PEO-PPO block copolymer can have a hydrophilic-lipophilic balance (HLB) greater than about 10 (e.g. 12-18). Specific Pluronic^{®} polymers useful for yielding a coated surface include Pluronic^{®} L44, L64, P85, and F127 (including F127NF). Another class of alkylene ether containing polymers is polyethylene glycol (PEG M_{w} <100,000Da) or alternatively polyethylene oxide (PEO, M_{w}>100,000). In some embodiments, a PEG may have an M_{w} of about 1000Da, 5000Da, 10,000Da or 20,000Da.

In other embodiments, the coating material may include a polymer containing carboxylic acid moieties. The carboxylic acid subunit may be an alkyl, alkenyl or aromatic moiety containing subunit. One non-limiting example is polylactic acid (PLA). In other embodiments, the coating material may include a polymer containing phosphate moieties, either at a terminus of the polymer backbone or pendant from the backbone of the polymer. In yet other embodiments, the coating material may include a polymer containing sulfonic acid moieties. The sulfonic acid subunit may be an alkyl, alkenyl or aromatic moiety containing subunit. One non-limiting example is polystyrene sulfonic acid (PSSA) or polyanethole sulfonic acid. In further embodiments, the coating material may include a polymer including amine moieties. The polyamino polymer may include a natural polyamine polymer or a synthetic polyamine polymer. Examples of natural polyamines include spermine, spermidine, and putrescine.

In other embodiments, the coating material may include a polymer containing saccharide moieties. In a non-limiting example, polysaccharides such as xanthan gum or dextran may be suitable to form a material which may reduce or prevent cell sticking in the microfluidic device. For example, a dextran polymer having a size about 3kDa may be used to provide a coating material for a surface within a microfluidic device.

In other embodiments, the coating material may include a polymer containing nucleotide moieties, i.e. a nucleic acid, which may have ribonucleotide moieties or deoxyribonucleotide moieties, providing a polyelectrolyte surface. The nucleic acid may contain only natural nucleotide moieties or may contain unnatural nucleotide moieties which comprise nucleobase, ribose or phosphate moiety analogs such as 7-deazaadenine, pentose, methyl phosphonate or phosphorothioate moieties without limitation.

In yet other embodiments, the coating material may include a polymer containing amino acid moieties. The polymer containing amino acid moieties may include a natural amino acid containing polymer or an unnatural amino acid containing polymer, either of which may include a peptide, a polypeptide or a protein. In one non-limiting example, the protein may be bovine serum albumin (BSA) and/or serum (or a combination of multiple different sera) comprising albumin and/or one or more other similar proteins as coating agents. The serum can be from any convenient source, including but not limited to fetal calf serum, sheep serum, goat serum, horse serum, and the like. In certain embodiments, BSA in a coating solution is present in a range of form about 1 mg/mL to about 100 mg/mL, including 5 mg/mL, 10 mg/mL, 20 mg/mL, 30 mg/mL, 40 mg/mL, 50 mg/mL, 60 mg/mL, 70 mg/mL, 80 mg/mL, 90 mg/mL, or more or anywhere in between. In certain embodiments, serum in a coating solution may be present in a range of from about 20% (v/v) to about 50% v/v, including 25%, 30%, 35%, 40%, 45%, or more or anywhere in between. In some embodiments, BSA may be present as a coating agent in a coating solution at 5 mg/mL, whereas in other embodiments, BSA may be present as a coating agent in a coating solution at 70 mg/mL. In certain embodiments, serum is present as a coating agent in a coating solution at 30%. In some embodiments, an extracellular matrix (ECM) protein may be provided within the coating material for optimized cell adhesion to foster cell growth. A cell matrix protein, which may be included in a coating material, can include, but is not limited to, a collagen, an elastin, an RGD-containing peptide (e.g. a fibronectin), or a laminin. In yet other embodiments, growth factors, cytokines, hormones or other cell signaling species may be provided within the coating material of the microfluidic device.

In some embodiments, the coating material may include a polymer containing more than one of alkylene oxide moieties, carboxylic acid moieties, sulfonic acid moieties, phosphate moieties, saccharide moieties, nucleotide moieties, or amino acid moieties. In other embodiments, the polymer conditioned surface may include a mixture of more than one polymer each having alkylene oxide moieties, carboxylic acid moieties, sulfonic acid moieties, phosphate moieties, saccharide moieties, nucleotide moieties, and/or amino acid moieties, which may be independently or simultaneously incorporated into the coating material.

**Covalently linked coating materials.** In some embodiments, the at least one inner surface includes covalently linked molecules that provide a layer of organic and/or hydrophilic molecules suitable for maintenance/expansion of biological micro-object(s) within the microfluidic device, providing a conditioned surface for such cells.

The covalently linked molecules include a linking group, wherein the linking group is covalently linked to one or more surfaces of the microfluidic device, as described below. The linking group is also covalently linked to a moiety configured to provide a layer of organic and/or hydrophilic molecules suitable for maintenance/expansion of biological micro-object(s).

In some embodiments, the covalently linked moiety configured to provide a layer of organic and/or hydrophilic molecules suitable for maintenance/expansion of biological micro-object(s) may include alkyl or fluoroalkyl (which includes perfluoroalkyl) moieties; mono- or polysaccharides (which may include but is not limited to dextran); alcohols (including but not limited to propargyl alcohol); polyalcohols, including but not limited to polyvinyl alcohol; alkylene ethers, including but not limited to polyethylene glycol; polyelectrolytes (including but not limited to polyacrylic acid or polyvinyl phosphonic acid); amino groups (including derivatives thereof, such as, but not limited to alkylated amines, hydroxyalkylated amino group, guanidinium, and heterocylic groups containing an unaromatized nitrogen ring atom, such as, but not limited to morpholinyl or piperazinyl); carboxylic acids including but not limited to propiolic acid (which may provide a carboxylate anionic surface); phosphonic acids, including but not limited to ethynyl phosphonic acid (which may provide a phosphonate anionic surface); sulfonate anions; carboxybetaines; sulfobetaines; sulfamic acids; or amino acids.

In various embodiments, the covalently linked moiety configured to provide a layer of organic and/or hydrophilic molecules suitable for maintenance/expansion of biological micro-object(s) in the microfluidic device may include non-polymeric moieties such as an alkyl moiety, a substituted alkyl moiety, such as a fluoroalkyl moiety (including but not limited to a perfluoroalkyl moiety), amino acid moiety, alcohol moiety, amino moiety, carboxylic acid moiety, phosphonic acid moiety, sulfonic acid moiety, sulfamic acid moiety, or saccharide moiety. Alternatively, the covalently linked moiety may include polymeric moieties, which may be any of the moieties described above.

In some embodiments, the covalently linked alkyl moiety may comprises carbon atoms forming a linear chain (e.g., a linear chain of at least 10 carbons, or at least 14, 16, 18, 20, 22, or more carbons) and may be an unbranched alkyl moiety. In some embodiments, the alkyl group may include a substituted alkyl group (e.g., some of the carbons in the alkyl group can be fluorinated or perfluorinated). In some embodiments, the alkyl group may include a first segment, which may include a perfluoroalkyl group, joined to a second segment, which may include a non-substituted alkyl group, where the first and second segments may be joined directly or indirectly (e.g., by means of an ether linkage). The first segment of the alkyl group may be located distal to the linking group, and the second segment of the alkyl group may be located proximal to the linking group.

In other embodiments, the covalently linked moiety may include at least one amino acid, which may include more than one type of amino acid. Thus, the covalently linked moiety may include a peptide or a protein. In some embodiments, the covalently linked moiety may include an amino acid which may provide a zwitterionic surface to support cell growth, viability, portability, or any combination thereof.

In other embodiments, the covalently linked moiety may include at least one alkylene oxide moiety, and may include any alkylene oxide polymer as described above. One useful class of alkylene ether containing polymers is polyethylene glycol (PEG M_{w} <100,000Da) or alternatively polyethylene oxide (PEO, M_{w}>100,000). In some embodiments, a PEG may have an M_{w} of about 1000Da, 5000Da, 10,000Da or 20,000Da.

The covalently linked moiety may include one or more saccharides. The covalently linked saccharides may be mono-, di-, or polysaccharides. The covalently linked saccharides may be modified to introduce a reactive pairing moiety which permits coupling or elaboration for attachment to the surface. Exemplary reactive pairing moieties may include aldehyde, alkyne or halo moieties. A polysaccharide may be modified in a random fashion, wherein each of the saccharide monomers may be modified or only a portion of the saccharide monomers within the polysaccharide are modified to provide a reactive pairing moiety that may be coupled directly or indirectly to a surface. One exemplar may include a dextran polysaccharide, which may be coupled indirectly to a surface via an unbranched linker.

The covalently linked moiety may include one or more amino groups. The amino group may be a substituted amine moiety, guanidine moiety, nitrogen-containing heterocyclic moiety or heteroaryl moiety. The amino containing moieties may have structures permitting pH modification of the environment within the microfluidic device, and optionally, within the sequestration pens and/or flow paths (e.g., channels).

The coating material providing a conditioned surface may comprise only one kind of covalently linked moiety or may include more than one different kind of covalently linked moiety. For example, the fluoroalkyl conditioned surfaces (including perfluoroalkyl) may have a plurality of covalently linked moieties which are all the same, e.g., having the same linking group and covalent attachment to the surface, the same overall length, and the same number of fluoromethylene units comprising the fluoroalkyl moiety. Alternatively, the coating material may have more than one kind of covalently linked moiety attached to the surface. For example, the coating material may include molecules having covalently linked alkyl or fluoroalkyl moieties having a specified number of methylene or fluoromethylene units and may further include a further set of molecules having charged moieties covalently attached to an alkyl or fluoroalkyl chain having a greater number of methylene or fluoromethylene units, which may provide capacity to present bulkier moieties at the coated surface. In this instance, the first set of molecules having different, less sterically demanding termini and fewer backbone atoms can help to functionalize the entire substrate surface and thereby prevent undesired adhesion or contact with the silicon/silicon oxide, hafnium oxide or alumina making up the substrate itself. In another example, the covalently linked moieties may provide a zwitterionic surface presenting alternating charges in a random fashion on the surface.

**Conditioned surface properties.** Aside from the composition of the conditioned surface, other factors such as physical thickness of the hydrophobic material can impact DEP force. Various factors can alter the physical thickness of the conditioned surface, such as the manner in which the conditioned surface is formed on the substrate (e.g. vapor deposition, liquid phase deposition, spin coating, flooding, and electrostatic coating). In some embodiments, the conditioned surface has a thickness in the range of about 1nm to about 10nm; about 1 nm to about 7 nm; about 1nm to about 5nm; or any individual value therebetween. In other embodiments, the conditioned surface formed by the covalently linked moieties may have a thickness of about 10 nm to about 50 nm. In various embodiments, the conditioned surface prepared as described herein has a thickness of less than 10nm. In some embodiments, the covalently linked moieties of the conditioned surface may form a monolayer when covalently linked to the surface of the microfluidic device (e.g., a DEP configured substrate surface) and may have a thickness of less than 10 nm (e.g., less than 5 nm, or about 1.5 to 3.0 nm). These values are in contrast to that of a surface prepared by spin coating, for example, which may typically have a thickness in the range of about 30nm. In some embodiments, the conditioned surface does not require a perfectly formed monolayer to be suitably functional for operation within a DEP-configured microfluidic device.

In various embodiments, the coating material providing a conditioned surface of the microfluidic device may provide desirable electrical properties. Without intending to be limited by theory, one factor that impacts robustness of a surface coated with a particular coating material is intrinsic charge trapping. Different coating materials may trap electrons, which can lead to breakdown of the coating material. Defects in the coating material may increase charge trapping and lead to further breakdown of the coating material. Similarly, different coating materials have different dielectric strengths (i.e. the minimum applied electric field that results in dielectric breakdown), which may impact charge trapping. In certain embodiments, the coating material can have an overall structure (e.g., a densely-packed monolayer structure) that reduces or limits that amount of charge trapping.

In addition to its electrical properties, the conditioned surface may also have properties that are beneficial in use with biological molecules. For example, a conditioned surface that contains fluorinated (or perfluorinated) carbon chains may provide a benefit relative to alkyl-terminated chains in reducing the amount of surface fouling. Surface fouling, as used herein, refers to the amount of indiscriminate material deposition on the surface of the microfluidic device, which may include permanent or semi-permanent deposition of biomaterials such as protein and its degradation products, nucleic acids and respective degradation products and the like.

**Unitary or Multi-part conditioned surface.** The covalently linked coating material may be formed by reaction of a molecule which already contains the moiety configured to provide a layer of organic and/or hydrophilic molecules suitable for maintenance/expansion of biological micro-object(s) in the microfluidic device, as is described below. Alternatively, the covalently linked coating material may be formed in a two-part sequence by coupling the moiety configured to provide a layer of organic and/or hydrophilic molecules suitable for maintenance/expansion of biological micro-object(s) to a surface modifying ligand that itself has been covalently linked to the surface.

**Methods of preparing a covalently linked coating material.** In some embodiments, a coating material that is covalently linked to the surface of a microfluidic device (e.g., including at least one surface of the sequestration pens and/or flow paths) has a structure of Formula 1 or Formula 2. When the coating material is introduced to the surface in one step, it has a structure of Formula 1, while when the coating material is introduced in a multiple step process, it has a structure of Formula 2.

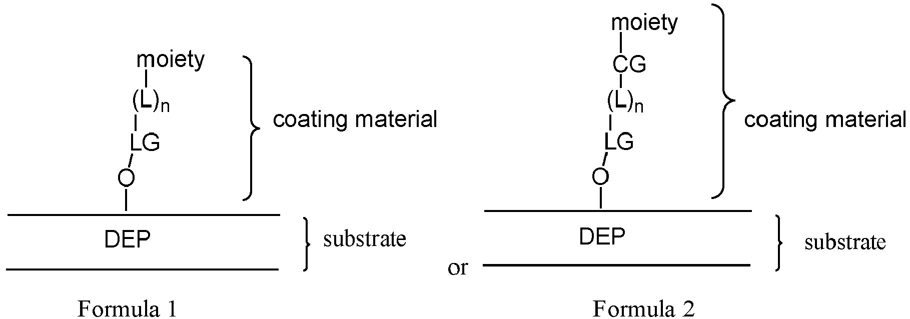

The coating material may be linked covalently to oxides of the surface of a DEP-configured or EW- configured substrate. The DEP- or EW- configured substrate may comprise silicon, silicon oxide, alumina, or hafnium oxide. Oxides may be present as part of the native chemical structure of the substrate or may be introduced as discussed below.

The coating material may be attached to the oxides via a linking group ("LG"), which may be a siloxy or phosphonate ester group formed from the reaction of a siloxane or phosphonic acid group with the oxides. The moiety configured to provide a layer of organic and/or hydrophilic molecules suitable for maintenance/expansion of biological micro-object(s) in the microfluidic device can be any of the moieties described herein. The linking group LG may be directly or indirectly connected to the moiety configured to provide a layer of organic and/or hydrophilic molecules suitable for maintenance/expansion of biological micro-object(s) in the microfluidic device. When the linking group LG is directly connected to the moiety, optional linker ("L") is not present and n is 0. When the linking group LG is indirectly connected to the moiety, linker L is present and n is 1. The linker L may have a linear portion where a backbone of the linear portion may include 1 to 200 non-hydrogen atoms selected from any combination of silicon, carbon, nitrogen, oxygen, sulfur and phosphorus atoms, subject to chemical bonding limitations as is known in the art. It may be interrupted with any combination of one or more moieties selected from the group consisting of ether, amino, carbonyl, amido, or phosphonate groups, arylene, heteroarylene, or heterocyclic groups. In some embodiments, the backbone of the linker L may include 10 to 20 atoms. In other embodiments, the backbone of the linker L may include about 5 atoms to about 200 atoms; about 10 atoms to about 80 atoms; about 10 atoms to about 50 atoms; or about 10 atoms to about 40 atoms. In some embodiments, the backbone atoms are all carbon atoms.

In some embodiments, the moiety configured to provide a layer of organic and/or hydrophilic molecules suitable for maintenance/expansion of biological micro-object(s) may be added to the surface of the substrate in a multi-step process, and has a structure of Formula 2, as shown above. The moiety may be any of the moieties described above.

In some embodiments, the coupling group CG represents the resultant group from reaction of a reactive moiety Rₓ and a reactive pairing moiety Rₚₓ (i.e., a moiety configured to react with the reactive moiety Rₓ). For example, one typical coupling group CG may include a carboxamidyl group, which is the result of the reaction of an amino group with a derivative of a carboxylic acid, such as an activated ester, an acid chloride or the like. Other CG may include a triazolylene group, a carboxamidyl, thioamidyl, an oxime, a mercaptyl, a disulfide, an ether, or alkenyl group, or any other suitable group that may be formed upon reaction of a reactive moiety with its respective reactive pairing moiety. The coupling group CG may be located at the second end (i.e., the end proximal to the moiety configured to provide a layer of organic and/or hydrophilic molecules suitable for maintenance/expansion of biological micro-object(s) in the microfluidic device) of linker L, which may include any combination of elements as described above. In some other embodiments, the coupling group CG may interrupt the backbone of the linker L. When the coupling group CG is triazolylene, it may be the product resulting from a Click coupling reaction and may be further substituted (e.g., a dibenzocylcooctenyl fused triazolylene group).

In some embodiments, the coating material (or surface modifying ligand) is deposited on the inner surfaces of the microfluidic device using chemical vapor deposition. The vapor deposition process can be optionally improved, for example, by pre-cleaning the cover 110, the microfluidic circuit material 116, and/or the substrate (e.g., the inner surface 208 of the electrode activation substrate 206 of a DEP-configured substrate, or a dielectric layer of the support structure 104 of an EW-configured substrate), by exposure to a solvent bath, sonication or a combination thereof. Alternatively, or in addition, such pre-cleaning can include treating the cover 110, the microfluidic circuit material 116, and/or the substrate in an oxygen plasma cleaner, which can remove various impurities, while at the same time introducing an oxidized surface (e.g. oxides at the surface, which may be covalently modified as described herein). Alternatively, liquid-phase treatments, such as a mixture of hydrochloric acid and hydrogen peroxide or a mixture of sulfuric acid and hydrogen peroxide (e.g., piranha solution, which may have a ratio of sulfuric acid to hydrogen peroxide in a range from about 3:1 to about 7:1) may be used in place of an oxygen plasma cleaner.

In some embodiments, vapor deposition is used to coat the inner surfaces of the microfluidic device 200 after the microfluidic device 200 has been assembled to form an enclosure 102 defining a microfluidic circuit 120. Without intending to be limited by theory, depositing such a coating material on a fully-assembled microfluidic circuit 120 may be beneficial in preventing delamination caused by a weakened bond between the microfluidic circuit material 116 and the electrode activation substrate 206 dielectric layer and/or the cover 110. In embodiments where a two-step process is employed the surface modifying ligand may be introduced via vapor deposition as described above, with subsequent introduction of the moiety configured provide a layer of organic and/or hydrophilic molecules suitable for maintenance/expansion of biological micro-object(s). The subsequent reaction may be performed by exposing the surface modified microfluidic device to a suitable coupling reagent in solution.

Figure 2H depicts a cross-sectional views of a microfluidic device 290 having an exemplary covalently linked coating material providing a conditioned surface. As illustrated, the coating materials 298 (shown schematically) can comprise a monolayer of densely-packed molecules covalently bound to both the inner surface 294 of a base 286, which may be a DEP substrate, and the inner surface 292 of a cover 288 of the microfluidic device 290. The coating material 298 can be disposed on substantially all inner surfaces 294, 292 proximal to, and facing inwards towards, the enclosure 284 of the microfluidic device 290, including, in some embodiments and as discussed above, the surfaces of microfluidic circuit material (not shown) used to define circuit elements and/or structures within the microfluidic device 290. In alternate embodiments, the coating material 298 can be disposed on only one or some of the inner surfaces of the microfluidic device 290.

In the embodiment shown in Figure 2H, the coating material 298 can include a monolayer of organosiloxane molecules, each molecule covalently bonded to the inner surfaces 292, 294 of the microfluidic device 290 via a siloxy linker 296. Any of the above-discussed coating materials 298 can be used (e.g. an alkyl-terminated , a fluoroalkyl terminated moiety, a PEG- terminated moiety, a dextran terminated moiety, or a terminal moiety containing positive or negative charges for the organosiloxy moieties), where the terminal moiety is disposed at its enclosure-facing terminus (i.e. the portion of the monolayer of the coating material 298 that is not bound to the inner surfaces 292, 294 and is proximal to the enclosure 284).

In other embodiments, the coating material 298 used to coat the inner surface(s) 292, 294 of the microfluidic device 290 can include anionic, cationic, or zwitterionic moieties, or any combination thereof. Without intending to be limited by theory, by presenting cationic moieties, anionic moieties, and/or zwitterionic moieties at the inner surfaces of the enclosure 284 of the microfluidic circuit 120, the coating material 298 can form strong hydrogen bonds with water molecules such that the resulting water of hydration acts as a layer (or "shield") that separates the biological micro-objects from interactions with non-biological molecules (e.g., the silicon and/or silicon oxide of the substrate). In addition, in embodiments in which the coating material 298 is used in conjunction with coating agents, the anions, cations, and/or zwitterions of the coating material 298 can form ionic bonds with the charged portions of non-covalent coating agents (e.g. proteins in solution) that are present in a medium 180 (e.g. a coating solution) in the enclosure 284.

In still other embodiments, the coating material may comprise or be chemically modified to present a hydrophilic coating agent at its enclosure-facing terminus. In some embodiments, the coating material may include an alkylene ether containing polymer, such as PEG. In some embodiments, the coating material may include a polysaccharide, such as dextran. Like the charged moieties discussed above (e.g., anionic, cationic, and zwitterionic moieties), the hydrophilic coating agent can form strong hydrogen bonds with water molecules such that the resulting water of hydration acts as a layer (or "shield") that separates the biological micro-objects from interactions with non-biological molecules (e.g., the silicon and/or silicon oxide of the substrate).

Further details of appropriate coating treatments and modifications may be found at U.S. Patent Application Publication No. US2016/0312165, which is incorporated by reference in its entirety.

**Additional system components for maintenance of viability of cells within the sequestration pens of the microfluidic device.** In order to promote growth and/or expansion of cell populations, environmental conditions conducive to maintaining functional cells may be provided by additional components of the system. For example, such additional components can provide nutrients, cell growth signaling species, pH modulation, gas exchange, temperature control, and removal of waste products from cells. These types of additional components have been described, for example, in U.S. Patent Application Publication No. US2016/0312165.

### EXAMPLES

### Example 1: Human T Cell Expansion in an OptoSelect^{™} Chip

T cell expansion was achieved within an OptoSelect chip, a nanofluidic device manufactured by Berkeley Lights, Inc. and controlled by an optical instrument which was also manufactured by Berkeley Lights, Inc. The instrument included: a mounting stage for the chip coupled to a temperature controller; a pump and fluid medium conditioning component; and an optical train including a camera and a structured light source suitable for activating phototransistors within the chip. The OptoSelect^{™} chip included a substrate configured with OptoElectroPositioning (OEP^{™}) technology, which provides a phototransistor-activated OET force. The chip also included a plurality of microfluidic channels, each having a plurality of NanoPen^{™} chambers (or sequestration pens) fluidically connected thereto. The volume of each sequestration pen was around 1x10⁶ cubic microns.

CD3⁺ human T lymphocytes isolated from peripheral blood were mixed with anti-CD3/anti-CD28 magnetic beads (DYNABEADS^{™}, Thermo Fisher Scientific, Inc.) at a ratio of 1 bead/1 cell. The mixture was incubated for 5 hours in a 5% CO₂ incubator at 37°C. Following the incubation, the T cell/bead mixture was resuspended, then flowed through a fluidic inlet and into the microfluidic channels within the chip. The flow was stopped and T cells/beads were randomly loaded into sequestration pens by tilting the chip and allowing gravity to pull the T cells/beads into the pens.

After loading the T cells and beads into the sequestration pens, T cell culture medium (RPMI, 10% FBS, 2% Human AB serum, 50 U/ml IL2; R&D Systems) was perfused through the microfluidic channels of the chip for a period of 4 days. The sequestration pens, and any T cells and beads contained therein, were imaged every 30 minutes for the entire 4-day culture period.

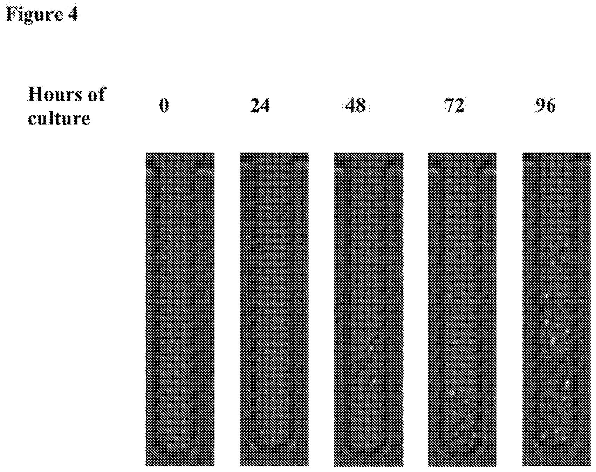

Figure 4 is a series of time-lapse images of a single pen at 0, 24, 48, 72, and 96 hours of culture, in which CD3⁺ human T lymphocytes were successfully expanded on chip in accordance with the foregoing method. As can be seen, a small number of T cells at time t=0 resulted in an oligo-clonal population of T cells after 96 hours of on-chip culture.

Alternative 1: the foregoing experiment could be repeated with an animal component-free T cell culture medium. For example, the FBS in the foregoing T cell culture medium could be removed and the RPMI could be replaced with advanced RPMI (or a similar base medium having high levels of phosphate and including insulin and ferritin supplements) and the resulting T cell expansion would be substantially the same. In addition, the T cell culture medium could be supplemented with IL7 (e.g., 5 ng/ml of IL7).

Alternative 2: the foregoing experiment could be repeated without the 5 hour off-chip pre-incubation of the T cells and beads. Instead, one or more beads could be placed in each sequestration pen, either before or after loading the T cells into the sequestration pens, and the resulting T cell expansion would be substantially the same.

### Example 2: Selective Expansion of Human T Cells in an OptoSelect^{™} Chip

T cell expansion was achieved within an OptoSelect chip (Berkeley Lights, Inc.), which was controlled by an optical instrument also manufactured by Berkeley Lights, Inc., as described in Example 1.

Initially, human CD14⁺ monocytes isolated from peripheral blood were cultured for 7 days in DC culture medium (RPMI, 10% FBS, 2% Human AB serum, 100 ng/ml GM-CSF, 50 ng/ml IL-4; R&D Systems) to promote differentiation of dendritic cells (DCs). 250 µg/ml LPS (R&D Systems) was added to the culture medium during the last 2 days of culture to promote DC activation.

Allogeneic donor T lymphocytes were mixed with DCs from the foregoing culture at a ratio of ∼10 T cells/1 DC and incubated for 5 hours in a 5% CO₂ incubator at 37°C. Following the incubation, the T cells/DCs mixture was resuspended, then flowed through a fluidic inlet and into the microfluidic channels within the chip. The flow was stopped and T cells/DCs were randomly loaded into sequestration pens by tilting the chip and allowing gravity to pull the T cells/DCs into the pens.

After loading the T cells/DCs into sequestration pens, T cell culture medium (RPMI, 10% FBS, 2% Human AB serum, 50 U/ml IL2; R&D Systems) was perfused through the microfluidic channels of the chip for a period of 4 days. The sequestration pens, and any T cells and DCs contained therein, were imaged every 30 minutes for the entire 4-day culture period.

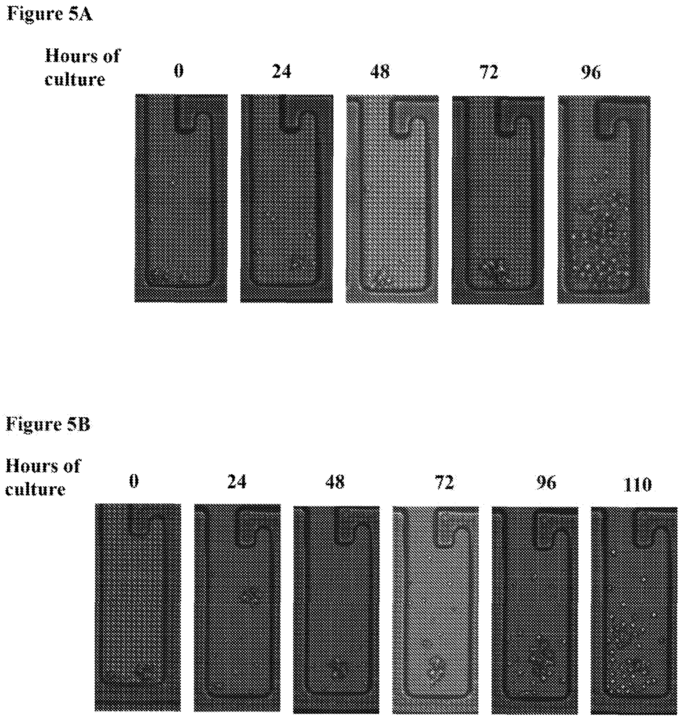

Figure 5A is a series of time-lapse images of a single sequestration pen at 0, 24, 48, 72, and 96 hours of culture, in which CD3⁺ human T lymphocytes were selectively expanded on chip in accordance with the foregoing method. As can be seen, the DCs stimulated significant expansion of the T cells after 96 hours of on-chip culture. However, only 1%-2% of sequestration pens initially seeded with at least one T cell and at least one DC exhibited T cell expansion, demonstrating that the expansion observed in Figure 5A was selective.

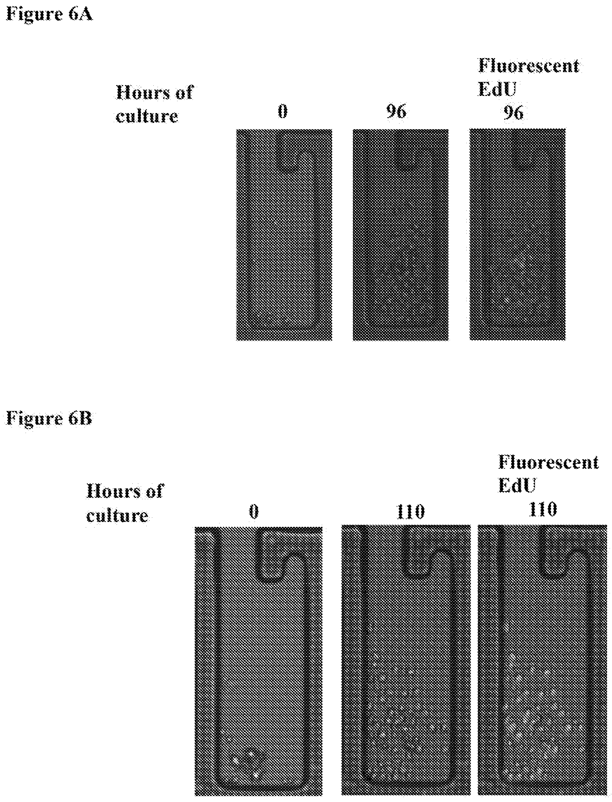

During the last 16 hours of the 4-day culture period, the culture medium used to perfuse the chip was supplemented with Click-It EdU reagent (Thermo Fisher Scientific, Inc.), allowing the T cells to take up the reagent and incorporate it into their DNA. Following the culture period, the cells were washed, fixed with 3.7% formaldehyde, and permeabilized with 0.1% Triton-X. EdU incorporation was detected by monitoring fluorescence in the Texas Red channel. Figure 6A provides an image showing the EdU fluorescence signal overlaid on a bright-field image of the selectively expanded T cells. The EdU data shows that the increase in the number of T cells in the pen resulted from cell growth and division, consistent with the conclusion that the originally-penned T cells were activated by the DCs.

Alternative: the foregoing experiment could be repeated with an animal component-free T cell culture medium. For example, the FBS in the foregoing T cell culture medium could be removed and the RPMI could be replaced with advanced RPMI (or a similar base medium having high levels of phosphate and including insulin and ferritin supplements) and the resulting T cell expansion would be substantially the same. In addition, the T cell culture medium could be supplemented with IL7 (e.g., 5 ng/ml of IL7).

### Example 3: Antigen-Specific Expansion of Human T Cells in an OptoSelect^{™} Chip

T cell expansion was achieved within an OptoSelect chip (Berkeley Lights, Inc.), which was controlled by an optical instrument also manufactured by Berkeley Lights, Inc., as described in Example 1.

Initially, human CD14⁺monocytes isolated from peripheral blood were cultured for 7 days in DC culture medium (RPMI, 10% FBS, 2% Human AB serum, 100 ng/ml GM-CSF, 50 ng/ml IL-4; R&D Systems) to promote differentiation of dendritic cells (DCs). 250 µg/ml LPS (R&D Systems) was added to the culture medium during the last 2 days of culture to promote DC activation. At the same time as the addition of the LPS, the DCs were also pulsed with 10 µM Tetanus toxin (TT) antigen (Sigma-Aldrich Co.) and 10 µM Epstein Barr Virus (EBV) antigen (EastCoast Bio, Inc.).

Autologous donor T lymphocytes were mixed with TT- and EBV-pulsed DCs from the foregoing culture at a ratio of ∼10 T cells/1 DC and incubated for 5 hours in a 5% CO₂ incubator at 37°C. Following the incubation, the T cells/DCs mixture was resuspended, then flowed through a fluidic inlet and into the microfluidic channels within the chip. The flow was stopped and T cells/DCs were randomly loaded into sequestration pens by tilting the chip and allowing gravity to pull the T cells/DCs into the pens.

After loading the T cells/DCs into sequestration pens, T cell culture medium (RPMI, 10% FBS, 2% Human AB serum, 50 U/ml IL2; R&D Systems) was perfused through the microfluidic channels of the chip for a period of 5 days. The pens and any T cells and beads contained therein were imaged every 30 minutes for the entire 5-day culture period.

Figure 5B is a time-lapse series of images of a single sequestration pen at 0, 24, 48, 72, 96, and 110 hours of culture, in which CD3⁺ human T lymphocytes were selectively expanded on chip in accordance with the foregoing method. As seen, the DCs stimulated significant expansion of the T cells after 110 hours of on-chip culture. However, only 1%-2% of pens initially seeded with at least one T cell and at least one DC exhibited T cell expansion, demonstrating that the expansion observed in Figure 5B was antigen specific.

During the last 16 hours of the 5-day culture period, the culture medium used to perfuse the chip was supplemented with Click-It EdU reagent (Thermo Fisher Scientific, Inc.), allowing the T cells to take up the reagent and incorporate it into their DNA. Following the culture period, the cells were washed, fixed with 3.7% formaldehyde, and permeabilized with 0.1% Triton-X. EdU incorporation was detected by monitoring fluorescence in the Texas Red channel. Figure 6B provides an image showing the EdU fluorescence signal overlaid on a bright-field image of the selectively expanded T cells. The EdU data shows that the increase in the number of T cells in the pen resulted from cell growth and division, consistent with the conclusion that the originally-penned T cells were activated by the DCs.

Alternative: the foregoing experiment could be repeated with an animal component-free T cell culture medium. For example, the FBS in the foregoing T cell culture medium could be removed and the RPMI could be replaced with advanced RPMI (or a similar base medium having high levels of phosphate and including insulin and ferritin supplements) and the resulting T cell expansion would be substantially the same. In addition, the T cell culture medium could be supplemented with IL7 (e.g., 5 ng/ml of IL7).

### Example 4: Culturing and Export of T Lymphocytes in an OptoSelect^{™} Chip having a Conditioned Surface

Materials. CD3+ cells were from AllCells Inc. Anti-CD3/anti-CD28 antibodies were bound to magnetic beads (Dynabeads^{®}, Thermofisher Scientific, Cat. No. 11453D). Culture medium was RPMI-1640 (GIBCO^{®}, ThermoFisher Scientific, Cat. No. 11875-127), supplemented with 10% FBS, 2% Human AB serum, and 50 U/ml IL2 (R&D Systems).

System and Microfluidic Device. T cell expansion was achieved within an OptoSelect chip (Berkeley Lights, Inc.), which was controlled by an optical instrument also manufactured by Berkeley Lights, Inc., substantially as described in Example 1. In this example, however, the internal surfaces of the OptoSelect chip were conditioned with covalently-linked dextran.

Microfluidic device priming. 250 microliters of 100% carbon dioxide was flowed in at a rate of 12 microliters/sec, followed by 250 microliters of PBS containing 0.1% Pluronic^{®} F27 (Life Technologies^{®} Cat# P6866) flowed in at 12 microliters/sec, and finally 250 microliters of PBS flowed in at 12 microliters/sec. Introduction of the culture medium follows.

Media perfusion. Medium was perfused through the microfluidic device according to either of the following two methods:
1. Perfuse at 0.01 microliters/sec for 2h; perfuse at 2 microliters/sec for 64 sec; and repeat.
2. Perfuse at 0.02 microliters/sec for 100 sec; stop flow 500 sec; perfuse at 2 microliters/sec for 64 sec; and repeat.

Experiment: CD3+ cells were mixed with anti-CD3/anti-CD28 magnetic beads at a ratio of 1 bead/cell. The mixture was incubated in culture medium for 5 hours in a 5% CO₂ incubator at 37°C, after which the T cell/bead mixture was resuspended for use.

The T cell suspension (including anti-CD3/anti-CD28 beads) was introduced into the microfluidic device by flowing the resuspension through a fluidic inlet and into the microfluidic channel. The flow was stopped and T cells/beads were randomly loaded into sequestration chambers by tilting the chip and allowing gravity to pull the T cells/beads into the chambers.

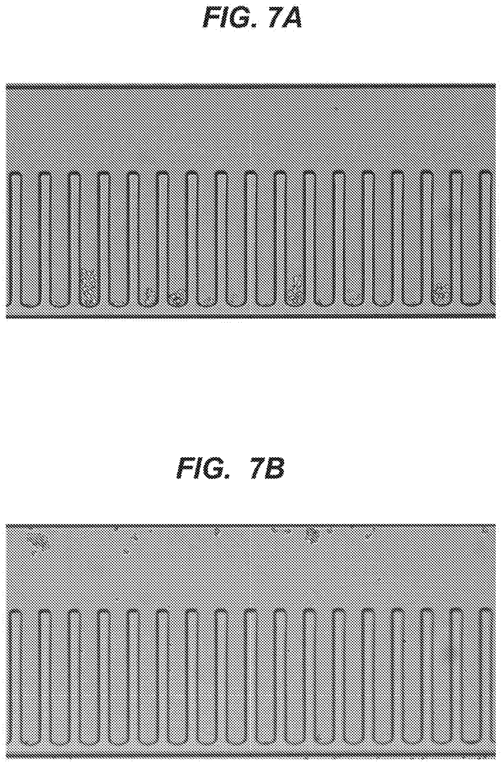

After loading the T cells/beads into the sequestration chambers, the culture medium was perfused through the microfluidic channel of the nanofluidic chip for a period of 4 days. Figure 7A showed the growth of T cells on the dextran conditioned surface of the sequestration chambers of the microfluidic device. The growth of T cell on the dextran-conditioned surface was improved relative to a non-conditioned surface of a similar microfluidic device (data not shown).

The T cells were then removed from the sequestration chambers by tilting the microfluidic device and allowing gravity to pull the T cells from the chambers. Figure 7B shows a representative image of T cells removed from sequestration chambers following twenty minutes of gravity-unloading. The expanded T cells were readily exported from the dextran-conditioned sequestration pens, which was an improvement over the extent of T cell export achieved using an OptoSelect chip that lacked a dextran-conditioned surface (data not shown).

### Example 5: Activation and Expansion of T Lymphocytes in an OptoSelect^{™} Chip having a Conditioned Surface

T cell expansion was achieved within an OptoSelect chip (Berkeley Lights, Inc.), which was controlled by an optical instrument also manufactured by Berkeley Lights, Inc., substantially as described in Example 1. Similar to Example 4, the OptoSelect chip featured a conditioned surface. In this example, the conditioned surface included a streptavidin-linked polyethylene glycol (PEG, ∼1kD)-containing polymer, which was bound to biotinylated anti-CD3 agonist antibodies.

The OptoSelect chip was flushed with free streptavidin (1 mg/mL, loaded by injection into outlet port), incubated at RT for 1 hour, then rinsed with PBS (by injection) x1. Next, biotinylated anti-CD3 antibody (5 micrograms/mL, Miltenyi 130-093-377) was flowed into the chip, flow was stopped, and the chip was incubated for 1 hour at 37°C with humidification. The chip was then rinsed by flowing culture medium through the device.

T cells were loaded into the chip (prepared as described above) by flowing a culture of T cells through an inlet and into the microfluidic channels within the chip, then stopping flow. The chip was tilted to allow gravity loading of T cells into sequestration pens in the chip. Following gravity loading, the T cells were cultured within the sequestration pens of the chip for 3 to 4 days. The medium used for T cell culture contained 2 ug/mL soluble, functional grade anti-CD28 antibody (clone 15E8, Miltenyi 130-093-375).

T cells cultured in the microfluidic device as described above were monitored by time-lapse imaging. The resulting images revealed that the T cells moved around and divided, thereby evidencing characteristics of T cell activation.

Variations of the foregoing experiment could include: changing the surface density of bound anti-CD3 agonist antibody; changing the length of the PEG polymer; binding the anti-CD28 antibody to the conditioned surface; varying the ratio of anti-CD3 agonist antibody-to-anti-CD28 antibody; and/or replacing the anti-CD3 antibody with a peptide-MHC complex. This latter modification could be used to achieve antigen-specific activation and expansion of T cells.

### Example 6: Introduction of a T-cell Activating Surface within a Microfluidic Device

The internal surfaces of an OptoSelect^{™} chip (Berkeley Lights, Inc.), which included a first silicon electrode activation substrate forming a base, a second ITO substrate forming a cover, and photopatterned silicone microfluidic circuit material forming walls separating the two substrates, were covalently modified to include azido moieties, as described in U.S. Patent Application Publication No. US20160312165. Basically, the OptoSelect chip was treated in an oxygen plasma cleaner (Nordson Asymtek) for 1 min, using 100W power, 240 mTorr pressure and 440 sccm oxygen flow rate. The plasma treated chip was treated in a vacuum reactor with 3-azidoundecyl) trimethoxysilane (synthesized from 11-bromoundecyltrimethoxysilane (Gelest Cat. # SIB1908.0) by reaction with sodium azide, 300 microliters) in a foil boat in the bottom of the vacuum reactor, in the presence of magnesium sulfate heptahydrate (0.5g, Acros Cat. # 10034-99-8) as a water reactant source in a separate foil boat in the bottom of the vacuum reactor. The chamber was then pumped to 750 mTorr using a vacuum pump and sealed. The vacuum reactor was placed within an oven heated to 110°C for 24-48 h. This resulted in modification of the internal surfaces of the chip, such that the modified surfaces had the structure:

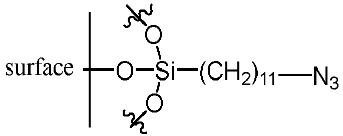

After cooling to room temperature and introducing argon to the evacuated chamber, the microfluidic device was removed from the reactor and was suitable for further reaction.

Next, to functionalize the surface with streptavidin, the OptoSelect chip was first flushed repeatedly with 100% carbon dioxide, and then loaded with a DBCO-streptavidin solution. The DBCO-streptavidin solution was made by resuspending a lyophilized power of streptavidin that was covalently attached to DBCO moieties (NANOCS Cat # SV1-DB-1) to a 1.0 micromolar concentration in IX PBS (pH 7.4, Gibco). After incubation for 15-30 minutes, during which the DBCO and azide groups coupled, the OptoSelect chip was washed repeatedly with IX PBS to flush out unbound DBCO-streptavidin. While the DBCO modified streptavidin solution was prepared at a 1 micromolar concentration, a concentration in the range of about 0.5 to about 2 micromolar is effective for modifying the internal surfaces of an OptoSelect chip having azido moieties.

This streptavidin-functionalized surfaces were further modified with a biotinylated anti-CD3 antibody (OKT3 clone, functional grade, Miltenyi, Cat. #130-093-377) and a biotinylated anti-CD28 antibody (15E8 clone, functional grade, Miltenyi, Cat. #130-093-386). These antibodies were suspended in PBS + 2% Bovine Serum Albumin, at concentrations of about 1 - 10 micrograms/mL, in a 1:1 ratio. This antibody solution was perfused through the OptoSelect chip with streptavidin-functionalized surfaces, facilitating conjugation of the antibodies to the internal surfaces of the chip via the biotin-streptavidin binding interaction. After one hour of incubation, the OptoSelect chip was flushed with PBS and then cell culture medium, at which point the chip was ready for cell loading.

Although the foregoing example focuses on the functionalization of the internal surfaces of the OptoSelect chip with antibodies, other biomolecules of interest could be conjugated in the same manner (i.e., via biotin modification of the biomolecules and biotin-streptavidin mediated binding to streptavidin functionalized surfaces). Moreover, biotinylated antibodies (or other biomolecules) could be reacted with streptavidin prior to reaction with the azido-modified surfaces of the OptoSelect chip. DBCO-streptavidin and biotinylated biomolecule can be prepared separately in PBS solution at concentrations in the range of 0.5 - 2 micromolar, then mixed at any desired ratio, as described below. After allowing the biotinylated biomolecules to conjugate to the streptavidin for at least 15 minutes, the resulting complexes can be used to modify the surface of an azido-modified OptoSelect chip, as described above.

If more than one type of antibody/biomolecule is used to modify the surfaces of the OptoSelect chip, mixing of the two (or more) types of antibodies/biomolecules can occur prior to the conjugation step with DBCO-streptavidin, or DBCO-streptavidin conjugates of several individual types of biomolecules can be prepared and mixed prior to surface functionalization. Thus, while the biomolecule modified surface in the above Example was a 1:1 ratio of anti-CD3 and anti-CD28 antibodies, the ratio could be about 1:5 to about 5:1 (e.g., about 2:1 to about 1:2). More generally, biomolecule-modified surfaces useful for activating T-cells may include two T cell-activating biomolecules, either of which could be a CD3 agonist, a CD28 agonist, or a MHC protein (e.g., MBL International, Catalog # MR01008), and the two biomolecules could be present in a range of about 1:10 to about 10:1 relative to one another. Moreover, the biomolecule-modified surfaces could include mixtures of 3, 4, 5, or more T cell-activating biomolecules.

Further variations of the foregoing experiment could include: changing the surface density of bound anti-CD3 agonist antibody and/or bound anti-CD28 agonist antibody; changing the length of the hydrocarbon chain in the azido moiety-containing molecules used to initially functionalize the internal chip surfaces; providing the anti-CD28 antibody in soluble form; and/or replacing the anti-CD3 antibody with a peptide-MHC complex. This latter modification could be used to achieve antigen-specific activation and expansion of T cells.

### Example 7: Embodiments

The following numbered items provide further nonlimiting details on the embodiment described herein.
Item 1. A method of expanding T lymphocytes in a microfluidic device having a flow path and a sequestration pen fluidically connected to the flow path, the method comprising:
   introducing one or more T lymphocytes into the sequestration pen in the microfluidic device;
   contacting the one or more T lymphocytes with an activating agent; and
   perfusing culture medium through the flow path of the microfluidic device for a period of time sufficient to allow the one or more T lymphocytes introduced into the sequestration pen to undergo expansion.
Item 2. The method of item 1, wherein the sequestration pen has a volume of about 5x105 to about 5x106 cubic microns.
Item 3. The method of item 1 or 2, wherein at least one inner surface of the sequestration pen comprises a coating material.
Item 4. The method of item 3, wherein the coating material is covalently bound to the at least one inner surface of the sequestration pen, and wherein the coating material comprises a polymer comprising alkylene ether moieties, saccharide moieties, amino acid moieties, or a combination thereof.
Item 5. The method of item 4, wherein the coating material comprises dextran.
Item 6. The method of item 4, wherein the coating material comprises poly-ethylene glycol moieties.
Item 7. The method of any one of items 4 to 6, wherein the coating material comprises one or more proteins.
Item 8. The method of item 3, wherein the coating material comprises molecules, each of which includes a linking group and an alkyl moiety, wherein the linking group is covalently bonded to the at least one inner surface of the sequestration pen.
Item 9. The method of item 8, wherein the linking group is a siloxy linking group.
Item 10. The method of item 8 or 9, wherein the alkyl moiety comprises a linear chain of carbons comprising at least 10 carbon atoms.
Item 11. The method of any one of items 8 to 10, wherein the alkyl moiety is a fluoroalkyl moiety.
Item 12. The method of any one of items 8 to 10, wherein the alkyl moiety is a perflouroalkyl moiety.
Item 13. The method of any one of items 8 to 12, wherein the molecules of the coating material form a densely-packed monolayer structure covalently bound to the inner substrate surface.
Item 14. The method of item 3, wherein the coating material comprises molecules having a linking group and a cationic moiety and/or an anionic moiety, wherein the linking group is covalently bonded to the inner substrate surface.
Item 15. The method of item 14, wherein the cationic moiety comprises a quaternary ammonium group.
Item 16. The method of item 14 or 15, wherein the anionic moiety comprises a phosphonic acid, carboxylic acid, or sulfonic acid.
Item 17. The method of any one of items 14 to 16, wherein the coating material comprises molecules having a linking group and a zwitterionic moiety.
Item 18. The method of item 17, wherein the zwitterionic moiety is selected from carboxybetaines, sulfobetaines, sulfamic acids, and amino acids.
Item 19. The method of any one of items 3 to 18, wherein the activating agent is covalently linked to the coating material.
Item 20. The method of any one of items 3 to 18, wherein the activating agent is stably bound to the coating material.
Item 21. The method of item 20, wherein the activating agent is stably bound to the coating material via a biotin-streptavidin linkage.
Item 22. The method of any one of items 1 to 21, wherein the one or more T lymphocytes are isolated from a peripheral blood sample taken from a subject.
Item 23. The method of any one of items 1 to 21, wherein the one or more T lymphocytes are isolated from a solid tumor sample of a subject.
Item 24. The method of item 23, wherein the solid tumor sample is a fine needle aspirate (FNA).
Item 25. The method of item 23, wherein the solid tumor sample is a biopsy.
Item 26. The method of any one of items 23 to 25, wherein the solid tumor is a breast cancer, a cancer originating in the urinary tract, ureter, bladder, or urethra, renal cell carcinoma, testicular cancer, prostate cancer, or a cancer of the seminal vesicles, seminal ducts, or penis, ovarian cancer, uterine cancer, cervical cancer, vaginal cancer, or a cancer of the fallopian tubes, a cancer of the nervous system, neuroblastoma, retinoblastoma, intestinal cancer, colorectal cancer, lung cancer, or melanoma.
Item 27. The method of any one of items 23 to 25, wherein the solid tumor is a medullary breast cancer, a mesothelioma, or a melanoma.
Item 28. The method of any one of items 22 to 27, wherein the one or more T lymphocytes are from a population of T lymphocytes isolated from the peripheral blood sample or the solid tumor sample.
Item 29. The method of item 28, wherein the population is enriched for CD3+ T lymphocytes.
Item 30. The method of item 28, wherein the population is enriched for CD3+CD4+ T lymphocytes.
Item 31. The method of item 28, wherein the population is enriched for CD3+CD8+ T lymphocytes.
Item 32. The method of any one of items 28 to 31, wherein the population is enriched for CD45RA+CD45RO- T lymphocytes.
Item 33. The method of any one of items 28 to 31, wherein the population is enriched for CD45RA-CD45RO+ T lymphocytes.
Item 34. The method of any one of items 28 to 33, wherein the population is enriched for CCR7+ T lymphocytes.
Item 35. The method of any one of items 28 to 34, wherein the population is enriched for CD62L+ T lymphocytes.
Item 36. The method of any one of items 28 to 35, wherein the population is depleted of CD69+ T lymphocytes.
Item 37. The method of any one of items 28 to 36, wherein the population is depleted of PD 1+ and/or PD-L1+ T lymphocytes.
Item 38. The method of any one of items 28 to 37, wherein the method comprises depleting one, two, three, or four of CD45RO+ T lymphocytes, CD45RA+ T lymphocytes, CD69+ T lymphocytes, PD-1+ T lymphocytes, and PD-L1+ T lymphocytes by contacting the T lymphocytes with antibodies to CD45RO, CD45RA, CD69, PD-1, and/or PD-L1 and removing from the population T lymphocytes bound to the antibodies.
Item 39. The method of item 38, wherein the antibodies are associated with a solid support.
Item 40. The method of item 39, wherein the solid support is a population of magnetic beads.
Item 41. The method of any one of items 1 to 40, wherein introducing the one or more T lymphocytes into the sequestration pen comprises flowing a fluid containing the one or more T lymphocytes into a microfluidic channel of the microfluidic device, wherein the microfluidic channel is part of the flow path of the microfluidic device, and wherein the sequestration pen opens off of the microfluidic channel.
Item 42. The method of item 41, wherein introducing the one or more T lymphocytes into the sequestration pen further comprises using dielectrophoresis (DEP) to select at least one T lymphocyte located in the microfluidic channel and move it into the sequestration pen.
Item 43. The method of item 42, wherein the at least one selected T lymphocyte is selected, at least in part, because its cell surface comprises the marker CD3+, CD4+, CD8+, or any combination thereof.
Item 44. The method of item 42, wherein the at least one selected T lymphocyte is selected, at least in part, because its cell surface comprises the markers CD3+ and CD4+.
Item 45. The method of item 42, wherein the at least one selected T lymphocyte is selected, at least in part, because its cell surface comprises the markers CD3+ and CD8+.
Item 46. The method of any one of items 42 to 45, wherein the at least one selected T lymphocyte is selected, at least in part, because its cell surface comprises the marker(s) CD45RA+, CD45RO+, CCR7+, CD62L+, or any combination thereof.
Item 47. The method of any one of items 42 to 46, wherein selecting the at least one T lymphocyte comprises labeling a population of T lymphocyte that comprises the at least one T lymphocyte with an antibody that specifically binds to CD3, CD4, CD8, CD45RA, CD45RO, CCR7, or CD62L, and moving the at least one T lymphocyte into the sequestration pen based upon its association with the antibody.
Item 48. The method of item 47, wherein the antibody comprises a fluorophore.
Item 49. The method of any one of items 42 to 48, wherein the at least one selected T lymphocyte is selected, at least in part, because its cell surface is not CD45RO+.
Item 50. The method of item 42 to 48, wherein introducing one or more T lymphocytes into the sequestration pen comprises labeling CD45RO+ T lymphocytes and introducing one or more T lymphocytes not associated with a label indicative of CD45RO into the sequestration pen.
Item 51. The method of any one of items 42 to 48, wherein the at least one selected T lymphocyte is selected, at least in part, because its cell surface is not CD45RA+.
Item 52. The method of any one of items 42 to 48, wherein introducing one or more T lymphocytes into the sequestration pen comprises labeling CD45RA+ T lymphocytes and introducing one or more T lymphocytes not associated with a label indicative of CD45RA into the sequestration pen.
Item 53. The method of any one of items 42 to 52, comprising labeling CCR7+ and CD62L+ T lymphocytes and moving one or more T lymphocytes associated with a label indicative of CCR7 and/or associated with a label indicative of CD62L into the sequestration pen.
Item 54. The method of any one of items 42 to 52, comprising labeling CCR7+ and CD62L+ T lymphocytes and moving one or more T lymphocytes not associated with a label indicative of CCR7 and/or not associated with a label indicative of CD62L into the sequestration pen.
Item 55. The method of any one of items 42 to 54, wherein the at least one selected T lymphocyte is selected, at least in part, because its cell surface is not CD69+.
Item 56. The method of any one of items 42 to 55, wherein the at least one selected T lymphocyte is selected, at least in part, because its cell surface is not PD-1+.
Item 57. The method of any one of items 42 to 56, wherein the at least one selected T lymphocyte is selected, at least in part, because its cell surface is not PD-L1+.
Item 58. The method of any one of items 42 to 57, wherein the at least one selected T lymphocyte is selected, at least in part, because it is labeled with an antigen associated with a fluorescent label.
Item 59. The method of item 58, wherein the antigen comprises a peptide which is complexed with a MHC protein.
Item 60. The method of any one of items 42 to 59, wherein the at least one selected T lymphocyte is selected, at least in part, because it is labeled with one or more antibodies associated with a fluorescent label.
Item 61. The method of item 60, wherein the one or more antibodies include an antibody to CD45RA or CD45RO, an antibody to CCR7, an antibody to CD62L, or any combination thereof.
Item 62. The method of item 60 or 61, wherein the one or more antibodies include an antibody to CD4.
Item 63. The method of items 60 or 61, wherein the one or more antibodies include an antibody to CD8.
Item 64. The method of item 41, wherein introducing the one or more T lymphocytes into the sequestration pen further comprises tilting the microfluidic device such that gravity pulls the one or more T lymphocyte into the sequestration pen.
Item 65. The method of any one of items 1 to 64, wherein the one or more T lymphocytes are contacted with the activating agent prior to being introduced into the sequestration pen.
Item 66. The method of item 65, wherein the one or more T lymphocytes are incubated with the activating agent for a period of at least one hour prior to being introduced into the microfluidic device.
Item 67. The method of item 65, wherein the one or more T lymphocytes are incubated with the activating agent for a period of at least five hours prior to being introduced into the microfluidic device.
Item 68. The method of any one of items 65 to 67, wherein introducing the one or more T lymphocytes into the sequestration pen further comprises introducing the activating agent into the sequestration pen.
Item 69. The method of any one of items 1 to 64, wherein the one or more T lymphocytes are contacted with the activating agent after being introduced into the sequestration pen.
Item 70. The method of any one of items 1 to 69, wherein the activating agent comprises anti-CD3 and/or anti-CD28 agonist antibodies.
Item 71. The method of item 70, wherein the activating agent comprises an anti-CD3 agonist antibody which is conjugated to a solid support.
Item 72. The method of item 70 or 71, wherein the activating agent comprises an anti-CD28 agonist antibody which is conjugated to a solid support.
Item 73. The method of item 70 or 71, wherein the activating agent comprises soluble anti-CD28 agonist antibodies.
Item 74. The method of any one of items 1 to 69, wherein the activating agent comprises a dendritic cell (DC).
Item 75. The method of item 74, wherein the DC is pulsed with a tumor antigen prior to contacting the one or more T lymphocytes.
Item 76. The method of item 75, wherein the tumor antigen is isolated from tumor cells that are autologous with the one or more T lymphocytes.
Item 77. The method of item 75, wherein the tumor antigen is identified through genomic analysis of tumor cells.
Item 78. The method of item 77, wherein the analyzed tumor cells are autologous with the one or more T lymphocytes.
Item 79. The method of any one of items 74 to 78, wherein the DC and the one or more T lymphocytes are autologous cells.
Item 80. The method of any one of items 1 to 79, wherein the culture medium is perfused through the flow path of the microfluidic device for a period of at least 24 hours.
Item 81. The method of any one of items 1 to 79, wherein the culture medium is perfused through the flow path of the microfluidic device for a period of at least 48 hours.
Item 82. The method of any one of items 1 to 79, wherein the culture medium is perfused through the flow path of the microfluidic device for a period of at least 96 hours.
Item 83. The method of any one of items 1 to 66, wherein the culture medium comprises human serum and IL2.
Item 84. The method of any one of items 1 to 67, wherein the culture medium comprises IL7, IL15, IL21, or any combination thereof.
Item 85. The method of any one items 1 to 84, wherein 20 or fewer, 10 or fewer, 6-10, 5 or fewer, about 5, about 4, about 3, about 2, or 1 T lymphocyte(s) are introduced into the sequestration pen in the microfluidic device.
Item 86. The method of any one of items 1 to 85, wherein the expansion comprises at least three rounds of mitotic cell division.
Item 87. The method of any one of items 1 to 86, wherein the method comprises exporting the expanded T lymphocytes from the microfluidic device.
Item 88. The method of any one of items 1 to 87, wherein the microfluidic device comprises a plurality of sequestration pens, and wherein one or more T lymphocytes is introduced into each sequestration pen of the plurality and contacted with the activating agent.
Item 89. The method of item 88, wherein 20 or fewer, 10 or fewer, 6-10, 5 or fewer, about 5, about 4, about 3, about 2, or 1 T lymphocyte(s) are introduced into each of a plurality of sequestration pens in the microfluidic device.
Item 90. A T lymphocyte produced according to the method of any one of items 1 to 89.
Item 91. A microfluidic device comprising a T lymphocyte produced according to the method of any one of items 1 to 89.
Item 92. The microfluidic device of item 91, wherein the microfluidic device comprises a sequestration pen, and wherein the T lymphocyte is located in the sequestration pen.
Item 93. A pharmaceutical composition comprising a T lymphocyte produced according to the method of any one of items 1 to 89 and a pharmaceutically acceptable carrier.
Item 94. A method of treating cancer in a subject, the method comprising introducing T lymphocytes into the subject, wherein the T lymphocytes are prepared by the method of any one of items 1 to 89.
Item 95. A method of treating cancer in a subject, the method comprising:
   isolating T lymphocytes from a tissue sample obtained from the subject;
   expanding the isolated T lymphocytes in a microfluidic device according to the method of any one of items 1 to 89;
   exporting the expanded T lymphocytes from the microfluidic device; and
   reintroducing the expanded T lymphocytes into the subject.
Item 96. The method of item 94 or 95, wherein the subject is a mammal.
Item 97. The method of item 96, wherein the subject is a human.
Item 98. The method of any one of items 95 to 97, wherein the tissue sample is a sample of peripheral blood.
Item 99. The method of any one of items 95 to 97, wherein the tissue sample is from a solid tumor.
Item 100. The method of item 99, wherein the tissue sample is a FNA or biopsy from the solid tumor.
Item 101. The method of item 99 or 100, wherein the solid tumor is a breast cancer, a cancer originating in the urinary tract, ureter, bladder, or urethra, renal cell carcinoma, testicular cancer, prostate cancer, or a cancer of the seminal vesicles, seminal ducts, or penis, ovarian cancer, uterine cancer, cervical cancer, vaginal cancer, or a cancer of the fallopian tubes, a cancer of the nervous system, neuroblastoma, retinoblastoma, intestinal cancer, colorectal cancer, lung cancer, or melanoma.
Item 102. The method of item 99 or 100, wherein the solid tumor is a medullary breast cancer, a mesothelioma, or a melanoma.
Item 103. The method of any one of items 95 to 102, wherein isolating T lymphocytes from the tissue sample comprises performing a selection for CD3+ cells, CD4+ cells, CD8+ cells, or any combination thereof in the tissue sample.
Item 104. The method of item 103, wherein isolating T lymphocytes from the tissue sample further comprises dissociating the tissue sample prior to performing the selection for CD3+ cells, CD4+ cells, CD8+ cells, or any combination thereof in the tissue sample.
Item 105. The method of any one of items 95 to 104, wherein expanding the isolated T lymphocytes comprises contacting the isolated T lymphocytes with an activating agent.
Item 106. The method of item 105, wherein the activating agent comprises a CD3 agonist and/or a CD28 agonist.
Item 107. The method of item 106, wherein the activating agent comprises an anti-CD3 agonist antibody.
Item 108. The method of item 106 or 107, wherein the activating agent comprise an anti-CD28 agonist antibody.
Item 109. The method of any one of items 105 to 108, wherein the activating agent is conjugated to one or more beads.
Item 110. The method of any one of items 105 to 108, wherein the microfluidic device comprises at least one sequestration pen configured to support expansion of the isolated T lymphocytes, wherein at least one surface of each of the at least one sequestration pen comprises a coating material, and wherein the activating agent is covalently linked to the coating material.
Item 111. The method of item 110, wherein the activating agent is stably bound to the coating material.
Item 112. The method of item 110, wherein the activating agent is stably bound to the coating material via a biotin-streptavidin linkage.
Item 113. The method of any one of items 95 to 112, wherein the activating agent comprises dendritic cells (DCs).
Item 114. The method of item 113, wherein the DCs are obtained from the subject being treated for cancer.
Item 115. The method of items 113 or 114, wherein the DCs are pulsed with a tumor antigen prior to contacting the isolated T lymphocytes with the activating agent.
Item 116. The method of item 115, wherein the tumor antigen is isolated from cancer cells obtained from the subject.
Item 117. The method of item 115, wherein the tumor antigen is synthetic.
Item 118. The method of any one of items 115 to 117, wherein the tumor antigen is identified, at least in part, through sequencing of nucleic acid molecules from cancer cells obtained from the subject.
Item 119. The method of any one of items 95 to 118, wherein isolated T lymphocytes that undergo expansion in response to being contacted by activating agent exhibit at least 100 fold expansion.
Item 120. The method of any one of items 95 to 118, wherein isolated T lymphocytes that undergo expansion in response to being contacted by activating agent exhibit at least 1000-fold expansion.
Item 121. The method of any one of items 95 to 120, wherein expanded T lymphocytes are selectively exported from the microfluidic device.
Item 122. The method of item 121, wherein the method comprises assaying the proliferation rate of one or more T lymphocytes in one or more pens, and selectively exporting T lymphocytes with a proliferation rate greater than or equal to a predetermined threshold.
Item 123. The method of item 121 or 122, wherein the method comprises assaying the expression of one or more cytokines by one or more T lymphocytes in one or more pens, and selectively exporting T lymphocytes that express the one or more cytokines.
Item 124. The method of item 123, wherein the one or more cytokines include INFgamma, TNFalpha, IL2, or any combination thereof.
Item 125. The method of item 121, wherein the method comprises assaying the expression of one or more regulatory T cell markers by one or more T lymphocytes in one or more pens, and selectively exporting T lymphocytes that express the one or more regulatory T cell markers.
Item 126. The method of item 125, wherein the one or more regulatory T cell markers include CTLA4.
Item 127. The method of any one of items 95 to 126, further comprising genomically profiling a sample of exported T lymphocytes.
Item 128. The method of any one of items 95 to 127, wherein the expanded T lymphocytes are further expanded after being exported from the microfluidic device but prior to being reintroduced into the subject.
Item 129. The method of any one of items 95 to 128, wherein the exporting of expanded T lymphocytes comprises exporting expanded T lymphocytes from their corresponding sequestration pen using a dielectrophoresis (DEP) force.

### EQUIVALENTS

The foregoing written specification is considered to be sufficient to enable one skilled in the art to practice the embodiments. The foregoing description and Examples detail certain embodiments and describes the best mode contemplated. It will be appreciated, however, that no matter how detailed the foregoing may appear in text, the embodiment may be practiced in many ways and should be construed in accordance with the appended claims and any equivalents thereof.

Various preferred features and embodiments of the present invention will now be described with reference to the following numbered paragraphs (paras).
1. A method of expanding T lymphocytes in a microfluidic device having a flow path and a sequestration pen fluidically connected to the flow path, the method comprising:
   introducing one or more T lymphocytes into the sequestration pen in the microfluidic device;
   contacting the one or more T lymphocytes with an activating agent; and
   perfusing culture medium through the flow path of the microfluidic device for a period of time sufficient to allow the one or more T lymphocytes introduced into the sequestration pen to undergo expansion.
2. The method of para 1, wherein the sequestration pen has a volume of about 5x10⁵ to about 5x10⁶ cubic microns.
3. The method of para 1, wherein at least one inner surface of the sequestration pen comprises a coating material.
4. The method of para 3, wherein the coating material is covalently bound to the at least one inner surface of the sequestration pen, and wherein the coating material comprises a polymer comprising alkylene ether moieties, saccharide moieties, amino acid moieties, or a combination thereof, optionally wherein the coating material comprises dextran or poly-ethylene glycol moieties, optionally wherein the coating material comprises one or more proteins.
5. The method of para 3, wherein the coating material comprises molecules, each of which includes a linking group and an alkyl moiety, wherein the linking group is covalently bonded to the at least one inner surface of the sequestration pen, optionally wherein the linking group is a siloxy linking group.
6. The method of para 5, wherein the alkyl moiety comprises a linear chain of carbons comprising at least 10 carbon atoms, and/or wherein the alkyl moiety is a fluoroalkyl moiety or a perflouroalkyl moiety.
7. The method of para 5, wherein the molecules of the coating material form a densely-packed monolayer structure covalently bound to the inner substrate surface.
8. The method of para 3, wherein the coating material comprises molecules having a linking group and a cationic moiety and/or an anionic moiety, wherein the linking group is covalently bonded to the inner substrate surface.
9. The method of para 8, wherein the cationic moiety comprises a quaternary ammonium group or the anionic moiety comprises a phosphonic acid, carboxylic acid, or sulfonic acid.
10. The method of para 8, wherein the coating material comprises molecules having a linking group and a zwitterionic moiety.
11. The method of para 10, wherein the zwitterionic moiety is selected from carboxybetaines, sulfobetaines, sulfamic acids, and amino acids.
12. The method of para 3, wherein the activating agent is covalently linked or stably bound to the coating material, optionally wherein the activating agent is stably bound to the coating material via a biotin-streptavidin linkage.
13. The method of para 1, wherein the one or more T lymphocytes are isolated from a peripheral blood sample taken from a subject or from a solid tumor sample of a subject, optionally wherein the solid tumor is a breast cancer, a cancer originating in the urinary tract, ureter, bladder, or urethra, renal cell carcinoma, testicular cancer, prostate cancer, or a cancer of the seminal vesicles, seminal ducts, or penis, ovarian cancer, uterine cancer, cervical cancer, vaginal cancer, or a cancer of the fallopian tubes, a cancer of the nervous system, neuroblastoma, retinoblastoma, intestinal cancer, colorectal cancer, lung cancer, or melanoma.
14. The method of para 13, wherein the solid tumor sample is a fine needle aspirate (FNA) or a biopsy.
15. The method of para 13, wherein the solid tumor is a medullary breast cancer, a mesothelioma, or a melanoma.
16. The method of para 13, wherein the one or more T lymphocytes are from a population of T lymphocytes isolated from the peripheral blood sample or the solid tumor sample.
17. The method of para 16, wherein the population is enriched for CD3⁺ T lymphocytes.
18. The method of para 16, wherein the population is enriched for CD3⁺CD4⁺ T lymphocytes.
19. The method of para 16, wherein the population is enriched for CD3⁺CD8⁺ T lymphocytes.
20. The method of para 16, wherein the population is enriched for CD45RA⁺CD45RO⁻ T lymphocytes.
21. The method of para 16, wherein the population is enriched for CD45RA⁻CD45RO⁺ T lymphocytes.
22. The method of para 16, wherein the population is enriched for CCR7⁺ T lymphocytes or CD62L⁺ T lymphocytes.
23. The method of para 16, wherein the population is depleted of CD69⁺ T-lymphocytes or PD-1⁺ and/or PD-L1⁺ T-lymphocytes.
24. The method of para 16, wherein the method comprises depleting one, two, three, or four of CD45RO⁺ T lymphocytes, CD45RA⁺ T lymphocytes, CD69⁺ T lymphocytes, PD-1⁺ T lymphocytes, and PD-L1⁺ T lymphocytes by contacting the T lymphocytes with antibodies to CD45RO, CD45RA, CD69, PD-1, and/or PD-L1 and removing from the population T lymphocytes bound to the antibodies.
25. The method of para 24, wherein the antibodies are associated with a solid support, optionally wherein the solid support is a population of magnetic beads.
26. The method of para 1, wherein introducing the one or more T lymphocytes into the sequestration pen comprises flowing a fluid containing the one or more T lymphocytes into a microfluidic channel of the microfluidic device, wherein the microfluidic channel is part of the flow path of the microfluidic device, and wherein the sequestration pen opens off of the microfluidic channel, optionally wherein introducing the one or more T lymphocytes into the sequestration pen further comprises using dielectrophoresis (DEP) to select at least one T lymphocyte located in the microfluidic channel and move it into the sequestration pen.
27. The method of para 26, wherein the at least one selected T lymphocyte is selected, at least in part, because its cell surface comprises the marker CD3⁺, CD4⁺, CD8⁺, or any combination thereof, optionally wherein (i) the at least one selected T lymphocyte is selected, at least in part, because its cell surface comprises the markers CD3⁺ and CD4⁺ or (ii) the at least one selected T lymphocyte is selected, at least in part, because its cell surface comprises the markers CD3⁺ and CD8⁺.
28. The method of para 26, wherein the at least one selected T lymphocyte is selected, at least in part, because its cell surface comprises the marker(s) CD45RA⁺, CD45RO⁺, CCR7⁺, CD62L⁺, or any combination thereof.
29. The method of para 26, wherein selecting the at least one T lymphocyte comprises labeling a population of T lymphocyte that comprises the at least one T lymphocyte with an antibody that specifically binds to CD3, CD4, CD8, CD45RA, CD45RO, CCR7, or CD62L, and moving the at least one T lymphocyte into the sequestration pen based upon its association with the antibody, optionally wherein the antibody comprises a fluorophore.
30. The method of para 26, wherein the at least one selected T lymphocyte is selected, at least in part, because its cell surface is not CD45RO⁺, optionally wherein introducing one or more T lymphocytes into the sequestration pen comprises labeling CD45RO⁺ T lymphocytes and introducing one or more T lymphocytes not associated with a label indicative of CD45RO into the sequestration pen.
31. The method of para 26, wherein the at least one selected T lymphocyte is selected, at least in part, because its cell surface is not CD45RA⁺, optionally wherein introducing one or more T lymphocytes into the sequestration pen comprises labeling CD45RA⁺ T lymphocytes and introducing one or more T lymphocytes not associated with a label indicative of CD45RA into the sequestration pen.
32. The method of para 26, comprising labeling CCR7⁺ and CD62L⁺ T lymphocytes and moving one or more T lymphocytes associated with a label indicative of CCR7 and/or associated with a label indicative of CD62L into the sequestration pen.
33. The method of para 26, comprising labeling CCR7⁺ and CD62L⁺ T lymphocytes and moving one or more T lymphocytes not associated with a label indicative of CCR7 and/or not associated with a label indicative of CD62L into the sequestration pen.
34. The method of para 26, wherein the at least one selected T lymphocyte is selected, at least in part, because its cell surface is not CD69⁺, not PD-1⁺, and/or not PD-L1⁺.
35. The method of para 26, wherein the at least one selected T lymphocyte is selected, at least in part, because it is labeled with an antigen associated with a fluorescent label, optionally wherein the antigen comprises a peptide which is complexed with a MHC protein.
36. The method of para 26, wherein the at least one selected T lymphocyte is selected, at least in part, because it is labeled with one or more antibodies associated with a fluorescent label.
37. The method of para 36, wherein the one or more antibodies include an antibody to CD45RA or CD45RO, an antibody to CCR7, an antibody to CD62L, or any combination thereof
38. The method of para 36, wherein the one or more antibodies include an antibody to CD4 or CD8.
39. The method of para 1, wherein the one or more T lymphocytes are contacted with the activating agent prior to being introduced into the sequestration pen, optionally wherein the one or more T lymphocytes are incubated with the activating agent for a period of at least one hour prior to being introduced into the microfluidic device or for a period of at least five hours prior to being introduced into the microfluidic device.
40. The method of para 39, wherein introducing the one or more T lymphocytes into the sequestration pen further comprises introducing the activating agent into the sequestration pen.
41. The method of para 1, wherein the one or more T lymphocytes are contacted with the activating agent after being introduced into the sequestration pen.
42. The method of para 1, wherein (i) the activating agent comprises anti-CD3 and/or anti-CD28 agonist antibodies, optionally wherein the activating agent comprises an anti-CD3 agonist antibody which is conjugated to a solid support, an anti-CD28 agonist antibody which is conjugated to a solid support, or soluble anti-CD28 agonist antibodies; or (ii) wherein the activating agent comprises a dendritic cell (DC), optionally wherein the DC is pulsed with a tumor antigen prior to contacting the one or more T lymphocytes, further optionally wherein the tumor antigen is isolated from tumor cells that are autologous with the one or more T lymphocytes.
43. The method of para 42, wherein the tumor antigen is identified through genomic analysis of tumor cells, optionally wherein the analyzed tumor cells are autologous with the one or more T lymphocytes.
44. The method of para 42, wherein the DC and the one or more T lymphocytes are autologous cells.
45. The method of para 1, wherein the culture medium is perfused through the flow path of the microfluidic device for a period of at least 24 hours, at least 48 hours, or at least 96 hours.
46. The method of para 1, wherein the culture medium comprises human serum and IL2, and/or wherein the culture medium comprises IL7, IL15, IL21, or any combination thereof.
47. The method of para 1, wherein 20 or fewer, 10 or fewer, 6-10, 5 or fewer, about 5, about 4, about 3, about 2, or 1 T lymphocyte(s) are introduced into the sequestration pen in the microfluidic device.
48. The method of para 1, wherein the expansion comprises at least three rounds of mitotic cell division, and/or wherein the method comprises exporting the expanded T lymphocytes from the microfluidic device.
49. The method of para 1, wherein the microfluidic device comprises a plurality of sequestration pens, and wherein one or more T lymphocytes is introduced into each sequestration pen of the plurality and contacted with the activating agent.
50. A T lymphocyte produced according to the method of any one of paras 1 to 49.
51. A microfluidic device comprising a T lymphocyte produced according to the method of any one of paras 1 to 49, optionally wherein the microfluidic device comprises a sequestration pen and the T lymphocyte is located in the sequestration pen.
52. A pharmaceutical composition comprising a T lymphocyte produced according to the method of any one of paras 1 to 49 and a pharmaceutically acceptable carrier.
53. A method of treating cancer in a subject, the method comprising introducing T lymphocytes into the subject, wherein the T lymphocytes are prepared by the method of any one of paras 1 to 49.
54. A method of treating cancer in a subject, the method comprising:
   isolating T lymphocytes from a tissue sample obtained from the subject;
   expanding the isolated T lymphocytes in a microfluidic device according to the method of any one of paras 1 to 49;
   exporting the expanded T lymphocytes from the microfluidic device; and
   reintroducing the expanded T lymphocytes into the subject.
55. The method of para 53, wherein the subject is a mammal, optionally wherein the subject is a human.
56. The method of para 54, wherein the tissue sample is a sample of peripheral blood or is from a solid tumor, optionally wherein the tissue sample is a FNA or biopsy from the solid tumor, optionally wherein the solid tumor is a breast cancer, a cancer originating in the urinary tract, ureter, bladder, or urethra, renal cell carcinoma, testicular cancer, prostate cancer, or a cancer of the seminal vesicles, seminal ducts, or penis, ovarian cancer, uterine cancer, cervical cancer, vaginal cancer, or a cancer of the fallopian tubes, a cancer of the nervous system, neuroblastoma, retinoblastoma, intestinal cancer, colorectal cancer, lung cancer, or melanoma.
57. The method of para 56, wherein the solid tumor is a medullary breast cancer, a mesothelioma, or a melanoma.
58. The method of para 54, wherein isolating T lymphocytes from the tissue sample comprises performing a selection for CD3⁺ cells, CD4⁺ cells, CD8⁺ cells, or any combination thereof in the tissue sample.
59. The method of para 54, wherein expanding the isolated T lymphocytes comprises contacting the isolated T lymphocytes with an activating agent.
60. The method of para 59, wherein the activating agent comprises a CD3 agonist and/or a CD28 agonist.
61. The method of para 59, wherein the microfluidic device comprises at least one sequestration pen configured to support expansion of the isolated T lymphocytes, wherein at least one surface of each of the at least one sequestration pen comprises a coating material, and wherein the activating agent is covalently linked to the coating material.
62. The method of para 54, wherein the activating agent comprises dendritic cells (DCs).
63. The method of para 62, wherein the DCs are obtained from the subject being treated for cancer and/or the DCs are pulsed with a tumor antigen prior to contacting the isolated T lymphocytes with the activating agent, optionally wherein the tumor antigen is isolated from cancer cells obtained from the subject or wherein the tumor antigen is synthetic.

## Claims

1. A method of expanding T lymphocytes in a microfluidic device having a flow path and a sequestration pen fluidically connected to the flow path, wherein the flow path comprises a microfluidic channel, wherein the sequestration pen comprises an isolation region and a connection region fluidically connecting the isolation region to the channel, and wherein the isolation region of the sequestration pen is an unswept region of the microfluidic device, the method comprising:
introducing 20 or fewer T lymphocytes into the sequestration pen in the microfluidic device;
contacting the T lymphocytes with an activating agent; and
perfusing culture medium through the flow path of the microfluidic device for a period of time sufficient to allow the T lymphocytes introduced into the sequestration pen to undergo expansion.

2. The method of claim 1, wherein 10 or fewer T lymphocytes are introduced into the sequestration pen in the microfluidic device; and optionally wherein 1-5 T lymphocytes are introduced into the sequestration pen in the microfluidic device.

3. The method of claim 1 or 2, wherein the sequestration pen has a volume of about 5x10⁵ to about 5x10⁶ cubic microns; and/or
wherein the connection region of the sequestration pen comprises a proximal opening into the microfluidic channel having a width W_{con} ranging from about 20 microns to about 100 microns and a distal opening into said isolation region, and wherein a length L_{con} of said connection region from the proximal opening to the distal opening is as least 1.0 times a width W_{con} of the proximal opening of the connection region, and optionally, wherein the length L_{con} of said connection region from the proximal opening to the distal opening is at least 1.5 or 2.0 times the width W_{con} of the proximal opening of the connection region.

4. The method of any one of claims 1-3, wherein at least one inner surface of the sequestration pen comprises a coating material, wherein the coating material is covalently bound to the at least one inner surface of the sequestration pen, and further wherein the coating material comprises a polymer comprising alkylene ether moieties, saccharide moieties, amino acid moieties, or a combination thereof; and optionally wherein the coating material comprises at least one of dextran moieties, polyethylene glycol moieties, and one or more proteins.

5. The method of any one of claims 1-3, wherein at least one inner surface of the sequestration pen comprises a coating material, wherein the coating material comprises molecules, each of which includes a linking group and an alkyl moiety, wherein the linking group is covalently bonded to the at least one inner surface of the sequestration pen; and optionally wherein at least one of:
the linking group is a siloxy linking group;
the alkyl moiety comprises a linear chain of carbons comprising at least 10 carbon atoms;
the alkyl moiety is a fluoroalkyl moiety or a perfluoroalkyl moiety; and
the molecules of the coating material form a densely-packed monolayer structure covalently bound to the inner substrate surface.

6. The method of any one of claims 1-3 , wherein at least one inner surface of the sequestration pen comprises a coating material, wherein the coating material comprises molecules having a linking group and a cationic moiety and/or an anionic moiety, wherein the linking group is covalently bonded to the inner substrate surface; and optionally wherein at least one of:
the cationic moiety of the coating material comprises a quaternary ammonium group;
the anionic moiety of the coating material comprises a phosphonic acid, carboxylic acid, or sulfonic acid; and
the coating material comprises molecules having a linking group and a zwitterionic moiety; and optionally further wherein the zwitterionic moiety is selected from carboxybetaines, sulfobetaines, sulfamic acids, and amino acids.

7. The method of any one of claims 1-3, wherein at least one inner surface of the sequestration pen comprises a coating material, and the activating agent is covalently linked or stably bound to the coating material; and optionally wherein the activating agent is stably bound to the coating material via a biotin-streptavidin linkage.

8. The method of any one of claims 1-7, wherein the T lymphocytes are from a population of T lymphocytes isolated from a peripheral blood sample taken from a subject or from a solid tumor sample of a subject; and optionally wherein:
the solid tumor sample is a fine needle aspirate (FNA) or a biopsy; and/or
the solid tumor is a breast cancer, a cancer originating in the urinary tract, ureter, bladder, or urethra, renal cell carcinoma, testicular cancer, prostate cancer, or a cancer of the seminal vesicles, seminal ducts, or penis, ovarian cancer, uterine cancer, cervical cancer, vaginal cancer, or a cancer of the fallopian tubes, a cancer of the nervous system, neuroblastoma, retinoblastoma, intestinal cancer, colorectal cancer, lung cancer, or melanoma.

9. The method of claim 8, wherein the method further comprises enriching the population for CD3⁺ T lymphocytes, CD3⁺CD4⁺ T lymphocytes, or CD3⁺CD8⁺ T lymphocytes; and optionally at least one of;
enriching the population for CD45RA⁺CD45RO⁻ T lymphocytes or CD45RA⁻CD45RO⁺ T lymphocytes;
enriching the population for CCR7⁺ T lymphocytes;
enriching the population for CD62L⁺ T lymphocytes;
depleting the population of CD69⁺ T-lymphocytes;
depleting the population of PD-1⁺ and/or PD-L1⁺ T-lymphocytes; and
depleting the population of one, two, three, or four of CD45RO⁺ T lymphocytes, CD45RA⁺ T lymphocytes, CD69⁺ T lymphocytes, PD-1⁺ T lymphocytes, and PD-L1⁺ T lymphocytes, wherein depleting comprises contacting the T lymphocytes with antibodies to CD45RO, CD45RA, CD69, PD-1, and/or PD-L1 and removing T lymphocytes bound to the antibodies from the population.

10. The method of any one of claims 1-9, wherein introducing the T lymphocytes into the sequestration pen comprises flowing a fluid containing the T lymphocytes into a microfluidic channel of the microfluidic device, wherein the microfluidic channel is part of the flow path of the microfluidic device, and the sequestration pen opens off of the microfluidic channel.

11. The method of claim 10, wherein introducing the T lymphocytes into the sequestration pen further comprises using dielectrophoresis (DEP) to select at least one T lymphocyte located in the microfluidic channel and move it into the sequestration pen.

12. The method of claim 11, wherein the at least one T lymphocyte is selected, at least in part, when its cell surface is CD3⁺, CD4⁺, CD8⁺, or any combination thereof; and optionally wherein:
the at least one T lymphocyte is selected, at least in part, when its cell surface comprises the marker(s) CD45RA⁺, CD45RO⁺, CCR7⁺, CD62L⁺, or any combination thereof; or
selecting the at least one T lymphocyte comprises labeling a population of T lymphocyte that comprises the at least one T lymphocyte with an antibody that specifically binds to CD3, CD4, CD8, CD45RA, CD45RO, CCR7, or CD62L, and moving the at least one T lymphocyte into the sequestration pen based upon its association with the antibody; and optionally further wherein the antibody comprises a fluorophore.

13. The method of claim 11 or 12, wherein the at least one T lymphocyte are selected, at least in part, when their cell surface is
not CD45RO⁺; and optionally wherein introducing the T lymphocytes into the sequestration pen comprises labeling CD45RO⁺ T lymphocytes and introducing the T lymphocytes not associated with a label indicative of CD45RO into the sequestration pen; or
not CD45RA⁺; and optionally wherein introducing the T lymphocytes into the sequestration pen comprises labeling CD45RA⁺ T lymphocytes and introducing the T lymphocytes not associated with a label indicative of CD45RA into the sequestration pen.

14. The method of any one of claims 11-13, comprising labeling CCR7⁺ and CD62L⁺ T lymphocytes and
moving the T lymphocytes associated with a label indicative of CCR7 and/or associated with a label indicative of CD62L into the sequestration pen; or
moving the T lymphocytes not associated with a label indicative of CCR7 and/or not associated with a label indicative of CD62L into the sequestration pen.

15. The method of any one of claims 11-14, wherein the at least one T lymphocyte is selected, at least in part, when at least one of:
its cell surface is at least one of not CD69⁺, not PD-1⁺, and not PD-L1⁺; and
it is labeled with an antigen associated with a fluorescent label; and optionally further wherein the antigen comprises a peptide which is complexed with a MHC protein.

16. The method of any one of claims 1-15, wherein the T lymphocytes are contacted with the activating agent prior to being introduced into the sequestration pen; and optionally wherein the T lymphocytes are incubated with the activating agent for a period of at least one hour prior to being introduced into the microfluidic device or for a period of at least five hours prior to being introduced into the microfluidic device.

17. The method of any one of claims 1-15, wherein the T lymphocytes are contacted with the activating agent after being introduced into the sequestration pen.

18. The method of any one of claims 1-17, wherein
the activating agent comprises anti-CD3 and/or anti-CD28 agonist antibodies; and optionally wherein the activating agent comprises an anti-CD3 agonist antibody which is conjugated to a solid support, an anti-CD28 agonist antibody which is conjugated to a solid support, or soluble anti-CD28 agonist antibodies; or
the activating agent comprises a dendritic cell (DC); and optionally wherein the DC is pulsed with a tumor antigen prior to contacting the T lymphocytes; and further optionally wherein the tumor antigen is isolated from tumor cells that are autologous with the T lymphocytes.

19. The method of any one of claims 1-18, wherein the culture medium comprises human serum and IL2, and/or wherein the culture medium comprises IL7, IL15, IL21, or any combination thereof.

20. The method of any one of claims 1-19, wherein the method comprises exporting the expanded T lymphocytes from the microfluidic device.

21. A T lymphocyte produced according to the method of any one of claims 1-20.

22. A pharmaceutical composition comprising a T lymphocyte produced according to the method of any one of claims 1-20 and a pharmaceutically acceptable carrier.
